# EUROPÄISCHE PATENTSCHRIFT

(11) **EP 0 526 708 B1**
(45) Veröffentlichungstag und Bekanntmachung des Hinweises auf die Patenterteilung: **18.10.2000**
(21) Anmeldenummer: 92109431.4
(22) Anmeldetag: 04.06.1992
(51) Int. Cl.: C07D 239/52, C07D 239/46, A61K 31/505

(54) **Sulfonamide, ihre Herstellung und Verwendung als Heilmittel und Zwischenprodukte**
Sulfonamide, preparation and use thereof as medicine and intermediate
Sulfonamide, sa préparation et son usage comme médicament et intermédiaire

(30) Priorität: 13.06.1991 CH 176091; 12.05.1992 CH 151692
(43) Veröffentlichungstag der Anmeldung: 10.02.1993
(73) Patentinhaber: F. HOFFMANN-LA ROCHE AG, 4002 Basel (CH)
(72) Erfinder: Burri, Kaspar, CH-4102 Binningen (CH); Clozel, Martine, F-68300 St.Louis (FR); Fischli, Walter, Ch-4123 Allschwil (CH); Hirth, Georges, F-68330 Huningue (FR); Löffler, Bernd-Michael, W-7814 Oberrimsingen (DE); Ramuz, Henri, CH-4127 Birsfelden (CH); Neidhart, Werner, F-68870 Bartenheim (FR)
(74) Vertreter: Witte, Hubert, Dr.

(56) Entgegenhaltungen:
- DE-A- 1 545 944

## Beschreibung

Die vorliegende Erfindung betrifft neue Sulfonamide und deren Verwendung als Heilmittel. Insbesondere betrifft die Erfindung neue Verbindungen der Formel 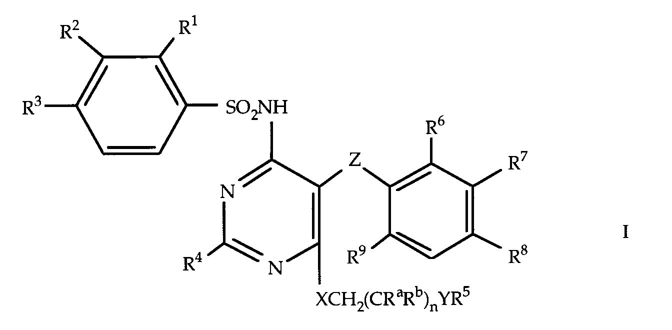 worin
- R¹: Wasserstoff, C₁₋₇-Alkyl, C₁₋₇-Alkoxy, C₁₋₇-Alkylthio, Halogen oder Trifluormethyl;
- R²: Wasserstoff, Halogen, C₁₋₇-Alkoxy, Trifluormethyl oder -OCH₂COOR^{a};
- R³: Wasserstoff, Halogen, C₁₋₇-Alkyl, C₁₋₇-Alkylthio, Trifluormethyl, C₃₋₈-Cycloalkyl, C₁₋₇-Alkoxy oder Trifluormethoxy;
- R² und R³: zusammen Butadienyl, Methylendioxy, Aethylendioxy oder Isopropylidendioxy;
- R⁴: Wasserstoff, C₁₋₇-Alkyl, C₃₋₈-Cycloalkyl, Trifluormethyl, C₁₋₇-Alkoxy, C₁₋₇-Alkylthio, C₁₋₇-Alkylthio-C₁₋₇-alkyl, Hydroxy-C₁₋₇-alkyl, Hydroxy-C₁₋₇-alkoxy, C₁₋₇-Alkoxy-C₁₋₇alkyl, Hydroxy-C₁₋₇alkoxy-C₁₋₇-alkyl, Hydroxy-C₁₋₇alkoxy-C₁₋₇-alkoxy, C₁₋₇-Alkylsulfinyl, C₁₋₇-Alkylsulfonyl, 2-Methoxy-3-hydroxypropoxy, 2-Hydroxy-3-phenylpropyl, Amino-C₁₋₇-alkyl, C₁₋₇-Alkylamino-C₁₋₇-alkyl, Di-C₁₋₇-alkylamino-C₁₋₇-alkyl, Amino, C₁₋₇-Alkylamino, Di-C₁₋₇alkylamino, Arylamino, Aryl, Arylthio, Aryloxy, Aryl-C₁₋₇-alkyl, worin Aryl unsubstituiertes Phenyl oder Phenyl substituiert mit C₁₋₇-Alkyl, Trifluormethyl, C₁₋₇-Alkoxy, Carboxyl, Halogen darstellt, unsubstituiertes Heterocyclyl aus der Gruppe von 2-Furyl, 3-Furyl, Pyrimidinyl, 2-Pyridyl, 3-Pyridyl, 4-Pyridyl oder 2, 3, oder 4-Pyridyl-N-oxid, 1,2-oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl, Chinazolyl oder Heterocyclyl wie vorher definiert substituiert mit C₁₋₇-Alkyl, C₁₋₇-Alkoxy, Halogen, Aryl oder Aryl-C₁₋₇-Alkyl;
- R⁵: Wasserstoff, C₁₋₇-Alkyl, C₁₋₇-Alkanoyl, Benzoyl, Heterocyclylcarbonyl, Heterocyclylmethyl, worin Heterocyclyl ausgewählt aus unsubstituiertem 2-Furyl, 3-Furyl, Pyrimidinyl, 2-Pyridyl, 3-Pyridyl, 4-Pyridyl, 2-, 3-, oder 4-Pyridyl-N-oxid, 1,2- oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl, Chinazolyl oder Heterocyclyl wie vorher definiert substituiert mit C₁₋₇-Alkyl, C₁₋₇-Alkoxy, Halogen, Phenyl oder Aryl-C₁₋₇-Alkyl ist; oder Tetrahydropyran-2-yl;
- R⁶ bis R⁹: Wasserstoff, Halogen, Trifluormethyl, C₁₋₇-Alkyl, C₁₋₇-Alkoxy, C₁₋₇-Alkylthio, Hydroxy, Hydroxymethyl, Cyano, Carboxyl, Formyl, Methylsulfinyl, Methylsulfonyl, Methylsulfonyloxy oder C₁₋₇-Alkyloxycarbonyloxy;
- R⁷: zusammen mit R⁶ oder R⁸ Butadienyl, Methylendioxy, Aethylendioxy oder Isopropylidendioxy;
- Z: -O-, -S-, Vinylen, -CO-, -OCHR¹⁰- oder -SCHR¹⁰;
- R¹⁰: Wasserstoff oder C₁₋₇-Alkyl;
- X und Y: unabhängig voneinander O, S oder NH;
- YR⁵: auch C₁₋₇-Alkylsulfinyl;
- R^{a}, R^{b}, R^{c} und R^{d}: unabhängig voneinander Wasserstoff oder C₁₋₇-Alkyl; oder
- R^{c} und R^{d}: zusammen Methylen, Aethylen oder Isopropyliden; und
- n: 1, 2 oder 3 bedeuten,
und Salze davon.

Der hier verwendete Ausdruck C₁₋₇ bezeichnet Gruppen mit 1-7 C-Atomen, vorzugsweise 1-4 C-Atomen. Alkyl-, Alkoxy- und Alkylthiogruppen sowie Alkylgruppen als Bestandteile von Alkanoylgruppen können geradkettig oder verzweigt sein. Beispiele solcher Alkylgruppen sind Methyl, Aethyl, Propyl, Isopropyl, Butyl, sek. und tert.Butyl. Halogen bezeichnet Fluor, Chlor, Brom und Jod, wobei Chlor bevorzugt ist. Cycloalkylreste enthalten 3 bis 8 C Atome wie Cyclopropyl, Cyclobutyl, Cyclopentyl oder Cyclohexyl. Beispiele von Arylresten sind Phenyl und substituierte Phenylreste, wobei als Substituenten insb. Halogen, nieder-Alkyl, nieder-Alkoxy, Carboxyl und Trifluormethyl in Betracht kommen. Beispiele von Heterocyclylresten sind insbesondere substituierte, z.B. durch nieder-Alkyl, nieder-Alkoxy, Halogen, Aryl, Aryl-nieder-alkyl mono- oder di-substituierte oder unsubstituierte mono- oder bicyclische 5- und 6-gliedrige heterocyclische Reste mit Sauerstoff, Stickstoff oder Schwefel als Heteroatom, wie 2- und 3-Furyl, Pyrimidinyl, 2-, 3- und 4-Pyridyl und Pyridyl-N-oxid, 1,2- und 1,4-Diazinyl, Morpholino, 2- und 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl und Chinazolyl.

Die Verbindungen der oben angegebenen Formel I sind Hemmstoffe für Endothelin-Rezeptoren. Sie können daher zur Behandlung von Erkrankungen, die mit Endothelin-Aktivitäten assoziiert sind, insbesondere Kreislauferkrankungen, wie Hypertonie, Ischämie, Vasospasmen und Angina pectoris verwendet werden.

Eine bevorzugte Gruppe von Verbindungen innerhalb der Formel I sind diejenigen, worin Z -O- ist und weiterhin diejenigen, in denen R⁶ nieder-Alkoxy, insbesondere Methoxy; und R⁷, R⁸ und R⁹ Wasserstoff bedeuten; oder R⁶ und R⁸ Wasserstoff, R⁷ nieder-Alkoxy, insbesondere Methoxy, und R⁹ Halogen, insbesondere Chlor bedeuten.

Bevorzugte Substituenten R¹ und R² sind Wasserstoff, bevorzugte Substituenten R³ nieder-Alkyl, oder zusammen mit R², Methylendioxy. Bevorzugte Substituenten R⁴ sind Wasserstoff, 2-Pyrimidinyl, 2- und 3-Furyl, 2- und 3-Thienyl, Morpholino und p-Methoxyphenyl. X ist vorzugsweise Sauerstoff. Bevorzugte Reste YR⁵ sind Hydroxy, nieder-Alkoxysulfinyl und Furoyloxy.

Die Verbindungen der Formel I können dadurch hergestellt werden, dass man
a) eine Verbindung der Formel 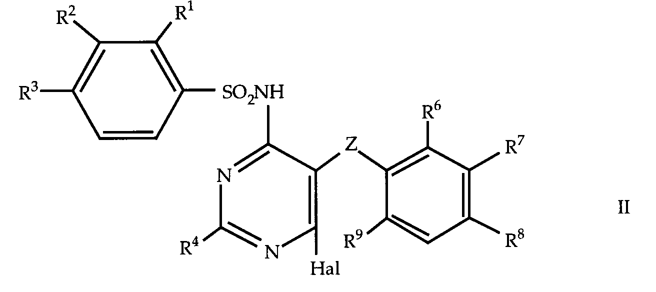 worin R¹, R², R³, R⁴ und R⁶ die oben genannte Bedeutung haben
   und Hal Halogen ist,
   mit einer Verbindung der Formel

   MXCH₂(CR^{a}R^{b})ₙYR⁵ III

   worin X, Y, n, R^{a}, R^{b} und R⁵ die oben genannte Bedeutung haben,
   und M ein Alkalimetall darstellt,
   umsetzt, oder
b) eine Verbindung der Formel 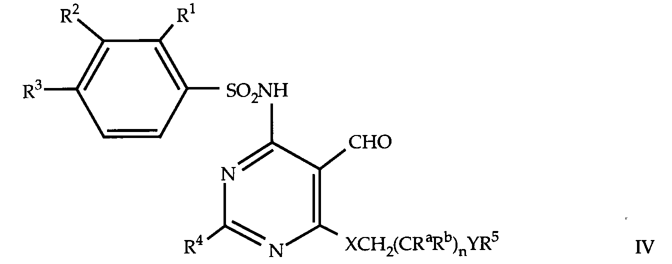 worin R¹-R⁵, R^{a}, R^{b}, X, Y und n die oben genannte Bedeutung haben,
   mit einer Verbindung der Formel 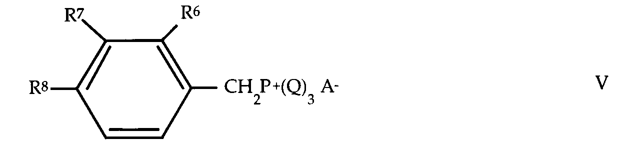 worin R⁶-R⁸ die oben genannte Bedeutung haben; Q Aryl und A⁻ ein Anion ist,
   umsetzt, oder
c) eine Verbindung der Formel 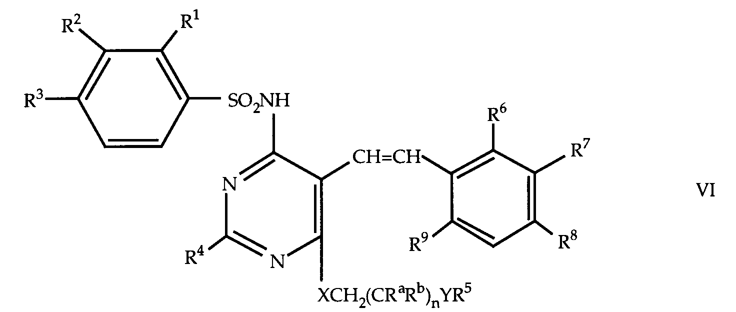 worin R¹-R⁸, R^{a}, R^{b}, X, Y und n die oben genannte Bedeutung haben, hydriert, oder
d) eine Verbindung der Formel 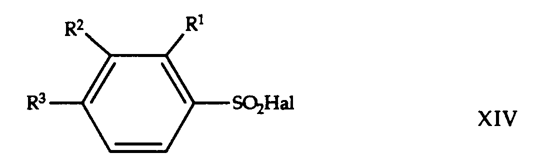 mit einer Verbindung der Formel 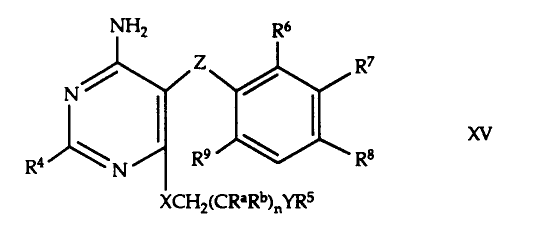 wobei R¹-R⁹, R^{a}, R^{b}, X, Y, Z und n die oben genannte Bedeutung haben,
   umsetzt, und gegebenenfalls in der erhaltenen Verbindung der Formel I enthaltene Substituenten abwandelt und/oder die erhaltene Verbindung der Formel I in ein Salz überführt.

Die Umsetzung einer Verbindung der Formel II mit einer Verbindung der Formel III wird zweckmässig unter Verwendung des der Verbindung III zugrundeliegenden Glykols durchgeführt, also z.B. in Aethylenglykol, wenn n = 2 ist. Das Alkalimetall M ist vorzugsweise Natrium. Die Reaktion wird zweckmässig unter Erwärmen, z.B. auf 40-120°C, vorgenommen. In einer bevorzugten Ausführungsform setzt man als Verbindung der Formel III das Mono-Natriumsalz von Aethylen-, Propylen- oder Butylenglykol ein.

Die Umsetzung einer Verbindung der Formel IV mit einer Verbindung der Formel V kann in an sich bekannter Weise unter den üblichen Bedingungen einer Wittig-Reaktion durchgeführt werden. Der Arylrest Q ist vorzugsweise Phenyl, Beispiele von Anionen A⁻ sind Cl⁻. Br⁻,HSO₄, und Tosyloxy. Die Reaktionspartner werden zweckmässig in Gegenwart eines säurebindenden Mittels, z.B. in Gegenwart einer starken Base, wie z.B. Butyllithium, Natriumhydrid oder dem Natriumsalz von Dimethylsulfoxid, oder K-tert.-Butylat, vornehmlich aber in Gegenwart eines gegebenenfalls durch niederes Alkyl substituierten Aethylenoxids wie 1,2-Butylenoxid, gegebenenfalls in einem Lösungsmittel, z.B. in einem Aether, wie Diäthyläther oder Tetrahydrofuran, oder in einem aromatischen Kohlenwasserstoff, wie Benzol in einem zwischen Raumtemperatur und dem Siedepunkt des Reaktionsgemisches liegenden Temperaturbereich miteinander urngesetzt. Mit der Wittig-Reaktion interferierende reaktive Gruppen in den Reaktionspartnern, wie Carboxyl oder Amino werden zweckmässig intermediär geschützt, z.B. als Carbonsäureester bzw. als tert.Butoxycarbonylaminoderivat.

Die Hydrierung einer Verbindung der Formel VI kann unter für die Hydrierung olefinischer Doppelbindungen an sich bekannter Weise, z.B. mit Wasserstoff bei Normaldruck oder erhöhtem Druck in Gegenwart von Edelmetallkatalysatoren wie Pd, insbesondere Pd auf Trägern wie Pd/C vorgenommen werden.

Bei der Umsetzung einer Verbindung der Formel XIV mit einer Verbindung der Formel XV werden in der letzten Verbindung als Substituenten R⁴-R⁹ gegebenenfalls anwesende Hydroxy- und Aminogruppen zweckmässig geschützt. Hydroxygruppen können z.B. durch Silylgruppen, wie Dimethyl-tert-butylsilylgruppen oder Acylgruppen, wie Acetyl; Aminogruppen durch tert-Butoxycarbonyl oder Benzyloxycarbonyl geschützt werden. Diese Schutzgruppen können in an sich bekannter Weise eingeführt und nach der Umsetzung der Verbindungen XIV und X wieder entfernt werden.

In der so erhaltenen Verbindung der Formel I können darin anwesende Substituenten abgewandelt werden. Beispielsweise kann eine Hydroxygruppe R⁵ verestert oder veräthert werden. Eine Hydroxygruppe R⁵ kann in eine Aethergruppe, z.B. den Tetrahydropyranyläther, oder eine Estergruppe, z.B. das Azetat, umgewandelt wird, andererseits können solche im primär erhaltenen Reaktionsprodukt enthaltene Gruppen oder Ketale, die z.B. als Substituent YR⁵ vorliegen können, in an sich bekannter Weise abgespalten werden. Methylthiogruppen können zu Methylsulfinyl- oder Methylsulfonylgruppen oxidiert werden. Weiterhin können N-heterocyclische Reste, wie Pyridyl, zu N-Oxiden oxidiert werden. Alle diese Reaktionen können nach an sich bekannten Methoden vorgenommen werden. Die Verbindungen der Formel I können in an sich bekannter Weise in Salze, z.B. Alkalisalze wie Na- und K-Salze überführt werden.

Die als Ausgangsmaterial eingesetzten Verbindungen können, soweit sie nicht bekannt sind oder ihre Herstellung nachstehend beschrieben ist, in Analogie zu bekannten bzw. den nachstehend beschriebenen Methoden hergestellt werden.

Verbindungen der Formel II können wie in dem nachstehenden Formelschema dargestellt, erhalten werden:

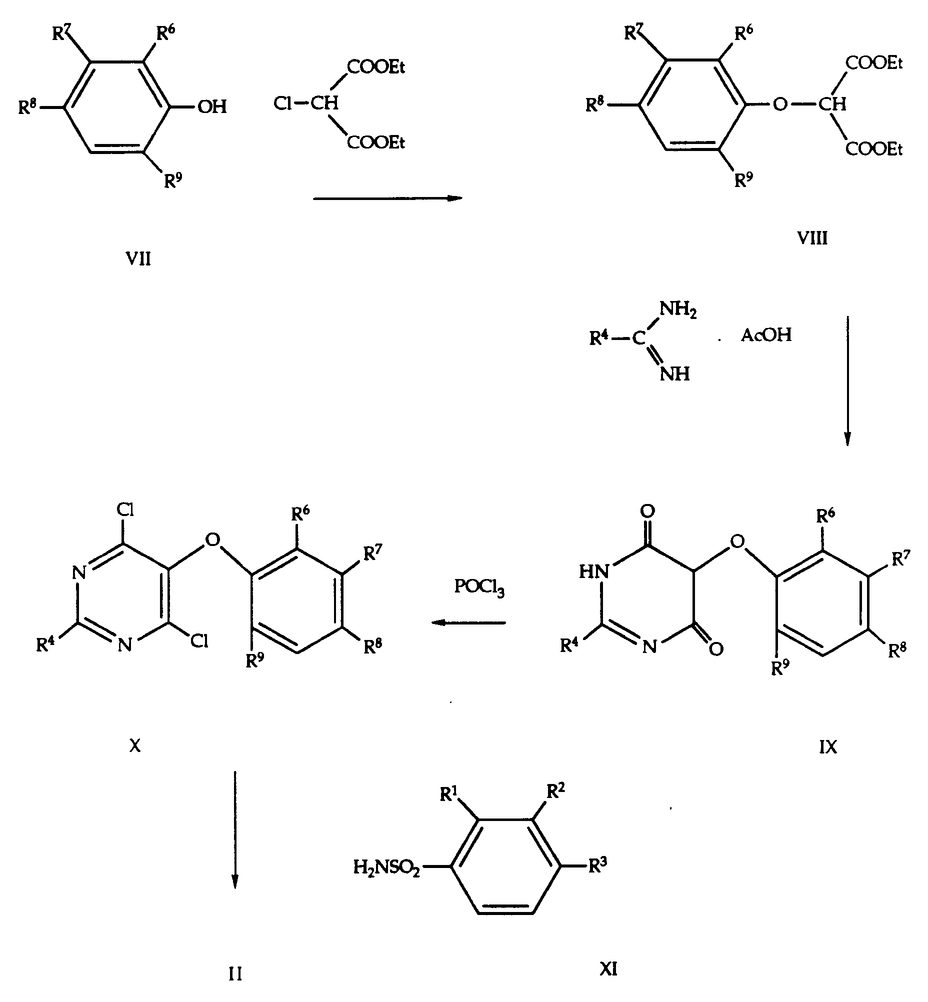

Alkylierung des Phenols VII mit Chlormalonsäurediäthylester liefert die Verbindung VIII, die mit Formamidinacetat oder einer homologen Verbindung wie Acetamidinacetat zum Pyrimidindionderivat IX kondensiert wird. Mit Phosphoroxichlorid erhält man daraus die Dichlorverbindung X, die bei der Umsetzung mit der stöchiometrischen Menge der Verbindung XI die Verbindung II liefert. Alle diese Umsetzungen sind Standardoperationen und können unter für solche Reaktionen üblichen und dem Fachmann geläufigen Bedingungen ausgeführt werden.

Verbindungen der Formel IV können nach dem im nachstehend skizzierten Reaktionsschema erhalten werden:

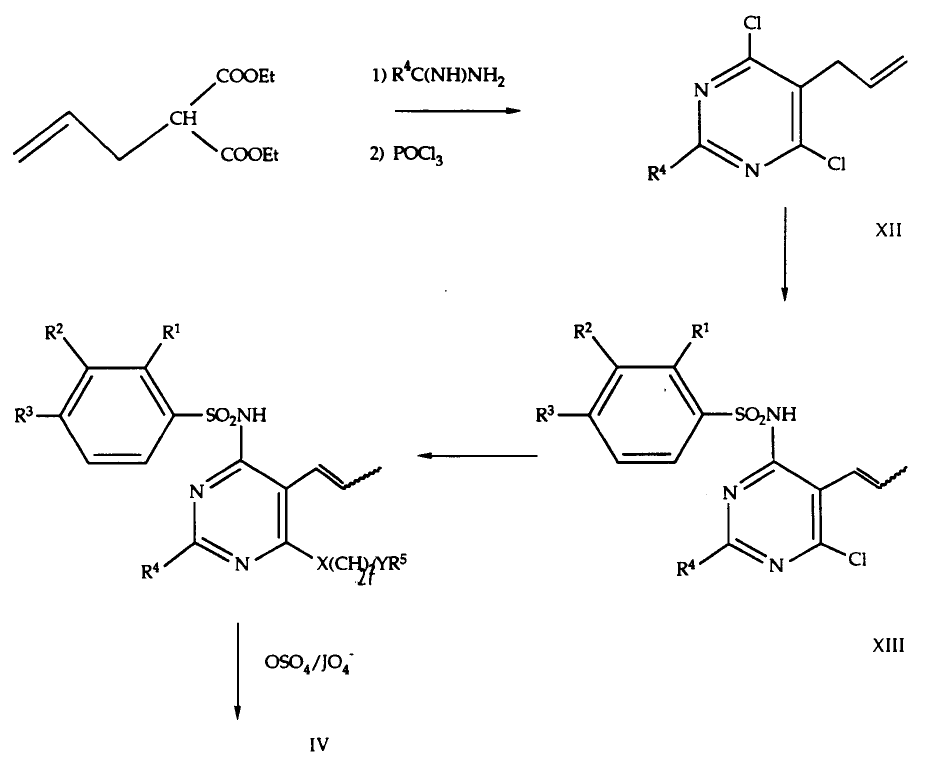

Kondensation von Allylmalonsäureester mit Formamidinacetat oder einem R⁴-substituierten Derivat gefolgt von einem Austausch der Hydroxygruppen gegen Chlor in dem erhaltenen Pyrimidindion liefert das Dichlorpyrimidin XII, das mit einem R¹,R²,R³-Benzolsulfonamid-Alkalisalz unter Umlagerung der Allyldoppelbindung zu der Verbindung XIII kondensiert wird. Umsetzung der Verbindung XIII mit einer Verbindung III in der bereits beschriebenen Weise führt zur Verbindung XIV. Oxidative Spaltung der Doppelbindung der Propenylseitenkette in der Verbindung XIV liefert schliesslich den Aldehyd IV.

**Die DE-OS 1 545 944 offenbart Phenylsulfonylaminopyrimidine, die eine blutdrucksenkende Wirkung haben. Diese Verbindungen unterscheiden sich von den erfindungsgemässen neuen Sulfonamiden durch unterschiedliche Substitutionsmuster sowohl am Phenylring der Sulfonamidgruppe, als auch in der 2-,5- und 6-Position am Pyrimidinring. Während bei den bekannten Verbindungen nur in der para-Position des Phenylrings eine Substitution bekannt war, sind bei den erfindungsgemässen Verbindungen Substituenten in ortho-, und/oder meta- und/oder para-Position am Phenylring der Sulfonamidgruppe vorhanden. Die erfindungsgemässen Verbindungen können in der 5-Position des Pyrimidinringes mit einem vinylischen Rest substituiert sein, der aus den oben erwähnten Druckschriften nicht bekannt ist.**

Die Hemmwirkung der Verbindungen der Formel I auf Endothelinrezeptoren kann mit der nachstehend beschriebenen Versuchsanordnungen gezeigt werden:

### I:Hemmung der Endothelin-Bindung an Human-Placentamembranen (vgl. Life Sci 44:1429 (1989))

Humanplacenta wird in 5 mM Tris-Puffer, pH 7.4, der 1 mM MgCl₂ und 250 mM Sucrose enthält, homogenisiert. Das Homogenisat wird mit 3000 g 15 Minuten bei 4°C zentrifugiert, der die Plasmamembranfraktion enthaltende Ueberstand mit 72000 g 30 Minuten zentrifugiert und der Bodenkörper mit 75 mM Tris-Puffer, pH 7.4, der 25 mM MgCl₂ enthält, gewaschen. Danach wird der aus jeweils 10 g Originalgewebe erhaltene Bodenkörper in 1 ml 75 mM Tris-Puffer pH 7.4, enthaltend 25 mM MgCl₂ und 250 mM Sucrose, suspendiert und in 1-ml-Aliquots bei -20°C eingefroren. Für den Bindungs-Assay werden die eingefrorenen Membranpräparate aufgetaut und nach 10 Minuten Zentrifugieren mit 25000 g bei 20°C in Assay-Puffer (50 mM Tris-Puffer pH 7.4, enthaltend 25 mM MnCl₂, 1 mM EDTA und 0,5% Rinderserumalbumin) resuspendiert. 100 ml dieser Membransuspension, enthaltend 70 mg Protein werden mit 50 ml ¹²⁵I-Endothelin (spezif. Aktivität 2200 Ci/mMol) in Assay-

Puffer (25000 cpm, Endkonzentration 20 pM) und 100 ml Assay-Puffer, der variierende Konzentrationen der Testverbindung enthält, inkubiert. Die Inkubation wird 2 Stunden bei 20°C oder 24 Stunden bei 4°C durchgeführt.

Die Trennung von freiem und Membran-gebundenen Radioliganden wird durch Filtration über Glasfaserfilter vorgenommen.

In der Tabelle 1 ist die in dieser Versuchsanordnung ermittelte Hemmwirkung von Verbindungen der Formel I als IC₅₀ angegeben, d.h., als Konzentration [mM] die erforderlich ist, 50% der spezifischen Bindung von ¹²⁵I-Endothelin zu hemmen.

**Tabelle 1**

| Verbindung von Beispiel | IC₅₀ [µM] |
|---|---|
| 1 | 0,115 |
| 2 | 0,100 |
| 6 | 0,200 |
| 12 | 0,125 |
| 24 | 0,073 |
| 25 | 0,050 |
| 27 | 0,099 |

### II. Hemmung Endothelin-induzierter Kontraktionen an isolierten Aortenringen der Ratte

Aus der Thoraxaorta von erwachsenen Wistar-Kyoto-Ratten wurden Ringe von 5 mm Länge herausgeschnitten. Das Endothel wurde durch leichtes Reiben der Innenfläche entfernt. Jeder Ring wurde bei 37°C in 10 ml Krebs-Henseleit-Lösung unter Begasung mit 95% O₂ und 5% CO₂ in ein isoliertes Bad eingetaucht. Die isometrische Spannung der Ringe wurde gemessen. Die Ringe wurden auf eine Vorspannung von 3 g gedehnt. Nach 10 Minuten Inkubation mit der Testverbindung oder Vehikel wurden kumulative Dosen von Endothelin-1 zugegeben. Die Aktivität der Testverbindung wurde durch Berechnung des Dosisverhältnisses, d.h. der durch 100 µM Testverbindung induzierten Rechtsverschiebung (Verschiebung zu höheren Werten) der EC₅₀ von Endothelin bestimmt, wobei EC₅₀ die für eine halbmaximale Kontraktion erforderliche Endothelin-Konzentration bezeichnet. Je grösser dieses Dosisverhältnis ist, desto potenter hemmt die Testverbindung die biologische Wirkung von Endothelin-1. Die EC₅₀ von Endothelin in Abwesenheit von Testverbindungen ist 0,3 nM.

Die mit Verbindungen der Formel I so erhaltenen Werte für die Rechtsverschiebung der EC₅₀ von Endothelin sind in Tabelle 2 angegeben.

**Tabelle 2**

| Verbindung von Beispiel | Dosisverhältnis (Rechtsverschiebung) |
|---|---|
| 1 | 165 |
| 6 | 395 |
| 24 | 257 |
| 25 | 238 |

### III. Die Hemmwirkung der Verbindungen der Formel I auf die Vasokonstriktion kann in vivo an der Ratte in der nachstehend beschriebenen Versuchsanordnung beobachtet werden:

Ratten wurden mit Na-Thiobutabarbital (100 mg/kg i.p.) anästhesiert. Ueber die Femoralarterie wurde ein Katheter zur Messung des systemischen arteriellen Blutdrucks und über die Femoralvene ein Katheter in die Vena cava inferior zur Injektion der Testverbindungen gelegt. Eine Dopplersonde wurde um die linke Nierenarterie gelegt und mit einem Doppler-Messgerät verbunden. Durch 45 Minuten langes Abklemmen der linken Nierenarterie am Austrittsort wurde eine renale Ischämie erzeugt. Die Testverbindungen wurden 10 Minuten vor der Einleitung der Ischämie intraarteriell (i.a.) in Dosen von 5 mg/kg oder intravenös (i.v.) in Dosen von 10 mg/kg verabreicht. In Kontrollversuchen war die renale Durchblutung im Vergleich zum präischämischen Wert um 43±4% vermindert.

Die mit zwei Verbindungen der Formel I erhaltenen Messwerte sind in Tabelle 3 angegeben.

**Tabelle 3**

| Verbindung von Beispiel | % Abnahme der renalen Durchblutung |
|---|---|
| 1 | 13,4 ± 5,2 |
| 6 | 11,7 ± 4,7 |

Die Verbindungen der Formel I können aufgrund ihrer Fähigkeit, die Endothelinbindung zu hemmen, als Mittel zur Behandlung von Erkrankungen verwendet werden, die mit die Vasokonstriktion erhöhenden Vorgängen assoziiert sind. Beispiele solcher Erkrankungen sind Bluthochdruck, Koronarerkrankungen, Herzinsuffizienz, renale und myocardiale Ischämie, Niereninsuffizienz, Dialyse, cerebrale Ischämie, Hirninfarkt, Migräne, subarachnoidale Hämorrhagie, Raynaud-Syndrom und pulmonärer Hochdruck. Sie können ebenfalls bei Atherosklerose, Verhinderung der Restenosierung nach Ballon-induzierter Gefässdilatation, Entzündungen, Magen- und Duodenalulcera, Ulcus cruris, Gram-negativer Sepsis, Schock, Glomerulonephritis, Nierenkolik, Glaukom, Asthma, bei der Therapie und Prophylaxe diabetischer Komplikationen und Komplikationen bei der Verabreichung von Cyclosporin, sowie anderen, mit Endothelin-Aktivitäten assoziierten Erkrankungen Anwendung finden.

Die Verbindungen der Formel I können oral, rectal, parenteral, z.B. intravenös, intramuskulär, subcutan, intrathecal oder transdermal; oder sublingual oder als ophthalmologische Zubereitung, oder als Aerosol verabreicht werden. Beispiele von Applikationsformen sind Kapseln, Tabletten, oral verabreichbare Suspensionen oder Lösungen, Suppositorien, Injektionslösungen, Augentropfen, Salben oder Spraylösungen.

Eine bevorzugte Anwendungsform ist die intravenöse, intramuskuläre oder orale Applikation. Die Dosierung, in denen die Verbindungen der Formel I in wirksamen Mengen verabreicht werden, hängen von der Art des spezifischen Wirkstoffs, dem Alter und den Bedürfnissen des Patienten und der Applikationsweise ab. Im allgemeinen kommen Dosierungen von etwa 0,1-100 mg/kg Körpergewicht pro Tag in Betracht. Die die Verbindungen der Formel I enthaltenden Präparate können inerte oder auch pharmakodnymisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Die nachstehenden Beispiele erläutern die Erfindung weiter. Von den darin verwendeten Abkürzungen bedeutet THF Tetrahydrofuran; DMSO Dimethylsulfoxid; MeOH Methanol; Sdp. Siedepunkt; und Smp. Schmelzpunkt.

### Beispiel 1

a) Zu einer Natriumglykolatlösung aus 3,0 g Aethylenglykol und 138 mg Natrium wurden 886 mg p-t-Butyl-N-[6-chlor-5-(o-methoxyphenoxy)-4-pyrimidinyl]benzolsulfonamid gegeben. Das Reaktionsgemisch wurde unter Argon 4 Stunden bei 95°C gerührt. Danach wurde das Aethylenglykol abdestilliert und der Rückstand zwischen Aethylacetat und 1N Salzsäure verteilt. Die organische Phase wurde getroclnet und das Lösungsmittel abdestilliert. Der Rückstand wurde aus Diisopropyläther kristallisiert. Man erhielt 870 mg p-t-Butyl-N-[6-(2-hydroxyäthoxy)-5-(o-methoxyphenoxy)-4-pyrimidinyl]benzolsulfonamid. Smp. 143-148°C.
b) 775 mg des vorstehend erhaltenen Sulfonamids wurden in 20 ml warmem Aethanol gelöst. Die Lösung wurde mit der stöchiometrischen Menge Natriumäthylat versetzt, danach wurde das Aethanol abdestilliert, bis sich ein Niederschlag bildete. Zur Vervollständigung der Ausfällung wurden 3 ml Isopropyläther zugegeben. Man erhielt 775 mg p-t-Butyl-N-[6-(2-hydroxyäthoxy)-5-(o-methoxyphenoxy)-4-pyrimidinyl]benzolsulfonamid-Natrium, Smp. >250°C.
   Das Ausgangsmaterial wurde wie folgt hergestellt:
c) Zu einer Natriummethylatlösung aus 150 ml Methanol und 4,6 g Natrium wurden nacheinander 25 g Guajacol und 37 g Chlormalonsäuredimethylester getropft. Die Suspension wurde 1 Stunde bei 45°C unter Feuchtigkeitsausschluss gerührt, danach wurde das Methanol abdestilliert. Der Rückstand wurde in 200 ml Toluol aufgenommen und mit Wasser, 1% Natronlauge und Wasser gewaschen, bis die organische Phase farblos war. Nach Trocknen und Eindampfen des Lösungsmittels wurde der Rückstand destilliert. Man erhielt 39,5 g Dimethyl-(o-methoxyphenoxy)malonat. Sdp. 128°C/7 Pa.
d) Zu einer Natriummethylatlösung aus 150 ml Methanol und 3,5 g Natrium wurde unter Eiskühlung 5,5 g Formamidinacetat und 12,7 g Dimethyl-(o-methoxyphenoxy)malonat gegeben. Das Reaktionsgemisch wurde 1 Stunde unter Feuchtigkeitsausschluss bei 0-5°C, dann 2 Stunden bei Raumtemperatur gerührt. Danach wurde das Lösungsmittel abdestilliert, der Rückstand in 100 ml Wasser aufgenommen, die wässrige Phase mit Toluol extrahiert und die organischen Phasen verworfen. Die wässrige Phase wurde angesäuert, wobei das 5-(o-Methoxyphenoxy)-6-hydroxy-4(3H)-pyrimidinon ausfiel.
e) 9,4 g des vorstehend erhaltenen Pyrimidinons wurden in 20 ml Acetonitril suspendiert und mit 12 g Collidin versetzt. Danach wurden 5 ml POCl₃ in 15 ml Acetonitril unter Feuchtigkeitsausschluss zugetropft. Das Reaktionsgemisch wurde 8 Stunden unter Rückflusstemperatur gerührt, danach wurde das Lösungsmittel und überschüssiges Reagens abdestilliert. Der Rückstand wurde in Methylenchlorid aufgenommen und mit Wasser, gesättigter Natriumhydrogencarbonatlösung und gesättigter Kochsalzlösung gewaschen. Die Lösung wurde eingeengt und über eine kurze Kieselgelsäule mit Methylenchlorid als Elutionsmittel gegeben. Das Eluat wurde eingeengt, der Rückstand aus Aethanol/Hexan umkristallisiert. Man erhielt 8,5 g 4,6-Dichlor-5-(o-methoxyphenoxy)pyrimidin, Smp. 79-80°C.
f) 0,8 g 4,6-Dichlor-(o-methoxyphenoxy)pyrimidin und 1,5 g p-t-Butylsulfonamid-K in 3 ml trockenem Dimethylsulfoxid wurden unter Argon 1,5 Stunden auf 120°C erwärmt. Danach wurde das Dimethylsulfoxid abdestilliert, der Rückstand zwischen Aethylacetat und 1N Salzsäure verteilt und die organische Phase neutral gewaschen. Die organische Phase wurde getrocknet, das Lösungsmittel abgedampft und der Rückstand mit 3 ml Methanol versetzt. Man erhielt 950 mg p-t-Butyl-N-[6-chlor-5-(o-methoxyphenoxy)4-pyrimidinyl]benzolsulfonamid, Smp. 152°C.

### Beispiel 2

In Analogie zu Beispiel 1, Abschnitt a) wurde aus p-Isopropyl-N-[6-chlor-5-(o-methoxyphenoxy)-4-pyrimidinyl]benzolsulfonamid das N-[6-(Hydroxy-äthoxy)-5-(o-methoxyphenoxy)-4-pyrimidinyl]-p-isopropylbenzolsulfonamid, Smp. 142-143°C erhalten. Die Verbindung wurde in Analogie zu Beispiel 1, Abschnitt b) in nahezu quantitativer Ausbeute in das wasserlösliche Natriumsalz übergeführt. Das Ausgangsmaterial wurde in Analogie zu Beispiel 1, Abschnitt f) durch Umsetzung von 540 mg 4,6-Dichlor-5-(o-methoxyphenoxy)pyrimidin und 360 mg p-Isopropylbenzolsulfonamid-Kalium erhalten.

### Beispiel 3

In Analogie zu Beispiel 1, Abschnitt a) wurde aus N-[6-Chlor-5-(o-tolyloxy)-4-pyrimidinyl]-p-t-butylsulfonamid das p-t-Butyl-N-[6-(2-hydroxyäthoxy)-5-(o-tolyloxy)-4-pyrimidinyl]benzolsulfonamid erhalten. Smp. 190-192°C.

Das Ausgangsmaterial wurde wie folgt hergestellt:
In Analogie zu Beispiel 1, Abschnitt c) wurde Diäthylbrommalonat mit Natrium-o-kresolat zu Diäthyl-(o-tolyloxy)malonat, Sdp. 120°C/7 Pa umgesetzt.
In Analogie zu Beispiel 1, Abschnitt d) wurde aus dem vorstehenden Malonester das 5-(o-Tolyloxy)-6-hydroxy-4(3H)-pyrimidinon erhalten, aus dem in Analogie zu Beispiel 1e) das 4,6-Dichlor-(o-tolyloxy)pyrimidin, Smp. 78-79°C (Aethanol/Hexan) erhalten wurde. Umsetzung der letzteren Verbindung mit p-t-Butylsulfonamid-Kalium lieferte schliesslich das N-[6-Chlor-5-(o-tolyloxy)-4-pyrimidinyl]-p-t-butylsulfonamid.

### Beispiel 4

In Analogie zu Beispiel 1, Abschnitt a) wurde aus p-t-Butyl-N-[2-chlor-5-(o,o-chlorphenoxy)-4-pyrimidinyl]benzolsulfonamid das p-t-Butyl-N-[6-(2-hydroxyäthoxy)-5-(o-chlorphenyloxy)-4-pyrimidinyl]benzolsulfonamid, Smp. 178-179°C (aus Diisopropyläther) erhalten.

Das Ausgangsmaterial wurde wie folgt hergestellt:
In Analogie zu Beispiel 1, Abschnitt e) wurde aus Diäthylbrommalonat und Natrium-o-chlorphenolat das Diäthyl-(o-chlorphenoxy)malonat als farblose Flüssigkeit erhalten, die in Analogie zu Beispiel 1, Abschnitt d) in das 5-(o-Chlorphenoxy)-6-hydroxy-4(3H)pyrimidinon übergeführt wurde. Aus letztere Verbindung wurde in Analogie zu Beispiel 1, Abschnitt e) das 4,6-Dichlor-5-(o-chlorphenoxy)pyrimidin, Smp. 76-77°C (aus Aethanol/Hexan) erhalten und aus diesem durch Umsetzung mit p-t-Butylsulfonamid-Kalium das p-t-Butyl-N-[2-chlor-5-(o-chlorphenoxy)-4-pyrimidinyl]benzolsulfonamid, Smp. 186-187°C (aus Methanol).

### Beispiel 5

In Analogie zu Beispiel 1, Abschnitt a) wurde aus N-[6-Chlor-5-(o-chlorphenoxy)-4-pyrimidinyl]-p-isopropylbenzolsulfonamid das N-[6-(2-Hydroxy-äthoxy)-5-(o-chlorphenoxy)-4-pyrimidinyl]-p-isopropylbenzolsulfonamid, Smp. 174-175°C (aus Aethylacetat) erhalten.

Das Ausgangsmaterial wurde in Analogie zu Beispiel 1, Abschnitt f) aus 4,6-Dichlor-5-(o-chlorphenoxy)pyrimidin und p-Isopropylbenzolsulfonamid-Kalium hergestellt. Smp. 174-176°C (aus Methanol).

### Beispiel 6

In Analogie zu Beispiel 1, Abschnitt a) wurden aus p-t-Butyl-N-[6-chlor-5-(m-methoxyphenoxy)-4-pyrimidinyl]benzolsulfonamid das p-t-Butyl-N-[6-(2-hydroxyäthoxy)-5-(m-methoxyphenoxy)-4-pyrimidinyl]benzolsulfonamid, Smp. 165-167°C (aus Diisopropyläther) erhalten.

Durch Umsetzung des Sulfonamids mit 0,5N KOH in Aethanol wurde das Kaliumsalz, Smp. 213-215°C erhalten.

Das Natriumsalz wurde in Analogie zu Beispiel 1, Abschnitt b) hergestellt. Smp. 265-270°C (aus Diisopropyläther).

Das Ausgangsmaterial wurde wie folgt hergestellt:
Diäthylbrommalonat wurde in Analogie zu Beispiel 1, Abschnitt c) mit Natrium-m-methoxyphenolat zu Diäthyl-(m-methoxyphenoxy)malonat, farblose Flüssigkeit, umgesetzt. Sdp. 143°C/0,05 Torr. Der so erhaltene Malonester wurde in Analogie zu Beispiel 1, Abschnitt d) in das 5-(m-Methoxyphenoxy)-6-hydroxy-4(3H)-pyrimidinon übergeführt, aus dem in Analogie zu Beispiel 1, Abschnitt e) das 4,6-Dichlor-5-(m-methoxyphenoxy)pyrimidin hergestellt wurde, Smp. 109-110°C. Umsetzung der letztgenannten Verbindung mit p-t-Butylbenzolsulfonamid-Kalium lieferte p-t-Butyl-N-[6-chlor-5-(m-methoxyphenoxy)-4-pyrimidinyl]benzolsulfonamid, Smp. 152°C (aus Methanol).

### Beispiel 7

In Analogie zu Beispiel 1, Abschnitt a) wurde aus p-t-Butyl-N-[6-chlor-5-phenoxy-4-pyrimidinyl]benzolsulfonamid das p-t-Butyl-N-[6-(2-hydroxy-äthoxy)-5-phenoxy-4-pyrimidinyl]benzolsulfonamid, Smp. 165-167°C (aus Diisopropyläther) erhalten.

Das Ausgangsmaterial wurde wie folgt hergestellt:
Diäthylbrommalonat wurde in Analogie zu Beispiel 1, Abschnitt c) mit Natriumphenolat zum Diäthylphenoxymalonat, Sdp. 140°C/0,05 Torr umgesetzt. Aus dem Malonester wurde in Analogie zu Beispiel 1, Abschnitt e) das 5-Phenoxy-6-hydroxy-4(3H)pyrimidinon erhalten und aus diesem in Analogie zu Beispiel 1, Abschnitt e) das 4,6-Dichlor-5-phenoxypyrimidin, Smp. 89-90°C (aus Aethanol/Hexan). Umsetzung der letztgenannten Verbindung mit p-t-Butylbenzolsulfonamid-Kalium lieferte p-t-Butyl-N-[6-chlor-5-phenoxy-4-pyrimidinyl]benzolsulfonamid, Smp. 143-144°C.

### Beispiel 8

In Analogie zu Beispiel 1, Abschnitt a) wurde aus 4,6-Dichlor-5-(p-methoxyphenoxy)-4-pyrimidin das p-t-Butyl-N-[6-(2-hydroxyäthoxy)-5-(p-methoxyphenoxy)-4-pyrimidinyl]benzolsulfonamid, Smp. 141-142°C erhalten.

Das Ausgangsmaterial wurde in Analogie zu Beispiel 1, Abschnitt c), d) und e) durch Umsetzung von Diäthylbrommalonat mit Natrium-p-methoxyphenolat zu Diäthyl-p-methoxyphenoxymalonat, Sdp. 140°C/7 Pa, und weitere Umsetzung zu 5-(p-Methoxyphenoxy)-6-hydroxy-4(3H)pyrimidinon bzw. 4,6-Dichlor-5-(p-methoxyphenoxy)-4-pyrimidin, Smp. 107-108°C (aus Aethanol/Hexan) hergestellt.

### Beispiel 9

In Analogie zu Beispiel 1, Abschnitt a) wurde aus p-t-Butyl-N-[6-chlor-5-(o-äthoxyphenoxy)-4-pyrimidinyl]benzolsulfonamid das p-t-Butyl-N-[6-(2-hydroxyäthoxy)-5-(o-äthoxyphenoxy)-4-pyrimidinyl]benzolsulfonamid, Smp. 120-121°C (aus Diisopropyläther) erhalten.

Das Ausgangsmaterial wurde in Analogie zu Beispiel 1, Abschnitt c), d), e) und f) aus Dimethylchlormalonat über folgende Zwischenstufen hergestellt:
Dimethyl-(o-äthoxyphenoxy)malonat, Sdp. 150°C/7 Pa,
5-(o-Aethoxyphenoxy)-6-hydroxy-4(3H)pyrimidinon,
4,6-Dichlor-5-(o-äthoxyphenoxy)-4-pyrimidin,
5-p-t-Butyl-N-[6-chlor-5-(o-äthoxyphenoxy)-4-pyrimidinyl]benzolsulfonamid, Smp. 162-163°C (aus Methanol).

### Beispiel 10

In Analogie zu Beispiel 1, Abschnitt a) wurde aus p-(2,2-Dimethylpropyl)-N-[6-chlor-5-(o-methoxyphenoxy)-4-pyrimidinyl]benzolsulfonamid das p-(2,2-Dimethylpropyl)-N-[6-(2-hydroxyäthoxy)-5-(o-methoxyphenoxy)-4-pyrimidinyl]benzolsulfonamid, Smp. 136-137°C (aus Diisopropyläther) erhalten. Das Ausgangsmaterial wurde in Analogie zu Beispiel 1, Abschnitt c), d) und f) über folgende Zwischenstufen hergestellt:
p-(2,2-Dimethylpropyl)benzolsulfochlorid, Sdp. 105°C/0,05 Torr.,
2,2-Dimethyl-p-(2,2-dimethylpropyl)benzolsulfonamid-Kalium,
p-(2,2-Dimethylpropyl)-N-[6-chlor-5-(o-methoxyphenoxy)-4-pyrimidinyl]-benzolsulfonamid, Smp. 164-165°C (aus Methanol).

### Beispiel 11

In Analogie zu Beispiel 1, Abschnitt a) wurde aus N-[6-Chlor-2-methyl-5-(m-methoxyphenoxy)-4-pyrimidinyl]-p-isopropylbenzolsulfonamid, Smp. 152-153°C, das p-Isopropyl-N-[6-(2-hydroxyäthoxy)-2-methyl-5-(m-methoxyphenoxy)-4-pyrimidinyl]benzolsulfonamid, Smp. 129-130°C (aus Diisopropyläther) erhalten.

Das Ausgangsmaterial wurde wie folgt hergestellt:
In Analogie zu Beispiel 1, Abschnitt e) wurde unter Verwendung von Acetamidinhydrochiorid anstelle von Formamidinacetat das Dimethyl-(m-methoxyphenoxy)malonat zum 5-(m-Methoxyphenoxy)-2-methyl-6-hydroxy-4(3H)pyrimidinon erhalten. Daraus wurde in Analogie zu Beispiel 1, Abschnitt e) das 4,6-Dichlor-2-methyl-5-(m-methoxyphenoxy)pyrimidin und daraus mit p-Isopropylbenzolsulfonamid-Kalium das N-[6-Chlor-2-methyl-5-(m-methoxyphenoxy)-4-pyrimidinyl]-p-isopropylbenzolsulfonamid hergestellt, Smp. 152-153°C (aus Methanol).

### Beispiel 12

In Analogie zu Beispiel 1, Abschnitt a) wurde aus N-[6-Chlor-5-(o-methoxy)-2-phenyl-4-pyrimidinyl]-p-isopropylbenzolsulfonamid das N-[6-(2-Hydroxyäthoxy)-5-(o-methoxyphenoxy)-2-phenyl-4-pyrimidinyl]-p-isopropylbenzolsulfonamid erhalten.

Das Ausgangsmaterial wurde in Analogie zu Beispiel 1, Abschnitt d), e) und f) aus Dimethyl-(o-methoxyphenoxy)malonat über 5-(o-Methoxy)-2-phenyl-6-hydroxy-4(3H)-pyrimidinon,
4,6-Dichlor-2-phenyl-5-(o-methoxyphenoxy)pyrimidin, Smp. 135-136°C und N-[6-Chlor-5-(o-methoxy)-2-phenyl-4-pyrimidinyl]-p-isopropylbenzolsulfonamid, Smp. 190-191°C (aus Methanol) hergestellt.

### Beispiel 13

Zu 780 mg Benzyltriphenylphosphoniumchlorid in 10 ml abs. Tetrahydrofuran wurden bei -20°C 1,3 ml 1,6M Butyllithium in Hexan gegeben. Das Reaktionsgemisch wurde 15 Minuten bei -20°C gerührt und danach mit 280 mg 2-[(5-Formyl-6-p-toluolsulfonamid-4-pyrimidinyl)oxy]äthylacetat versetzt. Das Reaktionsgemisch wurde auf Raumtemperatur aufwärmen gelassen und 2 Stunden bei Raumtemperatur gerührt. Das Tetrahydrofuran wurde unter vermindertem Druck abdestilliert, der Rückstand in Aethylacetat gelöst und die organische Phase mit Wasser und gesättigter Natriumchloridlösung gewaschen, getrocknet und eingedampft. Der Rückstand wurde über Silicagel mit Methylenchlorid/Aethylacetat (9:1 und 8:2) chromatographiert. Man erhielt 160 mg 2-[[5-[(E/Z)-Styryl]-6-p-toluolsulfonamid-4-pyrimidinyl]oxy]äthylacetat, Smp. 146-156°C.

Das Ausgangsmaterial wurde wie folgt hergestellt:
Aus 5-Allyl-4,6-dichlorpyrimidin-p-toluolsulfonamid-Kalium wurde N-[6-Chlor-5-[(E/Z)-propenyl]-4-pyrimidinyl]-p-toluolsulfonamid und daraus durch Umsetzung mit Aethylenglykol-Natrium das N-[6-(2-Hydroxyäthoxy)-5-[(E/Z)-propenyl]-4-pyrimidinyl]-p-toluolsulfonamid, Smp. 130-132°C hergestellt. Umsetzung mit Acetanhydrid in Gegenwart von Pyridin in Tetrahydrofuran lieferte 2-[[5-[(E/Z)-Propenyl]-6-p-toluolsulfonamid-4-pyrimidinyl]oxy]äthylacetat, Smp. 160-163°C.
390 mg der vorstehend genannten Verbindung und 8 mg Osmiumtetroxid wurden zu einem Gemisch von 2,5 ml Wasser und 7 ml Dioxan gegeben und dann innerhalb von 30 Minuten bei Raumtemperatur 450 mg Natrium-m-perjodat und nach 2 Stunden Rühren bei Raumtemperatur nochmals 8 mg Osmiumtetroxid. Das Reaktionsgemisch wurde weitere 5 Stunden gerührt und aufgearbeitet, wobei 2-[(5-Formyl-6-p-toluolsulfonamid-4-pyrimidinyl)pxy]äthylacetat, Smp. 130-144°C (nach Kristallisation aus Aethylacetat und Diäthyläther) erhalten wurde.

### Beispiel 14

120 mg 2-[[5[(E/Z)-Styryl]-6-p-toluolsulfonamid-4-pyrimidinyl]oxy]äthylacetat wurden in 3 ml abs. Aethanol und 3 ml abs. Tetrahydrofuran in Gegenwart von 4 mg 5% Palladiumkohle hydriert. Nach Beendigung der Wasserstoffaufnahme wurde der Katalysator abfiltriert und die organischen Phasen unter vermindertem Druck eingedampft. Der Rückstand wurde an Silicagel mit Aethylacetat chromatographiert und lieferte 110 mg 2-[[5-Phenäthyl-6-p-toluolsulfonamid-4-pyrimidinyl]oxy]äthylacetat, Smp. 120-123°C.

### Beispiel 15

80 mg 2-[(5-Phenäthyl-6-p-toluolsulfonamido-4-pyrimidinyl)oxy]äthylacetat wurden 15 Stunden bei 20°C in 5 ml Methanol mit 53 mg fein pulverisiertem Kaliumcarbonat gerührt. Danach wurde das Methanol unter vermindertem Druck entfernt, der Rückstand in Aethylacetat aufgenommen, die organische Phase mit Wasser und gesättigter Natriumchloridlösung gewaschen, getrocknet und eingedampft. Der Rückstand wurde über Silicagel mit Methylenchlorid/Aethylacetat (1:1) und Aethylacetat chromatographiert. Man erhielt 40 mg N-[6-(2-Hydroxyäthoxy)-5-phenäthyl-4-pyrimidinyl]-p-toluolsulfonamid als weisses Harz.

### Beispiel 16

In Analogie zu Beispiel 15 wurde aus 2-[[5-[(E/Z)-Styryl]-6-p-toluolsulfonamido]-4-pyrimidinyl]oxy]äthylacetat das N-[6-(2-Hydroxyäthoxy)-5-[(E/Z)-styryl]-4-pyrimidinyl]-p-toluolsulfonamid als weisses Harz erhalten.

### Beispiel 17

In Analogie zu Beispiel 1, Abschnitt a) wurde aus N-[6-Chlor-5-(2,4,6-trichlorphenoxy)-4-pyrimidinyl]-p-isopropylbenzolsulfonamid und Aethylenglykol-Na das N-[6-(2-Hydroxyäthoxy)-5-(2,4,6-trichlorphenoxy)-4-pyrimidinyl]-p-isopropylbenzolsulfonamid, Smp. 182-183°C (aus Methylenchlorid und Isopropyläther) erhalten.

Das Ausgangsmaterial wurde aus 4,6-Dichlor-5-(2,4,6-trichlorphenoxy)pyrimidin und p-Isopropylbenzolsulfonamid hergestellt, Smp. 217-218°C (aus Methylenchlorid und Isopropyläther).

### Beispiel 18

In Analogie zu Beispiel 1, Abschnitt a) wurde aus N-[6-Chlor-5-(2,4,6-trichlorphenoxy)-4-pyrimidinyl]-o-toluolsulfonamid und Aethylenglykol-Na das N-[6-(2-Hydroxyäthoxy)-6-(2,4,6-trichlorphenoxy)-4-pyrimidinyl]-o-toluolsulfonamid, Smp. 144-145°C (aus Isopropyläther) erhalten.

Das Ausgangsmaterial wurde aus 4,6-Dichlor-5-(2,4,6-trichlorphenoxy)pyrimidin und o-Toluolsulfonamid hergestellt, Smp. 107-109°C (aus Isopropyläther).

### Beispiel 19

In Analogie zu Beispiel 1, Abschnitt a) wurde aus N-[6-Chlor-5-(2,4,6-trichlorphenoxy)-4-pyrimidinyl]-2,4-xylolsulfonamid und Aethylenglykol-Na das N-[6-(2-Hydroxyäthoxy)-5-(2,4,6-trichlorphenoxy)-4-pyrimidinyl]-2,4-xylolsulfonamid, Smp. 157-158°C (aus Isopropyläther) erhalten.

Das Ausgangsmaterial wurde wie folgt hergestellt:
In einer Lösung von 18,0 g 2,4,6-Trichlorphenol und 32,0 g Diäthylbrommalonat in 180 ml Aceton und 20 ml Toluol wurden 16,9 g wasserfreies K₂CO₃ gegeben. Das Reaktionsgemisch wurde 24 Stunden unter Rühren am Rückfluss erhitzt, die Lösung vom Niederschlag abfiltriert und unter vermindertem Druck eingedampft. Der Rückstand wurde in Toluol aufgenommen, die organische Lösung mit einer 5% Natriumcarbonatlösung, dann mit Wasser gewaschen, über Natriumsulfat getrocknet und nach dem Abnutschen des Salzes unter vermindertem Druck eingedampft. Der Rückstand wurde unter ≤ 1 mmHg Druck destilliert, wobei ein farbloses Oel (Sdp. 171-174°C) erhalten wurde, aus dem mit Formamidinacetat und Natriummethylat das 5-(2,4,6-Trichlorphenoxy)-4,6(3H,5H)-pyrimidindion, Smp. >270°C, erhalten wurde, das vor der weiteren Umsetzung bei 80°C über Nacht unter vermindertem Druck getrocknet wurde.
Eine Lösung aus 7,6 g 5-(2,4,6-Trichlorphenoxy)-4,6(3H,5H)-pyrimidindion, 6,6 g Tetraäthylammoniumchlorid, 3,3 ml Collidin, 13,7 ml POCl₃ in 70 ml CH₃CN wurde 4,5 Stunden am Rückfluss erhitzt, die Lösung unter vermindertem Druck eingedampft, der Rückstand dreimal mit Aether behandelt, die vereinigten organischen Lösungen über Nacht filtriert, unter vermindertem Druck eingedampft und der Rückstand aus Aether und n-Hexan umkristallisiert. Man erhielt 4,6-Dichlor-5-(2,4,6-trichlorphenoxy)pyrimidin, Smp. 104-105°C.
Aus 4,6-Dichlor-5-(2,4,6-trichlorphenoxy)pyrimidin und 2,4-Xylolsulfonamid wurde das N-[6-Chlor-5-(2,4,6-trichlorphenoxy)-4-pyrimidinyl]-2,4-xylolsulfonamid, Smp. 267°C (aus Acetonitril und Isopropyläther) erhalten.

### Beispiel 20

Durch Umsetzung von 4,6-Dichlor-5-[(2-methoxy-4-methyl)phenoxy]pyrimidin mit p-t-Butylbenzolsulfonamid und danach mit Aethylenglykol-Na wurde das p-t-Butyl-N-[6-(2-hydroxyäthoxy)-5-[(2-methoxy-p-tolyl)oxy]-4-pyrimidinyl]benzolsulfonamid als Feststoff erhalten.

Das Ausgangsmaterial wurde durch Umsetzung von Methylguajakol mit Diäthylbrommalonat und danach mit Formamidinacetat zu 5-[(2-Methoxy-4-methyl)phenoxy]-4,6(3H,4H)-pyrimidindion, Smp. 234-236°C, und weitere Umsetzung der letzteren Verbindung mit POCl₃ hergestellt.

### Beispiel 21

Durch Umsetzung von 4,6-Dichlor-5-[(2-methoxy-4-methyl)phenoxy]pyrimidin mit p-Isopropylbenzolsulfonamid und danach mit Aethylenglykol-Na wurde das N-[5-(2-Methoxy-4-methyl)phenoxy]pyrimidinyl]-p-isopropylbenzolsulfonamid, Smp. 135-136°C (aus Aethylacetat) erhalten.

### Beispiel 22

Durch Umsetzung von 4,6-Dichlor-5-[(2-methoxy-4-methyl)phenoxy]pyrimidin mit o-Aethylbenzolsulfonamid und danach mit Aethylenglykol-Na wurde das N-[5-(2-Methoxy-4-methyl)phenoxy-6-(2-hydroxyäthoxy)-4-pyrimidinyl]-o-äthylbenzolsulfonamid als Feststoff erhalten.

### Beispiel 23

Durch Umsetzung von 4,6-Dichlor-5-(2-methoxy)phenoxy-2-methylpyrimidin mit p-tert-Butylphenylsulfonamid und danach mit Aethylenglykol-Na wurde das p-tert-Butyl-N-[6-(2-hydroxyäthoxy)-5-(o-methoxyphenoxy)-2-methyl-4-pyrimidinyl]benzolsulfonamid, Smp. 123-124°C (aus Essigester) erhalten.

### Beispiel 24

Durch Umsetzung von 4,6-Dichlor-5-(2-methoxy)phenoxy-2-methylpyrimidin mit p-Isopropylbenzolsulfonamid und danach mit Aethylenglykol-Na wurde das N-[6-(2-Hydroxyäthoxy)-5-(o-methoxyphenoxy)-2-methyl-4-pyrimidinyl)-p-isopropylbenzolsulfonamid, Smp. 124-126°C (aus Acetonitril, Isopropanol und Wasser) erhalten.

### Beispiel 25

Durch Umsetzung von 4,6-Dichlor-5-(2-methoxyphenoxy)-2-trifluormethylpyrimidin, p-Isopropylbenzolsulfonamid und danach mit Aethylenglykol-Na wurde das N-[6-(2-Hydroxyäthoxy)-5-(o-methoxyphenoxy)-(2-trifluormethyl)-4-pyrimidinyl]-p-isopropylbenzolsulfonamid als Feststoff erhalten.

### Beispiel 26

Durch Umsetzung von 4,6-Dichlor-5-(2-methoxyphenoxy)-2-trifluormethylpyrimidin mit p-tert-Butylbenzolsulfonamid und mit Aethylenglykol-Na wurde das p-tert-Butyl-N-[6-(2-hydroxyäthoxy)-5-(o-methoxyphenoxy)-2-(trifluormethyl)-4-pyrimidinyl]benzolsulfonamid, Smp. 190-192°C (aus Toluol) erhalten. Natriumsalz: Smp. 288-289°C.

### Beispiel 27

Durch Umsetzung von 5-(1,3-Benzodioxol-5-yloxy)-4,6-dichlorpyrimidin mit p-tert-Butylphenylsulfonamid, und danach mit Aethylenglykol-Na wurde das N-[5-(1,3-Benzodioxol-5-yloxy)-6-(2-hydroxyäthoxy)-4-pyrimidinyl]-p-tert-butylbenzolsulfonamid als Feststoff erhalten.

### Beispiel 28

Durch Umsetzung von 5-(1,3-Benzodioxol-5-yloxy)-4,6-dichlorpyrimidin mit p-Isopropylbenzolsulfonamid und danach mit Aethylenglykol-Na wurde das N-[5-(1,3-Benzodioxol-5-yloxy)-6-(2-hydroxyäthoxy)-4-pyrimidinyl]-p-isopropylbenzolsulfonamid als Feststoff erhalten.

### Beispiel 29

Durch Umsetzung von 5-(2-Methoxyphenoxy)-4,6-dichlorpyrimidin mit o-Methoxyphenylsulfonamid und danach mit Aethylenglykol-Na wurde das N-[6-(2-Hydroxyäthoxy)-5-(o-methoxyphenoxy)-4-pyrimidinyl]-o-methoxybenzolsulfonamid, Smp. 164-165°C (aus Essigester) erhalten.

### Beispiel 30

Durch Umsetzung von p-tert-Butyl-N-[6-chlor-5-(o-methoxyphenoxy)-2-methyl-4-pyrimidinyl]benzolsulfonamid mit dem Mononatriumsalz von 1,4-Butandiol wurde das p-tert-Buty-N-[6-(4-hydroxybutoxy)-5-(o-methoxyphenoxy)-2-methyl-4-pyrimidinyl]benzolsulfonamid als weisser Schaum erhalten.

### Beispiel 31

Durch Umsetzung von 4,6-Dichlor-5-(2-naphthyloxy)pyrimidin und p-Isopropylphenylsulfonamid und, danach dem Natriumsalz von Aethylenglykol N-[6-(2-Hydroxyäthoxy)-5-(2-naphthyloxy)-4-pyrimidinyl]-p-isopropylbenzolsulfonamid, Smp. 160-161°C (aus Isopropyläther) erhalten.

### Beispiel 32

Durch Umsetzung von 4,6-Dichlor-5-(2-naphthyloxy)pyrimidin mit p-tert-Butylphenylsulfonamid und danach mit Aethylenglykol-Na wurde das N-[6-(2-Hydroxyäthoxy)-5-(2-naphthyloxy)-4-pyrimidinyl]-p-tert-butylbenzolsulfonamid, Smp. 197-198°C (aus Isopropyläther) erhalten.

### Beispiel 33

Durch Umsetzung von 4,6-Dichlor-5-(o-methoxyphenoxy)-2-propylpyrimidin mit p-Isopropylphenylsulfonamid und danach mit Aethylenglykol-Na wurde das N-[6-(2-Hydroxyäthoxy)-5-(o-methoxyphenoxy)-2-propyl-4-pyrimidinyl]-p-isopropylbenzolsulfonamid als Feststoff erhalten.

### Beispiel 34

Durch Umsetzung von 4,6-Dichlor-5-(o-methoxyphenoxy)-2-propylpyrimidin mit p-tert-Butylphenylsulfonamid und danach mit Aethylenglykol-Na wurde das p-tert-Butyl-N-[6-(2-hydroxyäthoxy)-5-(o-methoxyphenoxy)-2-propyl-4-pyrimidinyl]benzolsulfonamid erhalten.

### Beispiel 35

Durch Umsetzung von 4,6-Dichlor-5-(o-methoxy)phenoxy-2-methylpyrimidin mit α,α,α,-Trifluor-p-toluolsulfonamid und danach mit Aethylenglykol-Na wurde das α,α,α-Trifluor-N-[6-(2-hydroxyäthoxy)-5-(o-methoxyphenoxy)-2-methyl-4-pyrimidinyl]-p-toluolsulfonamid, Smp. 144-145°C (aus Essigester) erhalten.

### Beispiel 36

Durch Umsetzung von 4,6-Dichlor-5-(o-methoxy)phenoxy-2-methylpyrimidin mit p-Chlorphenylsulfonamid und danach mit Aethylenglykol-Na wurde das p-Chlor-N-[6-(2-hydroxyäthoxy)-5-(o-methoxyphenoxy)-2-methyl-4-pyrimidinyl]benzolsulfonamid, Smp. 134-135°C (aus Essigester) erhalten.

### Beispiel 37

Durch Umsetzung von 4,6-Dichlor-5-(o-methoxy)phenoxy-2-methylpyrimidin mit p-(Trifluormethoxy)benzolsulfonamid und danach mit Aethylenglykol-Na wurde das N-[6-(2-Hydroxyäthoxy)-5-(o-methoxyphenoxy)-2-methyl-4-pyrimidinyl]-p-(trifluormethoxy)benzolsulfonamid, Smp. 138-140°C (aus Essigester) erhalten.

### Beispiel 38

Durch Umsetzung von 4,6-Dichlor-5-(o-methoxy)phenoxy-2-methylpyrimidin mit o-Aethylbenzolsulfonamid und danach mit Aethylenglykol-Na wurde das o-Aethyl-N-[6-(2-hydroxyäthoxy)-5-(o-methoxyphenoxy)-2-methyl-4-pyrimidinyl]benzolsulfonamid als weisser Schaum erhalten.

### Beispiel 39

Durch Umsetzung von 4,6-Dichlor-5-(o-methoxy)phenoxy-2-methylpyrimidin mit p-Toluolsulfonamid und danach mit Aethylenglykol-Na wurde das N-[6-(2-Hydroxyäthoxy)-5-(o-methoxyphenoxy)-2-methyl-4-pyrimidinyl]-p-toluolsulfonamid als weisser Schaum erhalten.

### Beispiel 40

Durch Umsetzung von 4,6-Dichlor-5-(o-methoxy)phenoxy-2-methylpyrimidin mit 2-Naphthylsulfonamid und danach mit Aethylenglykol-Na wurde das N-[6-(2-Hydroxyäthoxy)-5-(o-methoxyphenoxy)-2-methyl-4-pyrimidinyl]-2-naphthylsulfonamid als Schaum erhalten.

### Beispiel 41

Durch Umsetzung von p-tert-Butyl-N-[6-chlor-5-(o-methoxyphenoxy)-2-methyl-4-pyrimidinyl]benzolsulfonamid und dem Mononatriumsalz von 1,3-Propandiol wurde das p-tert-Butyl-N-[6-(3-hydroxypropoxy)-5-(o-methoxy-phenoxy)-2-methyl-4-pyrimidinyl]benzolsulfonamid als weisser Schaum erhalten.

### Beispiel 42

In Analogie zu Beispiel 1, Abschnitt a) wurden 300 mg p-t-Butyl-N-[6-chlor.5.[(o-methylthio)phenoxy]-4-pyrimidinyl]benzolsulfonamid zu p-t-Butyl-N-[6-(2-hydroxyäthoxy)-5-[(o-methylthio)phenoxy]-4-pyrimidinyl]benzolsulfonamid umgesetzt. Man erhielt 250 mg p-t-Butyl-N-[6-(2-hydroxyäthoxy)-5-[(o-methylthio)phenoxy]-4-pyrimidinyl]benzolsulfonamid, Smp. 149-150°C.

Das Ausgangsmaterial wurde wie folgt hergestellt:
a) Man erhielt (o-Methylthio)phenoxymalonsäuredimethylester aus Chlormalonsäuredimethylester und (o-Methylthio)phenol in Analogie zu Beispiel 1, Abschnitt c). Aus 17 g (o-Methylthio)phenol erhielt man 23 g Malonat aus Toluol-Hexan.
b) In Analogie zu Beispiel 1, Abschnitt d) erhielt man 9,15 g 5-[(o-Methylthio)phenoxy]-6-hydroxy-4(3H)-pyrimidinon, MS: 250 (M), aus 13,5 g des Malonats von a) und Formamidinacetat.
c) 2,5 g dieser Verbindung und 2,9 g Diisopropyläthylamin wurden in 15 ml Acetonitril suspendiert. Zu der Suspension wurden 2 ml POCl₃ getropft und anschliessend wurde das Gemisch während 5 Stunden am Rückfluss gekocht. Das 4,6-Dichlor-5-[(o-methylthio)phenoxy]pyrimidin wurde erhalten nach Aufarbeitung in Analogie zu Beispiel 1, Abschnitt e). Nach Kristallisation aus n-Hexan erhielt man 1 g Pyrimidinderivat, Smp. 89-90°C.
d) In Analogie zu Beispiel 1, Abschnitt f) wurden 580 mg 4,6-Dichlor-5-[(o-methylthio)phenoxy]pyrimidin mit 850 mg p-t-Butylbenzolsulfonamid-K zu p-t-Butyl-N-[6-chlor-5-[(o-methylthio)phenoxy]-4-pyrimidinyl]benzolsulfonamid umgesetzt. Nach Kristallisation aus MeOH, erhielt man 480 mg weisse Kristalle, Smp. 154-155°C.

### Beispiel 43

a) In Analogie zu Beispiel 1, Abschnitt a) wurden 350 mg p-t-Butyl-N-[6-chlor-5-(o-methoxyphenoxy)-2-phenyl-4-pyrimidinyl]benzolsulfonamid in p-t-Butyl-N-[6-(2-hydroxyäthoxy)-5-(o-methoxyphenoxy)-2-phenyl-4-pyrimidinyl]benzolsulfonamid überführt. Man erhielt 330 mg weisse Kristalle nach Kristallisation aus Diisopropyläther, Smp. 160-161°C.
b) 225 mg dieser Verbindung wurden in EtOH gelöst. Zu der Lösung wurde die stöchiometrische Menge KOH in MeOH zugefügt. Dann wurde das Lösungsmittelgemisch abdestilliert und zum Rückstand gab man Diisopropyläther, wobei das p-t-Butyl-N-[6-(2-hydroxyäthoxy)-5-(o-methoxyphenoxy)-2-phenyl-4-pyrimidinyl]benzolsulfonamid-Kalium erhalten wurde, MS: 588 [(M+K)⁺].

### Beispiel 44

In Analogie zu Beispiel 1, Abschnitt a) wurde aus N-[2-Amino-6-chlor-5-(o-methoxyphenoxy)-4-pyrimidinyl]-p-tert.-butylbenzolsulfonamid das N-[2-Amino-6-(2-hydroxyäthoxy)-5-(o-methoxyphenoxy)-4-pyrimidinyl]-p-tert.-butylbenzolsulfonamid, weisse Kristalle vom Smp. 168°C (aus Diisopropyläther) erhalten.

Das Ausgangsmaterial wurde wie folgt hergestellt:
a) Zu einer Lösung von 2,3 g Na in 100 ml Methanol gab man 7,65 g (5-o-Methoxy)phenoxymalonsäure-dimethylester und 3 g Guanidin-hydrochlorid. Die Suspension wurde während 3 Stunden bei Raumtemperatur unter Argon gerührt. Dann wurde das Methanol abdestilliert und der Rückstand in H₂O aufgenommen. Nach der üblichen Behandlung, wie bereits beschrieben, wurde die Verbindung gefällt, indem Essigsäure zugetropft wurde , bis das pH der Lösung 4,5 erreichte. Man erhielt 6,4 g Rohprodukt, von dem 1,35 g in 10 ml Dioxan suspendiert wurden. Man gab nacheinander 1,4 g N-Aethyldiisopropylamin, 2 ml POCl₃ und 1 g Triäthylbenzylammoniumchlorid zu. Das Gemisch wurde am Rückfluss bei kräftigem Rühren, unter Argonatmosphäre gekocht. Nach 30 Minuten wurde das Lösungsmittelgemisch abdestilliert, der Rückstand in Aethylacetat aufgenommen und mit H₂O und gesättigter NaHCO₃-Lösung ausgeschüttelt. Zur Reinigung wurde an Kieselgel chromatographiert (CH₂Cl₂-Aethylacetat, 9:1 Vol., als Fliessmittel). Man erhielt 2-Amino-4,6-dichlor-5-(o-methoxyphenoxy)pyrimidin als farblosen Feststoff. Smp. 190°C.
b) 0,5 g der vorstehenden Dichlor-Verbindung und 0,75 g p-tert.-Butylbenzolsulfonamid-Na in 2 ml DMSO wurden bei 90°C zu N-[2-Amino-6-chlor-5-(o-methoxyphenoxy)-4-pyrimidinyl]-p-tert-butylbenzolsulfonamid, Smp. 194-195°C, umgesetzt.

### Beispiel 45

a) In Analogie zu Beispiel 1, Abschnitt a) wurden 478 mg p-tert-Butyl-N-[6-chlor-2-methyl-5-[o-(methylthio)phenoxy]pyrimidinyl]benzolsulfonamid und Na-Glykolat in Aethylenglykol zu p-tert-Butyl-N-[6-(2-hydroxyäthoxy)-2-methyl-5-[o-(methylthio)phenoxy]-4-pyrimidinyl]benzolsulfonamid, Smp. 166-167°C, umgesetzt.
b) 225 mg dieser Verbindung wurden durch Zugabe der stöchiometrischen Menge wassriger NaOH in das Sulfonamidsalz übergeführt. Dann wurde mit Methanol verdünnt, wobei eine homogene Lösung erhalten wurde. Zu dieser Lösung wurden 100 mg NaJO₄, in 2 ml H₂O gelöst, gegeben und das Gemisch wurde bei Raumtemperatur während 8 Stunden gerührt. Dann wurde zur Trockene eingedampft. Der Rückstand wurde zwischen Aethylacetat und wässriger 0,1N H₂SO₄ verteilt. Nach Eindampfen der organischen Phase wurde das p-tert-Butyl-N-[6-(2-hydroxyäthoxy)-2-methyl-5-[o-(R,S-methylsulfinyl)phenoxy]-4-pyrimidinyl]benzolsulfonamid aus Diisopropyläther kristallisiert. Man erhielt 150 mg weisse Kristalle. MS: m/e = 520 (M+H)⁺.

Das Ausgangsmaterial wurde wie folgt hergestellt:
5,4 g o-(Methylthio)phenoxy-malonsäure-dimethylester und 2,1 g Acetamidin-hydrochlorid wurden zu 6-Hydroxy-2-methyl-5-[o-(methylthio)phenoxy]-4(3H)-pyrimidin umgesetzt und dieses in das 4,6-Dichlor-2-methyl-5-[o-(methylthiophenoxy)-pyrimidin, Smp. 132-133°C, übergeführt.
0,9 g der vorstehenden Dichlor-Verbindung und 1,3 g p-tert-Butylbenzolsulfonamid-K wurden zu p-tert-Butyl-N-[6-chlor-2-methyl-5-[o-(methylthio)phenoxy]-4-pyrimidinyl]benzolsulfonamid. Smp. 162-163°C umgesetzt.

### Beispiel 46

Zu einer Suspension von 170 mg NaH in 2 ml trockenem THF tropfte man unter einer Argon-Atmosphäre 1,22 g (S)-1,2-di-O-Isopropyliden-glycerol. Anschliessend wurden 1,03 g p-tert-Butyl-N-[6-chlor-5-(o-methoxyphenoxy)-2-(p-methoxyphenyl)-4-pyrimidinyl]benzolsulfonamid und 2 ml DMSO zugegeben. Man liess während 4 Stunden bei 95°C reagieren, wobei das THF abdestillierte. Dann wurde 0,5 ml H₂O zugefügt und das Lösungsmittelgemisch und der Ueberschuss an Reagenz wurden unter vermindertem Druck abdestilliert. Der Rückstand wurde in 20 ml Dioxan aufgenommen; 1 ml wässrige 1N HCl wurde zugefügt und man liess bei 65°C während 45 Minuten reagieren. Dann wurde zur Trockene eingedampft. Der Rückstand wurde zwischen Aethylacetat und 1N Salzsäure verteilt. Nach der üblichen Aufarbeitung wurde die Verbindung an Kieselgel gereinigt, mit Aethylacetat als Fliessmittel. Man erhielt 0,98 g (S)-4-tert-Butyl-N-[6-(2,3-dihydroxy-propyloxy)-5-(2-methoxy-phenoxy)-2-(4-methoxyphenyl)-pyrimidin-4-yl]-benzolsulfonamid, Smp. 141-142°C (aus Diäthyläther).

Das Ausgangsmaterial wurde wie folgt hergestellt:
In Analogie zu Beispiel 1, Abschnitt d) wurden 7,63 g Dimethyl-(o-methoxyphenoxy)malonat und 5,6 g p-Methoxy-benzamidin-Hydrochlorid zu 2-p-Methoxyphenyl-5-o-methoxyphenoxy-6-hydroxy-4(3H)-pyrimidinon kondensiert. Umsetzung dieser Verbindung in Analogie zu Beispiel 1, Abschnitt e), lieferte 4,6-Dichlor-2-p-methoxyphenyl-5-o-methoxyphenoxy-pyrimidin, Smp. 113-114°C, aus dem in Analogie zu Beispiel 1, Abschnitt f), das p-tert-Butyl-N-[6-chlor-5-(o-methoxyphenoxy)-2-(p-methoxyphenyl)-4-pyrimidinyl]benzolsulfonamid, Smp. 221-222°C erhalten wurde.

### Beispiel 47

210 mg 4-tert-Butyl-N-[5-(2-chlor-5-methoxy-phenoxy)-2-äthyl-6-(2-methylsulfanyl-äthoxy)-pyrimidin-4-yl]-benzolsulfonamid wurden in 5 ml MeOH und 0,2 ml 1N NaOH gelöst. Dazu gab man 95 mg NaJO₄ in 0,5 ml H₂O gelöst und es wurde während 5 Stunden bei Raumtemperatur gerührt, wobei eine Suspension entstand. Dann wurden 0,2 ml 1N HCl zugegeben und anschliessend zur Trockene eingedampft. Der Rückstand wurde zwischen Aethylacetat und 0,1N HCl verteilt und wie üblich aufgearbeitet. Zur Reinigung wurde die Verbindung an Kieselgel chromatographiert, mit Aethylacetat-MeOH (6:1 in Vol.) als Fliessmittel. Man erhielt 160 mg (RS)-4-tert-Butyl-N-[5-(2-chlor-5-methoxy-phenoxy)-2-äthyl-6-(2-methylsulfinyl-äthoxy)- pyrimidin-4-yl]-benzolsulfonamid als weisses Pulver. MS: 581 (M).

Das Ausgangsmaterial wurde wie folgt hergestellt:
In Analogie zu Beispiel 1, Abschnitt c), erhielt man aus 2-Chlor-5-methoxy-phenol und Chlormalonsäuredimethylester das Dimethyl-(2-chlor-5-methoxy-phenoxy)malonat, Smp. 68-69°C. Kondensation mit Propamidin-Hydrochlorid lieferte 2-Aethyl-5-(2-chlor-5-methoxy-phenoxy)-6-hydroxy-4(3H)-pyrimidinon, aus dem in Analogie zu Beispiel 1, Abschnitt e) das 4,6-Dichlor-2-äthyl-5-(2-chlor-5-methoxy-phenoxy)-pyrimdin. Smp. 113-113,5°C, erhalten wurde. Diese Verbindung wurde analog zu Beispiel 1, Abschnitt f) in das 4-tert-Butyl-N-[6-chlor-5-(2-chlor-5-methoxy-phenoxy)-2-äthylpyrimidin-4-yl]-benzolsulfonamid, Smp. 142-143°C (aus Aethanol) übergeführt.
Zu einer Suspension von 63 mg NaH in trockenem THF wurden 300 mg 2-(Methylthio)äthanol unter Argon getropft. Anschliessend wurden 300 mg des vorstehend erhaltenen Sulfonamids und 1 ml 1,3-Dimethyl-3,4,5,6-tetrahydro-2(1H)-pyrimidinon zugefügt. Man liess während 3 Stunden bei 80°C reagieren. Nach der üblichen Aufarbeitung des Reaktionsgutes und Reinigung an Kieselgel (CH₂Cl₂-Diäthyläther, 95/5 Vol, als Fliessmittel) erhielt man 160 mg 4-tert-Butyl-N-[5-(2-chlor-5-methoxy-phenoxy)-2-äthyl-6-(2-methylsulfanyl-äthoxy)-pyrimidin-4-yl]-benzolsulfonamid als weisses Pulver.

### Beispiel 48

a) In Analogie zu Beispiel 1 wurde aus 4-tert-Butyl-N-[6-chlor-5-(2-chlor-5-methoxy-phenoxy)-2-methylpyrimidin-4-yl]-benzolsulfonamid das 4-tert-Butyl-N-[5-(2-chlor-5-methoxy-phenoxy)-6-(2-hydroxy-äthoxy)-2-methylpyrimidin-4-yl]-benzolsulfonamid hergestellt. Aus 500 mg der Ausgangsverbindung erhielt man 430 mg weisse Kristalle. Smp. 141-141,5°C (aus Isopropyläther).
b) 140 mg der erhaltenen Verbindung wurden mit 3-Furancarbonsäure unter folgenden Bedingungen verestert: Man löst 140 mg des vorstehend erhaltenen Sulfonamids, 170 mg N-Aethyl-N'-(3-dimethylaminopropyl)carbodiimid-Hydrochlorid, 170 mg Et₃N und 5 mg Dimethylaminopyridin in 2 ml Dichlormethan und liess die Lösung 24 Stunden bei Raumtemperatur stehen. Dann gab man 5 ml THF und 1 ml H₂O zu und rührte die Lösung 30 Minuten. Anschliessend wurde zur Trockene eingedampft. Der Rückstand wurde zwischen Dichlormethan und 1N HCl verteilt, dann dreimal mit H₂O gewaschen und wie üblich isoliert. Die Verbindung wurde an Kieselgel gereinigt, mit Dichlormethan-Diäthyläther (95:5 in Vol.) als Fliessmittel. Man erhielt 120 mg 4-tert-Butyl-N-[5-(2-chlor-5-methoxy-phenoxy)-6-(2-(3-furoyloxy)äthoxy)-2-methyl-pyrimidin-4-yl]-benzolsulfonamid.

Die Ausgangsverbindung wurde wie folgt hergestellt:
In Analogie zu Beispiel 1, Abschnitt d), wurde (2-Chlor-5-methoxy-phenoxy)-malonsäure-dimethylester mit Acetamidin-Hydrochlorid zu (2-Chlor-5-methoxy-phenoxy)-2-methyl-6-hydroxy-4(3H)-pyrimidinon kondensiert. Daraus wurde in Analogie zu Beispiel 1, Abschnitt e), das 4,6-Dichlor-5-(2-chlor-5-methoxy-phenoxy)-2-methyl-pyrimidin. Smp. 125-130°C und daraus in Analogie zu Beispiel 1, Abschnitt f), das 4-tert-Butyl-N-[6-chlor-5-(2-chlor-5-methoxy-phenoxy)-2-methyl-pyrimidin-4-yl]-benzolsulfonamid, Smp. 182°C (aus MeOH) erhalten.

### Beispiel 49

In Analogie zu Beispiel 47 wurden 90 mg N-[5-(2-Chlor-5-methoxy-phenoxy)-6-(2-methylsulfanyl-äthoxy)-pyrimidin-4-yl]-1,3-benzodioxol-5-sulfonamid mit NaJO₄ zu (RS)-N-[5-(2-Chlor-5-methoxy-phenoxy)-6-(2-methylsulfinyl-äthoxy)-pyrimidin-4-yl]-1,3-benzodioxol-5-sulfonamid oxidiert. Man erhielt 65 mg weisses Pulver. MS: 542,1 (M+H⁺).

Die Ausgangsverbindung wurde wie folgt hergestellt:
In Analogie zu Beispiel 1, Abschnitt d), wurde Dimethyl-(2-chlor-5-methoxy-phenoxy)malonat mit Formamidin-Acetat zu (2-Chlor-5-methoxy-phenoxy)-6-hydroxy-4(3H)-pyrimidinon kondensiert. In Analogie zu Beispiel 1, Abschnitt e), wurde daraus das 4,6-Dichlor-5-(2-chlor-2-methoxy-phenoxy)-pyrimidin, Smp. 88-89°C (aus Aethanol) erhalten.
Umsetzung von 611 mg 4,6-Dichlor-5-(2-chlor-5-methoxy-phenoxy)-pyrimidin mit 813 mg (1,3-Benzodioxol-5-sulfonamid)-K lieferte 535 mg N-[6-Chlor-5-(2-chlor-5-methoxy-phenoxy)-pyrimidin-4-yl]-1,3-benzodioxol-5-sulfonamid. Die letztgenannte Verbindung wurde, wie bei der Herstellung des Ausgangsmaterials in Beispiel 47 beschrieben, zu N-[5-(2-Chlor-5-methoxy-phenoxy)-6-(2-methylsulfanyl-äthoxy)-pyrimidin-4-yl]-1,3-benzodioxol-5-sulfonamid umgesetzt.

### Beispiel 50

Eine Lösung aus 0,11 g Natrium in 3,0 ml Aethylenglykol wurde mit 0,265 g 4-tert-Butyl-N-[6-chlor-5-(2-methoxy-phenoxy)-2-thiophen-2-yl-pyrimidin-4-yl]-benzolsulfonamid auf 110°C erwärmt, nach 4 Stunden abgekühlt, auf Eis gegossen, und mit 1M-Weinsäure auf pH 3 gestellt. Die erhaltene Suspension wurde mit Essigester extrahiert, die organischen Extrakte vereinigt, mit Wasser gewaschen, mit Natriumsulfat getrocknet und unter reduziertem Druck eingeengt. Der Rückstand wurde auf Kieselgel mit CH₂Cl₂-Essigester 9:1 chromatographiert und lieferte 4-tert-Butyl-N-[6-(2-hydroxy-äthoxy)-5-(2-methoxy-phenoxy)-2-(thiophen-2-yl)-pyrimidin-4-yl]-benzolsulfonamid als weissen Schaum. MS: M⁺ = 555.

Das Ausgangsmaterial wurde wie folgt hergestellt.
a) Eine Lösung aus 5,17 g Na in 200 ml abs. Methanol wurde mit 21,15 g Diäthyl-(o-methoxyphenoxymalonat) und 16,2 g Thiophen-2-carboxamidinhydrochlorid versetzt und die Suspension bei Raumtemperatur über Nacht gerührt und unter reduziertem Druck eingedampft. Der Rückstand wurde in 1N-NaOH aufgenommen, die alkalische Lösung mit 1N-HCl angesäuert, der Niederschlag abgenutscht, mit Wasser sorgfältig gewaschen und im Hochvakuum bei 80°C getrocknet. Das 5-(o-Methoxyphenoxy)-2-(2-thienyl)-4,6-dihydroxy-pyrimidin vom Smp. >250°C (Zers.) wurde ohne weitere Reinigung in die nächste Stufe eingesetzt.
b) Eine Suspension aus 4,6 g 5-(o-Methoxyphenoxy)-2-(2-thienyl)-4,6-dihydroxy-pyrimidin, 4,7 ml N,N-Diisopropyl-N-äthylamin und 6,4 g PCl₅ wurde während 20 Stunden am Rückfluss gekocht. Das Gemisch wurde dann unter reduziertem Druck eingedampft, der Rückstand auf Eis gegossen und mit Essigester extrahiert. Die vereinigten Extrakte wurden mit Wasser gewaschen, getrocknet und im Vakuum eingedampft. Der Rückstand wurde an Kieselgel mit Toluol chromatographiert und lieferte 4,6-Dichlor-5-(2-methoxy-phenoxy)-2-thiophen-2-yl-pyrimidin, Smp. 118-120°C.
c) Eine Lösung von 0,353 g von 4,6-Dichlor-5-(2-methoxy-phenoxy)-2-thiophen-2-yl-pyrimidin in 5 ml DMSO wurde mit 0,376 g p-tert-Butylbenzolsulfonamid während 30 Minuten auf 150°C erwärmt. Die Lösung wurde im Hochvakuum etwas eingeengt und der ölige Rückstand auf Eis gegossen, sauer (pH = 3) gestellt und die Suspension mit Essigester extrahiert. Die organischen Extrakte wurden vereinigt, mit Wasser gewaschen, über Natriumsulfat getrocknet und unter vermindertem Druck eingeengt. Der Rückstand wurde an Kieselgel mit Toluol-Essigester 9:1 chromatographlert und lieferte 4-tert-Butyl-N-[6-chlor-5-(2-methoxy-phenoxy)-2-thiophen-2-yl-pyrimidin-4-yl]-benzolsulfonamid als einen weissen Schaum.

### Beispiel 51

In analoger Weise zum Beispiel 50 wurde aus 4-tert-Butyl-N-[6-chlor-5-(2-methoxy-phenoxy)-2-(thiophen-3-yl)-pyrimidin-4-yl]-benzolsulfonamid und Aethylenglykol-Na das 4-tert-Butyl-N-[6-(2-hydroxy-äthoxy)-5-(2-methoxy-phenoxy)-2-(thiophen-3-yl)-pyrimidin-4-yl]-benzolsulfonamid, Smp. 152-153°C (aus Toluol) erhalten.

Das 4-tert-Butyl-N-[6-chlor-5-(2-methoxy-phenoxy)-2-(thiophen-3-yl)-pyrimidin-4-yl]-benzolsulfonamid (Schaum) wurde ausgehend von Thiophen-3-carboxamidin-hydrochlorid über rac.-5-(2-Methoxy-phenoxy)-2-thiophen-3-yl-3,4,5,6-tetrahydro-pyrimidin-4,6-dion (Feststoff vom Smp. >250°C) und 4,6-Dichlor-5-(2-methoxy-phenoxy)-2-thiophen-3-yl-pyrimidin (Smp. 98-99°C) hergestellt.

### Beispiel 52

In Analogie zu Beispiel 50 wurde aus 4-tert-Butyl-N-[6-chlor-2-(furan-2-yl)-5-(2-methoxy-phenoxy)-pyrimidinyl-4-yl]-benzolsulfonamid und Aethylenglykol-Na das 4-tert-Butyl-N-[2-(furan-2-yl)-6-(2-hydroxy-äthoxy)-5-(2-methoxy-phenoxy)-pyrimidin-4-yl]-benzolsulfonamid als amorpher Feststoff erhalten.

Das 4-tert-Butyl-N-[6-chlor-2-(furan-2-yl)-5-(2-methoxy-phenoxy)-pyrimidin-4-yl]-benzolsulfonamid (Schaum) wurde ausgehend von Furan-2-carboxamidin-hydrochlorid über rac.-2-(Furan-2-yl)-5-(2-methoxy-phenoxy)-pyrimidin-4,6-dion (Feststoff mit einem Zersetzungspunkt von 255-258°C) und 4,6-Dichlor-2-(furan-2-yl)-5-(2-methoxy-phenoxy)-pyrimidin hergestellt.

### Beispiel 53

In Analogie zu Beispiel 50 wurde aus 4-tert-Butyl-N-[6-chlor-2-furan-3-yl-5-(2-methoxy-phenoxy)-pyrimidin-4-yl]-benzolsulfonamid und Aethylenglykol-Na das 4-tert-Butyl-N-[2-furan-3-yl-6-(2-hydroxy-äthoxy)-5-(2-methoxy-phenoxy)-pyrimidin-4-yl]-benzolsulfonamid als Feststoff vom Smp. 120-122+C (aus Toluol/n-Hexan) erhalten.

Das 4-tert-Butyl-N-[6-chlor-2-furan-3-yl-5-(2-methoxy-phenoxy)-pyrimidin-4-yl]-benzolsulfonamid (Schaum) wurde ausgehend von Furan-3-carboxamidin-hydrochlorid über rac.-2-(Furan-3-yl)-5-(2-methoxy-phenoxy)-4,6-dioxo-1,4,5,6-tetrahydro-pyrimidin (Feststoff mit einem Smp. von mehr als 300°C mit Zersetzung), und 4,6-Dichlor-2-(furan-3-yl)-5-(2-methoxy-phenoxy)-pyrimidin hergestellt.

### Beispiel 54

In Analogie zu Beispiel 50 wurde aus 4-tert-Butyl-N-[6-chlor-5-(2-methoxy-phenoxy)-2-(pyridin-2-yl)-pyrimidin-4-yl]-benzolsulfonamid und Aethylenglykol-Na das 4-tert-Butyl-N-[6-(2-hydroxy-äthoxy)-5-(2-methoxy-phenoxy)-2-(pyridin-2-yl)-pyrimidin-4-yl]-benzolsulfonamid als Feststoff mit einem Smp. über 250°C (aus Essigester) erhalten.

Das 4-tert-Butyl-N-[6-chlor-5-(2-methoxy-phenoxy)-2-(pyridin-2-yl)-pyrimidin-4-yl]-benzolsulfonamid (Smp. 197-198°C aus Isopropyläther) wurde ausgehend von Pyridin-2-carboxamidin-hydrochlorid über 5-(2-Methoxy-phenoxy)-2-pyridin-2-yl)pyrimidin-4,6-diol und 4,6-Dichlor-5-(2-methoxy-phenoxy)-2-pyridin-2-yl)-pyrimidin, Smp. 122-123°C hergestellt.

### Beispiel 55

In Analogie zu Beispiel 50 wurde aus 4-tert-Butyl-N-[6-chlor-5-(2-methoxy.phenoxy)-2-(pyridin-4-yl)-pyrimidin-4-yl]-benzolsulfonamid und Aethylenglykol-Na das 4-tert-Butyl-N-[6-(2-hydroxy-äthoxy)-5-(2-methoxy-phenoxy)-2-pyridin-4-yl)-pyrimidin-4-yl]-benzolsulfonamid als ein Feststoff vom Smp. 166-167°C aus Aceton-Aether erhalten.

Das 4-tert-Butyl-N-[6-chlor-5-(2-methoxy-phenoxy)-2-(pyridin-4-yl)-pyrimidin-4-yl]-benzolsulfonamid-Kalium (1:1). Smp. 193-196°C aus H₂O wurde ausgehend von Pyridin-4-carboxamidin-hydrochlorid über 5-(2-Methoxy-phenoxy)-2-(pyridin-4-yl)-pyrimidin-4,6-diol und 4,6-Dichlor-5-(2-methoxy-phenoxy)-2-pyridin-4-yl)-pyrimidin, Smp. 173-176°C hergestellt.

### Beispiel 56

In Analogie zu Beispiel 50 wurde aus 4-tert-Butyl-N-[6-chlor-5-(2-methoxy-phenoxy)-2-(pyridin-3-yl)-pyrimidin-4-yl]-benzolsulfonamid und Aethylenglykol-Na das 4-tert-Butyl-N-[6-(2-hydroxy-äthoxy)-5-(2-methoxy-phenoxy)-2-pyridin-3-yl)-pyrimidin-4-yl]-benzolsulfonamid-K als Schaum erhalten. MS: (M+H)⁺ = 551,2.

Das 4-tert-Butyl-N-[6-chlor-5-(2-methoxy-phenoxy)-2-pyridin-3-yl]-pyrimidin-4-yl]-benzolsulfonamid wurde ausgehend von Pyridin-3-carboxamidin-hydrochlorid über rac-5-(2-Methoxy-phenoxy)-2-pyrimidin-3-yl-tetrahydro-1H-pyrimidin-4,6-dion und 4,6-Dichlor-5-(2-methoxy-phenoxy)-2-(pyridin-3-yl)-pyrimidin (Smp. 164-165°C) hergestellt.

### Beispiel 57

Eine Suspension aus 525 mg 4-tert-Butyl-N-[6-chlor-5-(2-methoxy-phenoxy)-2-(pyridin-2-yl)-pyrimidin-4-yl)benzolsulfonamid in 1 ml Eisessig wurde mit 2,5 ml 40% Peressigsäure versetzt und langsam zum Rückfluss erhitzt. Nach 2 Minuten wurde abgekühlt, unter reduziertem Druck eingedampft und der Rückstand wurde aus Essigester umkristallisiert. Man erhielt 2-[4-(4-tert-Butyl-phenylsulfonylamino)-6-chlor-5-(2-methoxy-phenoxy)-pyrimidin-2-yl]-pyridin-1-oxid vom Smp. 201-202°C (unter Zersetzung).

### Beispiel 58

Zu einer Lösung von 46 mg Na in reinem Aethylenglykol wurden 216 mg 2-[4-(4-tert-Butyl-phenylsulfonylamino)-6-chlor-5-(2-methoxy-phenoxy)-pyrimidin-2-yl]-pyridin-1-oxid gegeben und die langsam entstehende Lösung bei 80°C über Nacht erwärmt. Die Lösung wurde auf wässerige Essigsäure gegossen, der Niederschlag mit Essigester extrahiert, mit Aether verrieben und abgenutscht. Man erhielt 2-[4-(4-tert-Butyl-phenylsulfonylamino)-6-(2-hydroxy-äthoxy)-5-(2-methoxy-phenoxy)-pyrimidin-2-yl]-pyridin-1-oxid als amorphe Masse, die im Hochvakuum bei 40°C getrocknet wurde. MS: (M+H)⁺ = 567,4.

### Beispiel 59

In Analogie zu Beispiel 57 wurde aus 4-tert-Butyl-N-[6-chlor-5-(2-methoxy-phenoxy)-2-(pyridin-4-yl)-pyrimidinyl-4-yl]-benzolsulfonamid und Peressigsäure das 4-[4-(4-tert-Butyl-phenylsulfonylamino)-6-chlor-5-(2-methoxy-phenoxy)-pyrimidin-2-yl]-pyridin-1-oxid, Smp. 247-249°C (aus CH₂Cl₂ und Isopropyläther) erhalten.

### Beispiel 60

In Analogie zu Beispiel 58 wurde aus 4-[4-(4-tert-Butyl-phenylsulfonylamino)-6-chlor-5-(2-methoxy-phenoxy)-pyrimidin-2-yl]-pyridin-1-oxid und Na-Aethylenglykolat in Aethylenglykol das 4-[4-(4-tert-Butyl-phenylsulfonylamino)-6-(2-hydroxy-äthoxy)-5-(2-methoxy-phenoxy)-pyrimidin-2-yl]-pyridin-1-oxid als amorphe Masse erhalten. MS: (M+H)⁺ = 567,4, (M+Na)⁺ = 589,4.

### Beispiel 61

In Analogie zu Beispiel 50 wurde aus 4-tert-Butyl-N-[6-chlor-2-(2-methoxy-äthyl)-5-(2-methoxy-phenoxy)-pyrimidinyl-4-yl]-benzolsulfonamid und Aethylenglykol-Na das 4-tert-Butyl-N-[6-(2-hydroxy-äthoxy)-2-[2-hydroxy-äthoxy)-äthyl]-5-(2-methoxy-phenoxy)-pyrimidin-4-yl]-benzolsulfonamid. MS: M⁺ = 562 erhalten.

Das entsprechende Natriumsalz (nach üblicher Methode hergestellt) ist ein weisser Feststoff, der im Hochvakuum getrocknet wurde. Das 4-tert-Butyl-N-[6-chlor-2-(2-methoxy-äthyl)-5-(2-methoxy-phenoxy)-pyrimidin-4-yl-benzolsulfonamid wurde ausgehend von Methoxy-propionamidin-Hydrochlorid über 2-(2-Methoxy-äthyl)-5-(o-methoxyphenoxy)-4,6-(1H,5H)-pyrimidindion und 4,6-Dichlor-2-(2-methoxy-äthyl)-5-(2-methoxy-phenoxy)-pyrimidin hergestellt.

### Beispiel 62

In Analogie zu Beispiel 50 wurde aus 4-tert-Butyl-N-[6-chlor-2-cyclopropyl-5-(2-methoxy-phenoxy)-pyrimidin-4-yl]-benzolsulfonamid und Aethylenglykol-Na das 4-tert-Butyl-N-[2-cyclopropyl-6-(2-hydroxy-äthoxy)-5-(2-methoxy-phenoxy)-pyrimidin-4-yl]-benzolsulfonamid als Schaum erhalten. MS: M⁺ = 513.

Das 4-tert-Butyl-N-[6-chlor-2-cyclopropyl-5-(2-methoxy-phenoxy)-pyrimidin-4-yl]-benzolsulfonamid wurde ausgehend von Cyclopropylformamidin-hydrochlorid über rac-2-Cyclopropyl-5-(2-methoxy-phenoxy)-1H-pyrimidin-4,6-dion (Smp. 243-244°C) und 4,6-Dichlor-2-cyclopropyl-5-(2-methoxy-phenoxy)pyrimidin (Smp. 80-82°C) hergestellt.

### Beispiel 63

In Analogie zu Beispiel 50 wurde aus 4-tert-Butyl-N-[6-chlor-2-äthyl-5-(2-methoxy-phenoxy)-pyrimidin-4-yl]-benzolsulfonamid und Aethylenglykol-Na das 4-tert-Butyl-N-[2-äthyl-6-(2-hydroxy-äthoxy)-5-(2-methoxy-phenoxy)-pyrimidin-4-yl]-benzolsulfonamid als Schaum erhalten.

Das 4-tert-Butyl-N-[6-chlor-2-äthyl-5-(2-methoxy-phenoxy)-pyrimidin-4-yl]-benzolsulfonamid wurde ausgehend von Propionamidin-hydrochlorid über rac-2-Aethyl-5-(2-methoxy-phenoxy)-1H-pyrimidin-4,6-dion (Smp. 265°C unter Zersetzung) und 4,6-Dichlor-2-äthyl-5-(2-methoxy-phenoxy)-pyrimidin (Smp. 70-71°C) hergestellt.

### Beispiel 64

In Analogie zu Beispiel 50 wurde aus 4-tert-Butyl-N-[6-chlor-2-isopropyl-5-(2-methoxy-phenoxy)-pyrimidin-4-yl]-benzolsulfonamid und Aethylenglykol-Na das 4-tert-Butyl-N-[6-(2-hydroxy-äthoxy)-2-isopropyl-5-(2-methoxy-phenoxy)-pyrimidin-4-yl]-benzolsulfonamid als Feststoff erhalten.

Das 4-tert-Butyl-N-[6-chlor-2-isopropyl-5-(2-methoxy-phenoxy)-pyrimidin-4-yl]-benzolsulfonamid wurde ausgehend von Isopropionamidinhydrochlorid über rac-2-Isopropyl-5-(2-methoxy-phenoxy)-1,4,5,6-tetrahydro-pyrimidin-4,6-dion und 4,6-Dichlor-2-isopropyl-5-(2-methoxy-phenoxy)-pyrimidin (Smp. 70-72°C) hergestellt.

### Beispiel 65

In Analogie zu Beispiel 50 wurde aus 4-Chlor-N-[6-chlor-5-(5-fluor-2-methoxy-phenoxy)-pyrimidin-4-yl]-benzolsulfonamid und Aethylenglykol-Na das 4-Chlor-N-[3-(5-fluor-2-methoxy-phenoxy)-6-(2-hydroxy-äthoxy)-pyrimidin-4-yl]-benzolsulfonamid, Smp. 152-154°C (aus CH₃CN und Isopropyläther) erhalten.

Das 4-Chlor-N-[6-chlor-5-(5-fluor-2-methoxy-phenoxy)-pyrimidin-4-yl]-benzolsulfonamid (Smp. 169-171°C) wurde aus 4,6-Dichlor-5-(5-fluor-2-methoxy-phenoxy)-pyrimidin und 4-Chlorbenzolsulfonamid-K hergestellt.

### Beispiel 66

In Analogie zu Beispiel 50 wurde aus N-[6-Chlor-5-(5-fluor-2-methoxy-phenoxy)-pyrimidin-4-yl]-4-trifluormethyl-benzolsulfonamid und Natriumäthylenglykolat das N-[5-(5-Fluor-2-methoxy-phenoxy)-6-(2-hydroxy-äthoxy)-pyrimidin-4-yl]-4-trifluormethyl-benzolsulfonamid, Smp. 154-155°C (aus Isopropyläther) erhalten.

Das N-[6-Chlor-5-(5-fluor-2-methoxy-phenoxy)-pyrimidin-4-yl]-4-trifluormethyl-benzolsulfonamid (Smp. 185-186°C) wurde aus 4,6-Dichlor-5-(5-fluor-2-methoxy-phenoxy)-pyrimidin und 4-Trifluormethyl-benzolsulfonamid-K hergestellt.

### Beispiel 67

In Analogie zu Beispiel 50, jedoch mit 100°C Reaktionstemperatur wurde aus 4-tert-Butyl-N-[6-chlor-5-(2-methoxy-phenoxy)-2-(pyrimidin-2-yl)-pyrimidin-4-yl]-benzolsulfonamid und Natriumäthylenglykolat in Aethylenglykol das 4-tert-Butyl-N-[6-(2-hydroxy-äthoxy)-5-(2-methoxy-phenoxy)-2-(pyrimidin-2-yl)-pyrimidin-4-yl]-benzolsulfonamid als Feststoff erhalten. Natriumsalz: Smp. 195-198°C.

Das 4-tert-Butyl-N-[6-chlor-5-(2-methoxy-phenoxy)-2-pyrimidin-2-yl)-pyrimidin-4-yl]-benzolsulfonamid wurde ausgehend von Pyrimidin-2-carboxamidin-hydrochlorid über rac-5-(2-Methoxy-phenoxy)-2-pyrimidin-2-yl-tetra-hydro-pyrimidin-4,6-dion und 4,6-Dichlor-5-(2-methoxy-phenoxy)-2,2'-bipyrimidin hergestellt.

### Beispiel 68

In Analogie zu Beispiel 50 wurde aus 4-tert-Butyl-N-[6-chlor-5-(3-methoxy-phenoxy)-2,2'-bipyrimidin-4-yl]-benzolsulfonamid und Na-Aethylenglykolat in Aethylenglykol das 4-tert-Butyl-N-[6-(2-hydroxy-äthoxy)-5-(3-methoxy-phenoxy)-2,2'-bipyrimidin-4-yl]-benzolsulfonamid als Feststoff erhalten.

Das 4-tert-Butyl-N-[6-chlor-5-(3-methoxy.phenoxy)-2,2'-bipyrimidin-4-yl]-benzolsulfonamid wurde ausgehend von rac-5-(3-Methoxy-phenoxy)-2-(pyrimidin-2-yl)-1,4,5,6-tetrahydro-pyrimidin-4,6-dion über 4,6-Dichlor-5-(3-methoxy-phenoxy)-2,2'-bipyrimidinyl hergestellt.

### Beispiel 69

In Analogie zu Beispiel 50 wurde aus 4-tert-Butyl-N-[6-chlor-5-(4-fluor-2-methoxy-phenoxy)-2,2'-bipyrimidin-4-yl]-benzolsulfonamid und Na-Aethylenglykolat in Aethylenglykol das 4-tert-Butyl-N-[5-(4-fluor-2-methoxy-phenoxy)-6-(2-hydroxy-äthoxy)-2,2'-bipyrimidin-4-yl]-benzolsulfonamid, Smp. 161-163°C erhalten.

Das 4-tert-Butyl-N-[6-chlor-5-(4-fluor-2-methoxy-phenoxy)-2,2'-bipyrimidin-4-yl]-benzolsulfonamid (Smp. 225-227°C) wurde ausgehend von 4-Fluor-2-methoxy-phenoxymalonsäure-diäthylester über 5-(4-Fluor-2-methoxy-phenoxy)-2,2'-bipyrimidin-4,6-diol (Zersetzungspunkt >131°C) und 4,6-Dichlor-5-(4-fluor-2-methoxy-phenoxy)-2,2'-bipyrimidin (Smp. 179-180°C) hergestellt.

### Beispiel 70

In Analogie zu Beispiel 50 wurde aus 4-tert-Butyl-N-[6-chlor-5-(4-fluor-2-methoxy-phenoxy)-2-methyl-pyrimidin-4-yl]-benzolsulfonamid und Na-Aethylenglykolat in Aethylenglykol das 4-tert-Butyl-N-[5-(4-fluor-2-methoxy-phenoxy)-6-(2-hydroxy-äthoxy)-2-methyl-pyrimidin-4-yl]-benzolsulfonamid, Smp. 141-142°C (aus CH₂Cl₂-Isopropyläther) erhalten.

Das 4-tert-Butyl-N-[6-chlor-5-(4-fluor-2-methoxy-phenoxy)-2-methyl-pyrimidin-4-yl]-benzolsulfonamid (Smp. 164-165°C) wurde ausgehend von 4-Fluor-2-methoxy-phenoxymalonsäure-diäthylester über rac-5-(4-Fluor-2-methoxy-phenoxy)-2-methyl-1,4,5,6-tetrahydropyrimidin-4,6-dion und 4,6-Dichlor-5-(4-fluor-2-methoxy-phenoxy)-2-methyl-pyrimidin (Smp. 129-130°C) hergestellt.

### Beispiel 71

In Analogie zu Beispiel 50 wurde aus 4-tert-Butyl-N-[6-chlor-5-(4-fluor-2-methoxy-phenoxy)-pyrimidin-4-yl]-benzolsulfonamid und Na-Aethylenglykolat in Aethylenglykol das 4-tert-Butyl-N-[5-(4-fluor-2-methoxy-phenoxy)-6-(2-hydroxy-äthoxy)-pyrimidin-4-yl]-benzolsulfonamid, Smp. 143-144°C (aus CH₂Cl₂-Isopropyläther) erhalten.

Das 4-tert-Butyl-N-[6-chlor-5-(4-fluor-2-methoxy-phenoxy)-pyrimidin-4-yl]-benzolsulfonamid (Smp. 146-147°C) wurde ausgehend von 4-Fluor-2-methoxy-phenoxymalonsäure-diäthylester über rac-5-(4-Fluor-2-methoxy-phenoxy)-1,4,5,6-tetrahydro-pyrimidin-4,6-dion und 4,6-Dichlor-5-(4-fluor-2-methoxy-phenoxy)pyrimidin (Smp. 100-101°C) hergestellt.

### Beispiel 72

In Analogie zu Beispiel 50 wurde aus N-[6-Chlor-5-(5-fluor-2-methoxy-phenoxy)-pyrimidin-4-yl]-4-isopropyl-benzolsulfonamid und Na-Aethylenglykolat in Aethylenglykol das N-[5-(5-Fluor-2-methoxy-phenoxy)-6-(2-hydroxy-äthoxy)-pyrimidin-4-yl]-4-isopropyl-benzolsulfonamid, Smp. 131-132°C (aus Isopropyläther) erhalten.

### Beispiel 73

In Analogie zu Beispiel 50 wurde aus N-[6-Chlor-5-(5-fluor-2-methoxy-phenoxy)-pyrimidin-4-yl]-4-tert-butyl-benzolsulfonamid und Na-Aethylenglykolat in Aethylenglkol das N-[5-(5-Fluor-2-methoxy-phenoxy)-6-(2-hydroxy-äthoxy)-pyrimidin-4-yl]-4-tert-butyl-benzolsulfonamid, Smp. 126-127°C (aus Isopropyläther) erhalten.

Das N-[6-Chlor-5-(5-fluor-2-methoxy-phenoxy)-pyrimidin-4-yl]-4-isopropyl-benzolsulfonamid, Smp. 138-139°C wurde ausgehend von 5-Fluor-2-methoxy-phenoxy)-malonsäure-diäthylester über rac-5-(5-Fluor-2-methoxy-phenoxy)-tetrahydro-pyrimidin-4,6-dion, 4,6-Dichlor-5-(5-fluor-2-methoxy-phenoxy)-pyrimidin (Smp. 98-100°C) und N-[6-Chlor-5-(5-fluor-2-methoxy-phenoxy)-pyrimidin-4-yl]-4-tert-butyl-benzolsulfonamid (Smp. 163-164°C) hergestellt.

### Beispiel 74

In Analogie zu Beispiel 50 wurde aus 4-tert-Butyl-N-[6-chlor-5-(2-fluor-6-methoxy-phenoxy)-pyrimidin-4-yl]-benzolsulfonamid und Na-Aethylenglykolat in Aethylenglykol das 4-tert-Butyl-N-[5-(2-fluor-6-methoxy)-6-(2-hydroxy-äthoxy)-pyrimidin-4-yl]-benzolsulfonamid, Smp. 158-159°C (aus CH₂Cl₂-Isopropyläther) erhalten.

Das 4-tert-Butyl-N-[6-chlor-5-(2-fluor-6-methoxy-phenoxy)-pyrimidin-4-yl]-benzolsulfonamid (Smp. 181-182°C) wurde ausgehend von 2-(2-Fluor-6-methoxy)-malonsäure-diäthylester über rac-5-(2-Fluor-6-methoxy-phenoxy)-1,4,5,6-tetrahydro-pyrimidin-4,6-dion und 4,6-Dichlor-5-(2-fluor-6-methoxy-phenoxy)-pyrimidin (Smp. 78-79°C) hergestellt.

### Beispiel 75

In Analogie zu Beispiel 50 wurde aus 4-tert-Butyl-N-[6-chlor-5-(3-methoxy-phenoxy)-2-(thiophen-2-yl)-pyrimidin-4-yl]-benzolsulfonamid und Na-Aethylenglykolat in Aethylenglykol das 4-tert-Butyl-N-[6-(2-hydroxy-äthoxy)-5-(3-methoxy-phenoxy)-2-(thiophen-2-yl)-pyrimidin-4-yl]-benzolsulfonamid, Smp. 159-161°C (Toluol/n-Hexan) erhalten.

Das 4-tert-Butyl-N-[6-chlor-5-(3-methoxy-phenoxy)-2-(thiophen-2-yl)-pyrimidin-4-yl]-benzolsulfonamid (Smp. 206-207°C) wurde ausgehend von rac-5-(3-Methoxy-phenoxy)-2-(thiophen-2-yl)-3,4,5,6-tetrahydropyrimidin-4,6-dion über 4,6-Dichlor-5-(3-methoxy-phenoxy)-2-thiophen-2-yl)-pyrimidin (Smp. 120-121°C) hergestellt.

### Beispiel 76

In Analogie zu Beispiel 50 wurde aus 4-tert-Butyl-N-[6-chlor-2-(2-methoxy-äthyl)-5-(3-methoxy-phenoxy)-pyrimidin-4-yl]-benzolsulfonamid und Na-Aethylenglykolat in Aethylenglykol, nach chromatographischer Trennung auf Kieselgel das 4-tert-Butyl-N-[6-(2-hydroxy-äthoxy)-2-(2-methoxy-äthyl)-5-(3-methoxy-phenoxy)-pyrimidin-4-yl]-benzolsulfonamid und das 4-tert-Butyl-N-[6-(3-hydroxy-äthoxy)-2-[2-(2-hydroxy-äthoxy)-äthyl]-5-(3-methoxy-phenoxy)-pyrimidin-4-yl]-benzolsulfonamid erhalten.

Das 4-tert-Butyl-N-[6-chlor-2-(2-methoxy-äthyl)-5-(3-methoxy-phenoxy)-pyrimidin-4-yl]-benzolsulfonamid wurde ausgehend von Methoxypropionamidin-hydrochlorid über 2-(2-Methoxy-äthyl)-5-(3-methoxy-phenoxy)-1,4,5,6-tetrahydro-pyrimidin-4,6-dion und 4,6-Dichlor-2-(2-chlor-äthyl)-5-(3-methoxy-phenoxy)-pyrimidin hergestellt.

### Beispiel 77

In Analogie zu Beispiel 50 wurde aus p-tert-Butyl-N-[6-chlor-5-(o-methoxy-phenoxy)-2-methyl-4-pyrimidinyl]-benzolsulfonamid und (S)-2,2-Dimethyl-1,3-dioxolan-4-methanol-Na das (S)-4-tert-Butyl-N-[6-(2,2-dimethyl-1,3-dioxolan-4-ylmethoxy)-5-(2-methoxy-phenoxy)-2-methyl-pyrimidin-4-yl]-benzolsulfonamid, Smp. 124-125°C (aus n-Hexan) erhalten.

### Beispiel 78

Eine Lösung von 1,85 g (S)-4-tert-Butyl-N-[6-(2,2-dimethyl-1,3-dioxolan-4-ylmethoxy)-5-(2-methoxy-phenoxy)-2-methyl-pyrimidin-4-yl]-benzolsulfonamid in EtOH (15 ml) wurden mit 3 ml konz. HCl versetzt und 2 Minuten auf 50°C erwärmt. Nach Eindampfen wurde der Rückstand mit Aether extrahiert und lieferte (R)-4-tert-Butyl-N-[6-(2,3-dihydroxy-propoxy)-5-(2-methoxy-phenoxy)-2-methyl-6-(2-pyrimidin-4-yl]-benzolsulfonamid als Schaum.

### Beispiel 79

Aus N-[6-Chlor-5-(5-fluor-2-methoxy-phenoxy)-pyrimidin-4-yl]-4-tert-butyl-benzolsulfonamid und (R)-2,2-Dimethyl-1,3-dioxolan-4-methanol-Na wurde das (R)-4-tert-Butyl-N-[5-(5-fluor-2-methoxy-phenoxy)-6-(2,2-dimethyl-1,3-dioxolan-4-ylmethoxy)-pyrimidin-4-yl]-benzolsulfonamid (Smp. >86°C) erhalten. Behandlung mit verdünnter Salzsäure lieferte (S)-4-tert-Butyl-N-(5-fluor-2-methoxy-phenoxy)-6-(2,3-dihydroxy-propoxy-pyrimidin-4-yl)-benzolsulfonamid als Schaum.

### Beispiel 80

Aus N-[6-Chlor-5-(5-fluor-2-methoxy-phenoxy)-pyrimidin-4-yl]-4-tert-butyl-benzolsulfonamid und (S)-2,2-Dimethyl-1,3-dioxolan-4-methanol-Natriumsalz wurde das (S)-4-tert-Butyl-N-[5-(5-fluor-2-methoxy-phenoxy)-6-(2,2-dimethyl-1,3-dioxolan-4-ylmethoxy)-pyrimidin-4-yl]-benzolsulfonamid (Smp. >86°C) erhalten. Behandlung mit verdünnter HCl lieferte (R)-4-tert-Butyl-N-(5-fluor-2-methoxy-phenoxy)-6-(2,3-dihydroxy-propoxy-pyrimidin-4-yl]-benzolsulfonamid als Schaum.

### Beispiel 81

Aus 4-tert-Butyl-N-[6-chlor-5-(2-methoxy-phenoxy)-2-thiophen-2-yl-pyrimidin-4-yl]-benzolsulfonamid und (S)-2,2-Dimethyl-1,3-dioxolan-4-methanol-Natriumsalz wurde das 4-tert-Butyl-N-[6-[(S)-1,3-dioxolan-4-yl-methoxy]-5-(2-methoxy-phenoxy)-2-thiophen-2-yl-pyrimidin-4-yl]-benzolsulfonamid als Schaum erhalten. Behandlung mit verdünnter Salzsäure in Dioxan lieferte (R)-4-tert-Butyl-N-[6-(2,3-dihydroxy-propoxy)-5-(2-methoxy-phenoxy)-2-(thiophen-2-yl)-pyrimidin-4-yl]-benzolsulfonamid als Schaum.

### Beispiel 82

Aus 4-tert-Butyl-N-[6-chlor-5-(2-methoxy-phenoxy)-2-thiophen-2-yl-pyrimidin-4-yl]-benzolsulfonamid und (R)-2,2-Dimethyl-1,3-dioxolan-4-methanol-Natriumsalz wurde das 4-tert-Butyl-N-[6-(R)-1,3-dioxolan-4-yl-methoxy]-5-(2-methoxy-phenoxy)-2-thiophen-2-yl-pyrimidin-4-yl]-benzolsulfonamid und daraus mit verdünnter HCl in Dioxan das (S)-4-tert-Butyl-N-[6-(2,3-dihydroxy-propoxy)-5-(2-methoxy-phenoxy)-2-(thiophen-2-yl)-pyrimidin-4-yl]-benzolsulfonamid erhalten.

### Beispiel 83

Aus 4-tert-Butyl-N-[6-chlor-5-(2-methoxy-phenoxy)-2-(thiophen-3-yl)-pyrimidin-4-yl]-benzolsulfonamid und (R)-2,2-Dimethyl-1,3-dioxolan-4-methanol-Natriumsalz wurde das (R)-4-tert-Butyl-N-[6-(2,2-dimethyl-1,3-dioxolan-4-ylmethoxy)-5-(2-methoxy-phenoxy)-2-(thiophen-3-yl)-pyrimidin-4-yl]-benzolsulfonamid und daraus mit verdünnter HCl in Dioxan 4-tert-Butyl-N-[6-[(S)-2,3-dihydroxy-propoxy]-5-(2-methoxy-phenoxy)-2-thiophen-3-yl-pyrimidin-4-yl]-benzolsulfonamid erhalten.

### Beispiel 84

Aus 4-tert-Butyl-N-[6-chlor-5-(2-methoxy-phenoxy)-2-(thiophen-3-yl)-pyrimidin-4-yl]-benzolsulfonamid und (S)-2,2-Dimethyl-1,3-dioxolan-4-methanol-Natriumsalz wurde das (S)-4-tert-Butyl-N-[(2,2-dimethyl-1,3-dioxolan-4-ylmethoxy)-5-(2-methoxy-phenoxy)-2-(thiophen-3-yl)-pyrimidin-4-yl]-benzolsulfonamid und daraus mit verdünnter HCl in Dioxan 4-tert-Butyl-N-[6-[(R)-2,3-dihydroxy-propoxy]-5-(2-methoxy-phenoxy)-2-thiophen-3-yl-pyrimidin-4-yl]-benzolsulfonamid erhalten.

### Beispiel 85

Aus 4-tert-Butyl-N-[6-chlor-2-furan-3-yl-5-(2-methoxy-phenoxy)-pyrimidin-4-yl]-benzolsulfonamid und (S)-2,2-Dimethyl-1,3-dioxolan-4-methanol-Natriumsalz wurde das (S)-4-tert-Butyl-N-[6-(2,2-dimethyl-1,3-dioxolan-4-yl-methoxy)-2-(furan-3-yl)-5-(2-methoxy-phenoxy)-pyrimidin-4-yl]-benzolsulfonamid und daraus mit verdünnter HCl in Dioxan (R)-4-tert-Butyl-N-[2-(furan-3-yl)-6-(2,3-dihydroxy-propoxy)-5-(2-methoxy-phenoxy)-pyrimidin-4-yl]-benzolsulfonamid erhalten.

### Beispiel 86

Aus 4-tert-Butyl-N-[6-chlor-2-furan-3-yl-5-(2-methoxy-phenoxy)-pyrimidin-4-yl]-benzolsulfonamid und (R)-2,2-Dimethyl-1,3-dioxolan-4-methanol-Natriumsalz wurde das (R)-4-tert-Butyl-N-[6-(2,2-dimethyl-1,3-dioxolan-4-ylmethoxy)-2-(furan-3-yl)-5-(2-methoxy-phenoxy)-pyrimidin-4-yl]-benzolsulfonamid und daraus mit verdünnter HCl in Dioxan (S)-4-tert-Butyl-N-[2-(furan-3-yl)-6-(2,3-dihydroxy-propoxy)-5-(2-methoxy-phenoxy)-pyrimidin-4-yl]-benzolsulfonamid erhalten.

### Beispiel 87

Durch Umsetzung von p-t-Butyl-N-[6-(2-hydroxyäthoxy)-5-(m-methoxy-phenoxy)-4-pyrimidinyl]benzolsulfonamid und 3-Methyl-5-isoxazolcarbonsäure in Gegenwart von Dimethylaminopyridin und Dicyclohexylcarbodiimid in Methylenchlorid wurde der 3-Methylisoxazol-5-carbonsäure-2-[6-(4-t-butylbenzolsulfonamino)-5-(3-methoxyphenoxy)pyrimidin-4-yloxy]äthylester als weisser Feststoff erhalten.

### Beispiel 88

In Analogie zu Beispiel 87 wurde unter Verwendung von Indol-2-carbonsäure der Indol-2-carbonsäure-2-[6-(4-t-butylbenzolsulfonamino)-5-(3-methoxy-phenoxy)pyrimidin-4-yloxy]äthylester erhalten.

### Beispiel 89

Eine Lösung von 391,5 mg 6-[2-(t-Butyl-dimethylsilyoxy)äthoxy]-5-(2-methoxy-phenoxy)pyrimidin-4-yl-amin in 20 ml Acetonitril wurde mit 200 mg NaH (60%ig) versetzt und 1 Stunde bei Raumtemperatur gerührt. Danach wurden 400 mg (2-Methoxy-5-chlorsulfonyl)phenoxyessigsäureäthylester zugesetzt. Das Reaktionsgemisch wurde 3,5 Stunden bei Raumtemperatur gerührt, auf Eis gegossen, mit Aethylacetat extrahiert und die organische Phase getrocknet. Nach Entfernen des Lösungsmittels und Chromatographie an Silicagel mit Methylenchlorid/Methanol (120:1) wurden 175 mg 4-[6-[2-(t-Butyl-dimethoxysilyloxy)äthoxy]-5-(2-methoxyphenoxy)pyrimidin-4-ylamino-sulfonyl]-2-methoxyphenoxyessigsäureäthylester als weisser Schaum erhalten. Die Verbindung wurde in 6 ml Acetonitril bei 0°C langsam mit 1 ml 40%iger HF versetzt. Das Reaktionsgemisch wurde 30 Minuten bei 0°C und 90 Minuten bei Raumtemperatur gerührt, danach auf Eis/2N KHCO₃-Lösung gegossen, mit Methylenchlorid extrahiert und die organische Phase getrocknet. Nach Entfernung des Lösungsmittels und Chromatograpbie des Rückstandes an Silicagel mit Methylenchlorid/Methanol (50:1) wurde der 5-[N-[6-(2-Hydroxy-äthoxy)-5-(2-methoxyphenoxy)pyrimidin-4-yl]aminosulfonyl]-2-methoxy-phenoxyessigsäureäthylester als weisser Feststoff erhalten.

Das Ausgangsmaterial wurde wie folgt hergestellt:
In eine Lösung von 7 g 4,6-Dichlor-5-(o-methoxyphenoxy)pyrimidin in 140 ml Aethanol wurden bei -78°C ca. 105 ml NH₃ eingeleitet. Danach wurde das Reaktionsgemisch 15 Stunden bei -78°C und 50 Stunden bei Raumtemperatur gerührt und schliesslich eingedampft. Der Rückstand wurde zwischen Aethylacetat und Wasser verteilt und die organische Phase aufgearbeitet. Man erhielt 6,45 g 4-Amino-6-chlor-5-(o-methoxyphenoxy)pyrimidin als weisse Kristalle.
2,3 g der vorstehend erhaltenen Verbindung wurden zu einer Lösung von 250 mg Natrium in 40 ml Aethylenglykol bei 50°C gegeben. Die Lösung wurde 12 Stunden auf 100°C erwärmt, danach zwischen halbgesättigter NH₄Cl-Lösung und Methylenchlorid verteilt und aufgearbeitet. Man erhielt 2,47 g 2-[6-Amino-5-(o-methoxyphenoxy)-4-pyrimidinyl]-1-äthanol als weisse Kristalle.
Eine Lösung von 2,5 g der vorstehenden Verbindung in 100 ml Methylenchlorid wurde mit 2,74 g Dimethylaminopyridin und 3,39 g t-Butyldimethylchlorsilan versetzt und 48 Stunden bei Raumtemperatur gerührt. Danach wurden weitere 1,35 g Dimethylaminopyridin und 1,65 g t-Butyldimethylchlorsilan zugesetzt und das Reaktionsgemisch weitere 18 Stunden bei Raumtemperatur gerührt. Das Reaktionsgemisch wurde filtriert, die Lösung eingeengt, der Eindampfrückstand zwischen halbgesättigter NH₄Cl-Lösung und Aethylacetat verteilt und die organische Phase aufgearbeitet. Man erhielt 2,78 g 6-[2-(t-Butyldimethylsilyloxy)äthoxy]-5-(2-methoxy-phenoxy)-pyrimidin-4-ylamin als weissen Feststoff.

### Beispiel A

Tabletten enthaltend die folgenden Bestandteile können in herkömmlicher Weise hergestellt werden:

| Bestandteile | pro Tablette |
|---|---|
| Verbindung der Formel I | 10,0 - 100,0 mg |
| Lactose | 125,0 mg |
| Maisstärke | 75,0 mg |
| Talk | 4,0 mg |
| Magnesiumstearat | 1,0 mg |

### Beispiel B

Kapseln enthaltend die folgenden Bestandteile können in herkömmlicher Weise hergestellt werden:

| Bestandteile | pro Kapsel |
|---|---|
| Verbindung der Formel I | 25,0 mg |
| Lactose | 150,0 mg |
| Maisstärke | 20,0 mg |
| Talk | 5,0 mg |

### Beispiel C

Injektionslösungen können folgende Zusammensetzung aufweisen:

| | |
|---|---|
| Verbindung der Formel I | 3,0 mg |
| Gelatine | 150,0 mg |
| Phenol | 4,7 mg |
| Wasser für Injektionslösungen | ad 1,0 ml |

### Beispiel D

In 3,5 ml Myglyol 812 und 0,08 g Benzylalkohol werden 500 mg Verbindung der Formel I suspendiert. Diese Suspension wird in einen Behälter mit Dosierventil eingef¨üllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

## Patentansprüche (Patentansprüche für folgende(n) Vertragsstaat(en): AT, BE, CH, DE, DK, FR, GB, IT, LI, LU, MC, NL, PT, SE)

1. Verbindungen der Formel 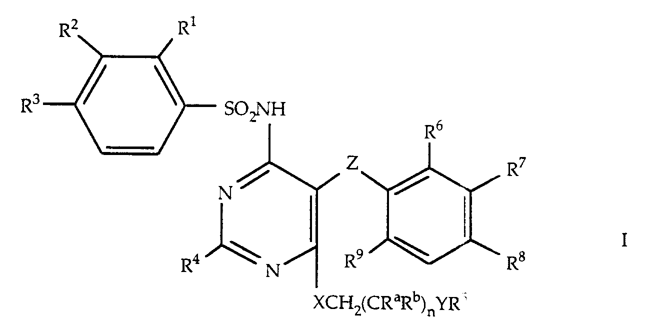 worin
R¹ Wasserstoff, C₁₋₇-Alkyl, C₁₋₇-Alkoxy, C₁₋₇-Alkylthio, Halogen oder Trifluormethyl;
R² Wasserstoff, Halogen, C₁₋₇-Alkoxy, Trifluormethyl oder -OCH₂COOR^{a};
R³ Wasserstoff, Halogen, C₁₋₇-Alkyl, C₁₋₇-Alkylthio, Trifluormethyl, C₃₋₈-Cycloalkyl, C₁₋₇-Alkoxy oder Trifluormethoxy;
R² und R³ zusammen Butadienyl, Methylendioxy, Aethylendioxy oder Isopropylidendioxy;
R⁴ Wasserstoff, C₁₋₇-Alkyl, C₃₋₈-Cycloalkyl, Trifluormethyl, C₁₋₇-Alkoxy, C₁₋₇-Alkylthio, C₁₋₇-Alkylthio-C₁₋₇-alkyl, Hydroxy-C₁₋₇-alkyl, Hydroxy-C₁₋₇-alkoxy, C₁₋₇-Alkoxy-C₁₋₇-alkyl, Hydroxy-C₁₋₇-alkoxy-C₁₋₇-alkyl, Hydroxy-C₁₋₇-alkoxy-C₁₋₇-alkoxy, C₁₋₇-Alkylsulfinyl, C₁₋₇-Alkylsulfonyl, 2-Methoxy-3-hydroxypropoxy, 2-Hydroxy-3-phenylpropyl, Amino-C₁₋₇-alkyl, C₁₋₇-Alkylamino-C₁₋₇-alkyl, Di-C₁₋₇-alkylamino-C₁₋₇-alkyl, Amino, C₁₋₇-Alkylamino, Di-C₁₋₇-alkylamino, Arylamino, Aryl, Arylthio, Aryloxy, Aryl-C₁₋₇-alky, worin Aryl unsubstituiertes Phenyl oder Phenyl substituiert mit C₁₋₇-Alkyl, Trifluormethyl, C₁₋₇-Alkoxy, Carboxyl, Halogen darstellt, unsubstituiertes Heterocyclyl aus der Gruppe von 2-Furyl, 3-Furyl, Pyrimidinyl, 2-Pyridyl, 3-Pyridyl, 4-Pyridyl oder 2, 3, oder 4-Pyridyl-N-oxid, 1,2-oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl, Chinazolyl oder Heterocyclyl wie vorher definiert substituiert mit C₁₋₇-Alkyl, C₁₋₇-Alkoxy, Halogen, Aryl oder Aryl-C₁₋₇-Alkyl;
R⁵ Wasserstoff, C₁₋₇-Alkyl, C₁₋₇-Alkanoyl, Benzoyl, Heterocyclylcarbonyl, Heterocyclylmethyl, worin Heterocyclyl ausgewählt aus unsubstituiertem 2-Furyl, 3-Furyl, Pyrimidinyl, 2-Pyridyl, 3-Pyridyl, 4-Pyridyl, 2-, 3-, oder 4-Pyridyl-N-oxid, 1,2- oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl, Chinazolyl oder Heterocyclyl wie vorher definiert substituiert mit C₁₋₇-Alkyl, C₁₋₇-Alkoxy, Halogen, Phenyl oder Aryl-C₁₋₇-Alkyl ist; oder Tetrahydropyran-2-yl;
R⁶ bis R⁹ Wasserstoff, Halogen, Trifluormethyl, C₁₋₇-Alkyl, C₁₋₇-Alkoxy, C₁₋₇-Alkylthio, Hydroxy, Hydroxymethyl, Cyano, Carboxyl, Formyl, Methylsulfinyl, Methylsulfonyl, Methylsulfonyloxy oder C₁₋₇-Alkyloxy-carbonyloxy;
R⁷ zusammen mit R⁶ oder R⁸ Butadienyl, Methylendioxy, Aethylendioxy oder Isopropylidendioxy;
Z -O-, -S-, Vinylen, -CO-, -OCHR¹⁰- oder -SCHR¹⁰;
R¹⁰ Wasserstoff oder C₁₋₇-Alkyl;
X und Y unabhängig voneinander O, S oder NH;
YR⁵ auch C₁₋₇-Alkylsulfinyl;
R^{a}, R^{b}, R^{c} und R^{d} unabhängig voneinander Wasserstoff oder C₁₋₇-Alkyl; oder
R^{c} und R^{d} zusammen Methylen, Aethylen oder Isopropyliden; und
n 1, 2 oder 3 bedeuten,
und Salze davon.

2. Verbindungen der Formel I von Anspruch 1, worin
R¹ Wasserstoff, C₁₋₇-Alkyl, C₁₋₇-Alkoxy, C₁₋₇-Alkylthio, Halogen oder Trifluormethyl;
R² Wasserstoff, Halogen, C₁₋₇-Alkoxy oder Trifluormethyl;
R³ Wasserstoff, Halogen, C₁₋₇-Alkyl, C₁₋₇-Alkylthio, Trifluormethyl, C₃₋₈-Cycloalkyl, C₁₋₇-Alkoxy oder Trifluormethoxy;
R² und R³ zusammen Butadienyl oder Methylendioxy;
R⁴ Wasserstoff, C₁₋₇-Alkyl, Trifluormethyl, C₁₋₇-Alkoxy, C₁₋₇-Alkylthio, C₁₋₇-Alkylthio-C₁₋₇-alkyl, Hydroxy-C₁₋₇-alkyl, Amino-C₁₋₇-alkyl, C₁₋₇-Alkylamino-C₁₋₇-alkyl, Di-C₁₋₇-alkylamino- C₁₋₇-alkyl, Amino, C₁₋₇-Alkylamino, Di-C₁₋₇-alkylamino, Arylamino, Aryl, Arylthio, Aryloxy, Aryl- C₁₋₇-alkyl, worin Aryl unsubstituiertes Phenyl oder Phenyl substituiert mit C₁₋₇-Alkyl, Trifluormethyl, C₁₋₇-Alkoxy, Carboxyl, Halogen ist, unsubstituiertes Heterocyclyl aus der Gruppe von 2-Furyl, 3-Furyl, Pyrimidinyl, 2-Pyridyl, 3-Pyridyl, 4-Pyridyl, 2-, 3-, oder 4-Pyridyl-N-oxid, 1,2- oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purrinolyl, Chinolyl, Isochinolyl, Chinazolyl oder Tetrahydropyran-2-yl oder Heterocyclyl, wie vorher definiert substituiert mit C₁₋₇-Alkyl, C₁₋₇-Alkoxy, Halogen, Aryl oder Aryl- C₁₋₇-alkyl;
R⁵ Wasserstoff, C₁₋₇-Alkanoyl, Benzoyl oder Tetrahydropyran-2-yl;
R⁶ bis R⁹ Wasserstoff, Halogen, Trifluormethyl, C₁₋₇-Alkyl, C₁₋₇-Alkoxy, C₁₋₇-Alkylthio, Hydroxy, Hydroxymethyl, Cyano, Carboxyl, Formyl, Methylsulfinyl, Methylsulfonyl, Methylsulfonyloxy oder C₁₋₇-Alkyloxy-carbonyloxy;
R⁷ zusammen mit R⁶ oder R⁸ Butadienyl oder -OCH₂O-;
Z -O-, -S-, Vinylen, -CO-, -OCHR¹⁰- oder -SCHR¹⁰;
R¹⁰ Wasserstoff oder C₁₋₇-Alkyl;
X und Y unabhängig voneinander O, S oder NH;
R^{a} und R^{b} Wasserstoff und
n 1, 2, oder 3 bedeuten,
und Salze davon.

3. Verbindungen nach Anspruch 1 oder 2, in denen Z -O- ist.

4. Verbindungen nach den Ansprüchen 1-3, in denen R⁶ C₁₋₇-Alkoxy und R⁷, R⁸ und R⁹ Wasserstoff bedeuten.

5. Verbindungen nach den Ansprüchen 1-3, in denen R⁶ und R⁸ Wasserstoff, R⁷ C₁₋₇-Alkoxy und R⁹ Halogen bedeuten.

6. Verbindungen nach den Ansprüchen 1-5, in denen R⁴ Wasserstoff, 2-Pyrimidinyl, 2- oder 3-Furyl, 2- oder 3-Thienyl, Morpholino oder p-Methoxyphenyl ist

7. Verbindungen nach den Ansprüchen 1-6, in denen YR⁵ Hydroxy, C₁₋₇-Alkoxysulfinyl oder Furoyloxy ist.

8. Die Verbindungen nach Anspruch 3,
p-t-Butyl-N-[6-(2-hydroxyäthoxy)-5-(o-methoxyphenoxy)-4-pyrimidinyl]benzolsulfonamid,
N-[6-(Hydroxyäthoxy)-5-(o-methoxyphenoxy)-4-pyrimidinyl]-p-isopropylbenzolsulfonamid,
p-t-Butyl-N-[6-(2-hydroxyäthoxy)-5-(o-tolyloxy)-4-pyrimidinyl]benzolsulfonamid,
p-t-Butyl-N-[6-(2-hydroxyäthoxy)-5-(o-chlorphenyloxy)-4-pyrimidinyl]benzolsulfonamid,
N-[6-(2-Hydroxyäthoxy)-5-(o-chlorphenoxy)-4-pyrimidinyl]-p-isopropylbenzolsulfonamid,
p-t-Butyl-N-[6-(2-hydroxyäthoxy)-5-(m-methoxyphenoxy)-4-pyrimidinyl]benzolsulfonamid,
p-t-Butyl-N-[6-(2-hydroxyäthoxy)-5-phenoxy-4-pyrimidinyl]benzolsulfonamid,
p-t-Butyl-N-[6-(2-hydroxyäthoxy)-5-(p-methoxyphenoxy)-4-pyrimidinyl]benzolsulfonamid,
p-t-Butyl-N-[6-(2-hydroxyäthoxy)-5-(o-äthoxyphenoxy)-4-pyrimidinyl]benzolsulfonamid,
p-(2,2-Dimethylpropyl)-N-[6-(2-hydroxyäthoxy)-5-(o-methoxyphenoxy)-4-pyrimidinyl]benzolsulfonamid,
p-Isopropyl-N-[6-(2-hydroxyäthoxy)-2-methyl-5-(m-methoxyphenoxy)-4-pyrimidinyl]benzolsulfonamid,
N-[6-(2-Hydroxyäthoxy)-5-(o-methoxyphenoxy)-2-phenyl-4-pyrimidinyl]-p-isopropylbenzolsulfonamid,
N-[6-(2-Hydroxyäthoxy)-5-(2,4,6-trichlorphenoxy)-4-pyrimidinyl]-p-isopropylbenzolsulfonamid,
N-[6-(2-Hydroxyäthoxy)-6-(2,4,6-trichlorphenoxy)-4-pyrimidinyl]-o-toluolsulfonamid,
N-[6-(2-Hydroxyäthoxy)-5-(2,4,6-trichlorphenoxy)-4-pyrimidinyl]-2,4-xylol-sulfonamid,
p-t-Butyl-N-[6-(2-hydroxyäthoxy)-5-[(2-methoxy-p-tolyl)oxy]-4-pyrimidinyl]-benzolsulfonamid,
p-tert-Butyl-N-[6-(2-hydroxyäthoxy)-5-(o-methoxyphenoxy)-2-methyl-4-pyrimidinyl]benzolsulfonamid,
N-[6-(2-Hydroxyäthoxy)-5-(o-methoxyphenoxy)-2-methyl-4-pyrimidinyl)-p-isopropylbenzolsulfonamid,
N-[6-(2-Hydroxyäthoxy)-5-(o-methoxyphenoxy)-(2-trifluormethyl)-4-pyrimidinyl]-p-isopropylbenzolsulfonamid,
p-tert-Butyl-N-[6-(2-hydroxyäthoxy)-5-(o-methoxyphenoxy)-2-(trifluormethyl)-4-pyrimidinyl]benzolsulfonamid,
N-[5-(1,3-Benzodioxol-5-yloxy)-6-(2-hydroxzyäthoxy)-4-pyrimidinyl]-p-tert-butylbenzolsulfonamid,
N-[5-(1,3-Benzodioxol-5-yloxy)-6-(2-hydroxyäthoxy)-4-pyrimidinyl]-p-isopropylbenzolsulfonamid,
N-[6-(2-Hydroxyäthoxy)-5-(o-methoxyphenoxy)-4-pyrimidinyl]-o-methoxybenzolsulfonamid,
p-tert-Butyl-N-[6-(4-hydroxybutoxy)-5-(o-methoxyphenoxy)-2-methyl-4-pyrimidinyl]benzolsulfonamid
N-[6-(2-Hydroxyäthoxy)-5-(2-naphthyloxy)-4-pyrimidinyl]-p-isopropylbenzolsulfonamid,
N-[6-(2-Hydroxyäthoxy)-5-(2-naphthyloxy)-4-pyrimidinyl]-p-tert-butylbenzolsulfonamid,
N-[6-(2-Hydroxyäthoxy)-5-(o-methoxyphenoxy)-2-propyl-4-pyrimidinyl]-p-isopropylbenzolsulfonamid,
p-tert-Butyl-N-[6-(2-hydroxyäthoxy)-5-(o-methoxyphenoxy)-2-propyl-4-pyrimidinyl]benzolsulfonamid,
α,α,α-Trifluor-N-[6-(2-hydroxyäthoxy)-5-(o-methoxyphenoxy)-2-methyl-4-pyrimidinyl]-p-toluolsulfonamid,
p-Chlor-N-[6-(2-hydroxyäthoxy)-5-(o-methoxyphenoxy)-2-methyl-4-pyrimidinyl]benzolsulfonamid,
N-[6-(2-Hydroxyäthoxy)-5-(o-methoxyphenoxy)-2-methyl-4-pyrimidinyl]-p-(trifluormethoxy)benzolsulfonamid,
o-Aethyl-N-[6-(2-hydroxyäthoxy)-5-(o-methoxyphenoxy)-2-methyl-4-pyrimidinyl]benzolsulfonamid,
N-[6-(2-Hydroxyäthoxy)-5-(o-methoxyphenoxy)-2-methyl-4-pyrimidinyl]-p-toluolsulfonamid,
N-[6-(2-Hydroxyäthoxy)-5-(o-methoxyphenoxy)-2-methyl-4-pyrimidinyl]-2-naphthylsulfonamid,
p-tert-Butyl-N-[6-(3-hydroxypropoxy)-5-(o-methoxyphenoxy)-2-methyl-4-pyrimidinyl]benzolsulfonamid,
p-t-Butyl-N-[6-(2-hydroxyäthoxy)-5-[(o-methylthio)phenoxy]-4-pyrimidinyl]benzolsulfonamid,
p-t-Butyl-N-[6-(2-hydroxyäthoxy)-5-(o-methoxyphenoxy)-2-phenyl-4-pyrimidinyl]benzolsulfonamid,
N-[2-Amino-6-(2-hydroxyäthoxy)-5-(o-methoxyphenoxy)-4-pyrimidinyl]-p-t-butylbenzolsulfonamid,
p-t-Butyl-N-[6-(2-hydroxyäthoxy)-2-methyl-5-[o-(methylthio)phenoxyl-4-pyrimidinyl]benzolsulfonamid und
p-t-Butyl-N-[6-(2-hydroxyäthoxy)-2-methyl-5-[o-(R,S-methylsulfinyl)phenoxy]-4-pyrimidinyl]benzolsulfonamid.

9. Die Verbindungen nach Anspruch 3,
4-tert-Butyl-N-[5-(2-chlor-5-methoxy-phenoxy)-6-(2-hydroxy-äthoxy)-2-methylpyrimidin-4-yl]-benzolsulfonamid,
4-tert-Butyl-N-[5-(2-chlor-5-methoxy-phenoxy)-6-(2-(3-furoyloxy)äthoxy)-2-methyl-pyrimidin-4-yl]-benzolsulfonamid,
4-tert-Butyl-N-[6-(2-hydroxy-äthoxy)-5-(2-methoxy-phenoxy)-2-(thiophen-2-yl)-pyrimidin-4-yl]-benzolsulfonamid,
4-tert-Butyl-N-[6-(2-hydroxy-äthoxy)-5-(2-methoxy-phenoxy)-2-(thiophen-3-yl)-pyrimidin-4-yl]-benzolsulfonamid,
4-tert-Butyl-N-[2-(furan-2-yl)-6-(2-hydroxy-äthoxy)-5-(2-methoxy-phenoxy)-pyrimidin-4-yl]-benzolsulfonamid,
4-tert-Butyl-N-[2-furan-3-yl-6-(2-hydroxy-äthoxy)-5-(2-methoxyphenoxy)-pyrimidin-4-yl]-benzolsulfonamid,
4-tert-Butyl-N-[6-(2-hydroxy-äthoxy)-5-(2-methoxy-phenoxy)-2-(pyridin-2-yl)-pyrimidin-4-yl]-benzolsulfonamid,
4-tert-Butyl-N-[6-(2-hydroxy-äthoxy)-5-(2-methoxy-phenoxy)-2-pyridin-4-yl)-pyrimidin-4-yl]-benzolsulfonamid,
4-tert-Butyl-N-[6-(2-hydroxy-äthoxy)-5-(2-methoxy-phenoxy)-2-pyridin-3-yl)-pyrimidin-4-yl]-benzolsulfonamid,
2-[4-(4-tert-Butyl-phenylsulfonylamino)-6-(2-hydroxy-äthoxy)-5-(2-methoxy-phenoxy)-pyrimidin-2-yl]-pyridin-1-oxid,
4-[4-(4-tert-Butyl-phenylsulfonylamino)-6-(2-hydroxy-äthoxy)-5-(2-methoxy-phenoxy)-pyrimidin-2-yl]-pyridin-1-oxid,
4-tert-Butyl-N-[6-(2-hydroxy-äthoxy)-2-[2-hydroxy-äthoxy)-äthyl]-5-(2-methoxyphenoxy)-pyrimidin-4-yl]-benzolsulfonamid,
4-tert-Butyl-N-[2-cyclopropyl-6-(2-hydroxy-äthoxy)-5-(2-methoxy-phenoxy)-pyrimidin-4-yl]-benzolsulfonamid,
4-tert-Butyl-N-[2-äthyl-6-(2-hydroxy-äthoxy)-5-(2-methoxy-phenoxy)-pyrimidin-4-yl]-benzolsulfonamid,
4-tert-Butyl-N-[6-(2-hydroxy-äthoxy)-2-isopropyl-5-(2-methoxy-phenoxy)-pyrimidin-4-yl]-benzolsulfonamid,
N-[5-(5-Fluor-2-methoxy-phenoxy)-6-(2-hydroxy-äthoxy)-pyrimidin-4-yl]-4-trifluormethyl-benzolsulfonamid,
4-tert-Butyl-N-[6-(2-hydroxy-äthoxy)-5-(3-methoxy-phenoxy)-2,2'-bipyrimidin-4-yl]-benzolsulfonamid,
4-tert-Butyl-N-[5-(4-fluor-2-methoxy-phenoxy)-6-(2-hydroxy-äthoxy)-2,2'-bipyrimidin-4-yl]-benzolsulfonamid,
4-tert-Butyl-N-[5-(4-fluor-2-methoxy-phenoxy)-6-(2-hydroxy-äthoxy)-2-methyl-pyrimidin-4-yl]-benzolsulfonamid,
4-tert-Butyl-N-[5-(4-fluor-2-methoxy-phenoxy)-6-(2-hydroxy-äthoxy)-pyrimidin-4-yl]-benzolsulfonamid,
N-[5-(5-Fluor-2-methoxy-phenoxy)-6-(2-hydroxy-äthoxy)-pyrimidin-4-yl]-4-isopropyl-benzolsulfonamid,
N-[5-(5-Fluor-2-methoxy-phenoxy)-6-(2-hydroxy-äthoxy)-pyrimidin-4-yl]-4-tert-butyl-benzolsulfonamid,
4-tert-Butyl-N-[5-(2-fluor-6-methoxy)-6-(2-hydroxy-äthoxy)-pyrimidin-4-yl]-benzolsulfonamid,
4-tert-Butyl-N-[6-(2-hydroxy-äthoxy)-5-(3-methoxy-phenoxy)-2-(thiophen-2-yl)-pyrimidin-4-yl]-benzolsulfonamid,
4-tert-Butyl-N-[6-(2-hydroxy-äthoxy)-2-(2-methoxy-äthyl)-5-(3-methoxy-phenoxy)-pyrimidin-4-yl]-benzolsulfonamid,
3-Methylisoxazol-5-carbonsäure-2-[6-(4-t-butylbenzolsulfonamino)-5-(3-methoxyphenoxy)pyrimidin-4-yloxy]äthylester,
Indol-2-carbonsäure-2-[6-(4-t-butylbenzolsulfonamino)-5-(3-methoxy-phenoxy)pyrimidin-4-yloxy]äthylester,
5-[N-[6-(2-Hydroxyäthoxy)-5-(2-methoxyphenoxy)pyrimidin-4-yl]aminosulfonyl]-2-methoxy-phenoxyessigsäureäthylester.

10. Verbindung nach Anspruch 1, dadurch gekennzeichnet, dass es (RS)-4-tert-Butyl-N-[5-(2-chlor-5-methoxy-phenoxy)-2-äthyl-6-(2-methylsulfinyl-äthoxy)-pyrimidin-4-yl]-benzolsulfonamid ist.

11. Verbindung nach Anspruch 1, dadurch gekennzeichnet, dass es (RS)-N-[5-(2-Chlor-5-methoxy-phenoxy)-6-(2-methylsulfinyl-äthoxy)-pyrimidin-4-yl]-1,3-benzodioxol-5-sulfonamid ist.

12. Verbindung nach Anspruch 1, dadurch gekennzeichnet, dass es 4-tert-Butyl-N-[6-(2-hydroxy-äthoxy)-5-(2-methoxy-phenoxy)-2-(pyrimidin-2-yl)-pyrimidin-4-yl]-benzolsulfonamid ist.

13. Verbindungen nach Anspruch 1 oder 2, in denen Z Vinylen ist.

14. Die Verbindungen nach Anspruch 13,
2-[[5-[(E/Z)-Styryl]-6-p-toluolsulfonamid-4-pyrimidinyl]oxy]äthylacetat,
N-[6-(2-Hydroxyäthoxy)-5-[(E/Z)-styryl]-4-pyrimidinyl]-p-toluolsulfonamid.

15. Verbindungen der Formel 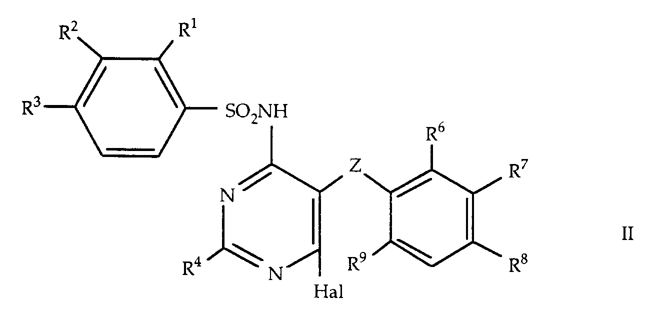 worin R¹-R⁴, R⁶-R⁹ und Z die in Anspruch 1 genannte Bedeutung haben und Hal Halogen ist.

16. Die Verbindungen der Ansprüche 1-14 zur Anwendung als Heilmittel.

17. Pharmazeutische Präparate, enthaltend eine Verbindung der Ansprüche 1-14 und übliche Träger- und Hilfsstoffe.

18. Verwendung von Verbindungen der Ansprüche 1-14 als Wirkstoffe bei der Herstellung von Arzneimitteln zur Behandlung von Erkrankungen, die mit Endothelin-Aktivitäten assoziiert sind, insbesondere Kreislauferkrankungen wie Hypertonie, Ischämie, Vasospasmen und Angina pectoris.

19. Verfahren zur Herstellung von Verbindungen der Ansprüche 1-14, dadurch gekennzeichnet, dass man
a) eine Verbindung der Formel 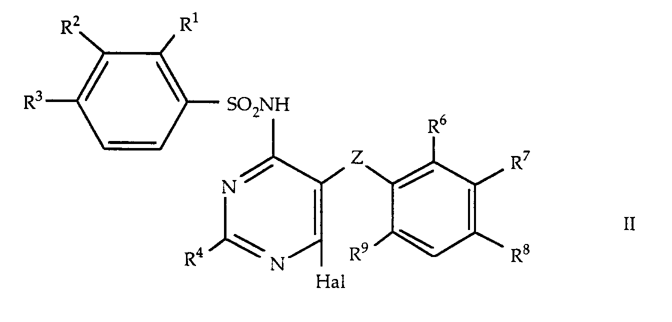 worin R¹-R⁴ und R⁶-R⁹ und Z die in Anspruch 1 genannte Bedeutung haben und Hal Halogen ist, mit einer Verbindung der Formel
MXCH₂(CR^{a}R^{b})ₙYR⁵ III
worin X, Y, n, R^{a}, R^{b} und R⁵ die in Anspruch 1 genannte Bedeutung haben, und M ein Alkalimetall darstellt,
umsetzt, oder
b) eine Verbindung der Formel 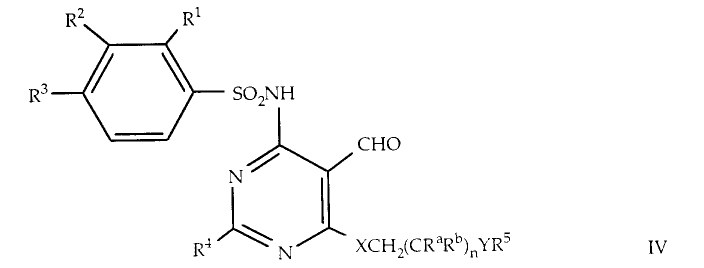 worin R¹-R⁵, R^{a}, R^{b}, X, Y und n die oben genannte Bedeutung haben, mit einer Verbindung der Formel 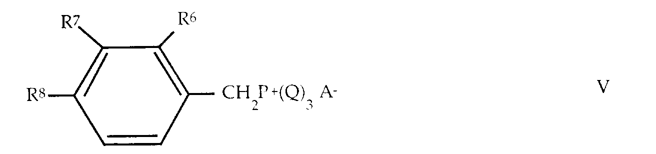 worin R⁶-R⁸ die oben genannte Bedeutung haben; Q Aryl und A⁻ ein Anion ist, umsetzt, oder
c) eine Verbindung der Formel 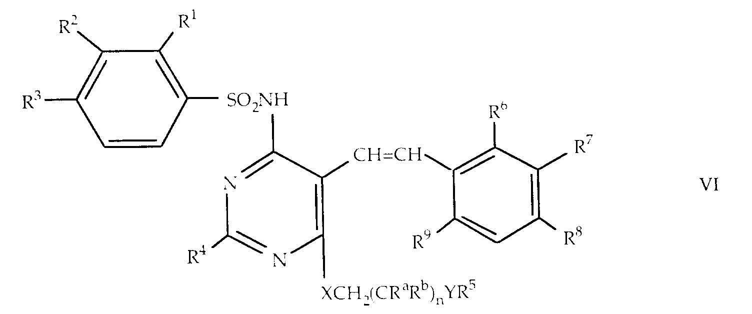 worin R¹-R⁹, R^{a}, R^{b}, X, Y und n die oben genannte Bedeutung haben, hydriert, oder
d) eine Verbindung der Formel 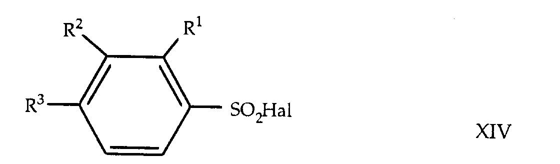 mit einer Verbindung der Formel 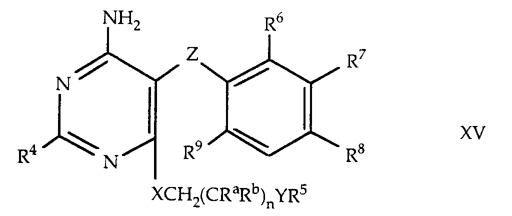 wobei R¹-R⁹, R^{a}, R^{b}, X, Y, Z und n die oben genannte Bedeutung haben, umsetzt, und gegebenenfalls in der erhaltenen Verbindung der Formel I enthaltene Substituenten abwandelt und/oder die erhaltene Verbindung der Formel I in ein Salz überführt.

## Patentansprüche (Patentansprüche für folgende(n) Vertragsstaat(en): GR)

1. Verfahren zur Herstellung von Verbindungen der Formel 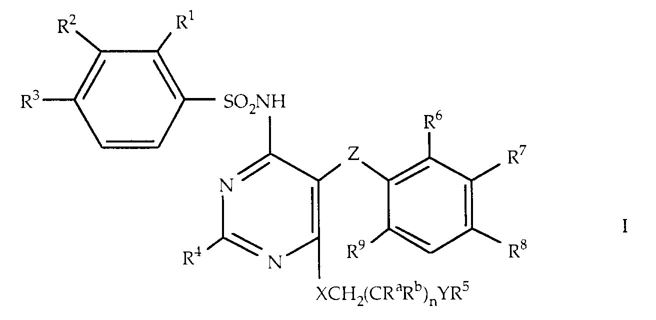 worin
R¹ Wasserstoff, C₁₋₇-Alkyl, C₁₋₇-Alkoxy, C₁₋₇-Alkylthio, Halogen oder Trifluormethyl;
R² Wasserstoff, Halogen, C₁₋₇-Alkoxy, Trifluormethyl oder -OCH₂COOR^{a};
R³ Wasserstoff, Halogen, C₁₋₇-Alkyl, C₁₋₇-Alkylthio, Trifluormethyl, C₃₋₈-Cycloalkyl, C₁₋₇-Alkoxy oder Trifluormethoxy;
R² und R³ zusammen Butadienyl, Methylendioxy, Aethylendioxy oder Isopropylidendioxy;
R⁴ Wasserstoff, C₁₋₇-Alkyl, C₃₋₈-Cycloalkyl, Trifluormethyl, C₁₋₇-Alkoxy,C₁₋₇-Alkylthio, C₁₋₇-Alkylthio-C₁₋₇-alkyl, Hydroxy-C₁₋₇-alkyl, Hydroxy-C₁₋₇-alkoxy, C₁₋₇-Alkoxy-C₁₋₇-alkyl, Hydroxy-C₁₋₇-alkoxy-C₁₋₇-alkyl, Hydroxy-C₁₋₇-alkoxy-C₁₋₇-alkoxy, C₁₋₇-Alkylsulfinyl, C₁₋₇-Alkylsulfonyl, 2-Methoxy-3-hydroxypropoxy, 2-Hydroxy-3-phenylpropyl, Amino-C₁₋₇-alkyl, C₁₋₇-Alkylamino-C₁₋₇-alkyl, Di-C₁₋₇-alkylamino-C₁₋₇-alkyl, Amino, C₁₋₇-Alkylamino, Di-C₁₋₇-alkylamino, Arylamino, Aryl, Arylthio, Aryloxy, Aryl-C₁₋₇-alkyl, worin Aryl unsubstituiertes Phenyl oder Phenyl substituiert mit C₁₋₇-Alkyl, Trifluormethyl, C₁₋₇-Alkoxy, Carboxyl, Halogen darstellt, unsubstituiertes Heterocyclyl aus der Gruppe von 2-Furyl, 3-Furyl, Pyrimidinyl, 2-Pyridyl, 3-Pyridyl, 4-Pyridyl oder 2, 3, oder 4-Pyridyl-N-oxid, 1,2-oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl, Chinazolyl oder Heterocyclyl wie vorher definiert substituiert mit C₁₋₇-Alkyl,C₁₋₇-Alkoxy, Halogen, Aryl oder Aryl-C₁₋₇-Alkyl;
R⁵ Wasserstoff, C₁₋₇-Alkyl, C₁₋₇-Alkanoyl, Benzoyl, Heterocyclylcarbonyl, Heterocyclylmethyl, worin Heterocyclyl ausgewählt aus unsubstituiertem 2-Furyl, 3-Furyl, Pyrimidinyl, 2-Pyridyl, 3-Pyridyl, 4-Pyridyl, 2-, 3-, oder 4-Pyridyl-N-oxid, 1,2- oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl, Chinazolyl oder Heterocyclyl wie vorher definiert substituiert mit C₁₋₇-Alkyl, C₁₋₇-Alkoxy, Halogen, Phenyl oder Aryl-C₁₋₇-Alkyl ist; oder Tetrahydropyran-2-yl;
R⁶ bis R⁹ Wasserstoff, Halogen, Trifluormethyl, C₁₋₇-Alkyl, C₁₋₇-Alkoxy, C₁₋₇-Alkylthio, Hydroxy, Hydroxymethyl, Cyano, Carboxyl, Formyl, Methylsulfinyl, Methylsulfonyl, Methylsulfonyloxy oder C₁₋₇-Alkyloxy-carbonyloxy;
R⁷ zusammen mit R⁶ oder R⁸ Butadienyl, Methylendioxy, Aethylendioxy oder Isopropylidendioxy;
Z -O-, -S-, Vinylen, -CO-, -OCHR¹⁰- oder -SCHR¹⁰;
R¹⁰ Wasserstoff oder C₁₋₇-Alkyl;
X und Y unabhängig voneinander O, S oder NH;
YR⁵ auch C₁₋₇-Alkylsulfinyl;
R^{a}, R^{b}, R^{c} und R^{d} unabhängig voneinander Wasserstoff oder C₁₋₇-Alkyl; oder
R^{c} und R^{d} zusammen Methylen, Aethylen oder Isopropyliden; und
n 1, 2 oder 3 bedeuten,
und Salze davon, dadurch gekennzeichnet, dass man
a) eine Verbindung der Formel 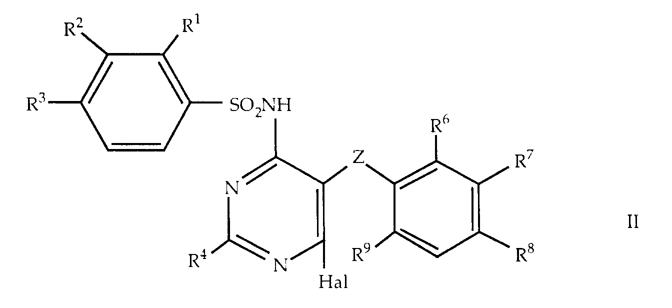 worin R¹-R⁴ und R⁶-R⁹ die oben genannte Bedeutung haben und Hal Halogen ist,
mit einer Verbindung der Formel
MXCH₂(CR^{a}R^{b})ₙYR⁵ III
worin X, Y, n, R^{a}, R^{b} und R⁵ die oben genannte Bedeutung haben, und M ein Alkalimetall darstellt,
umsetzt, oder
b) eine Verbindung der Formel 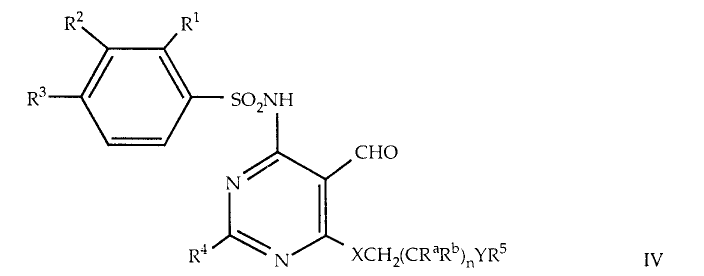 worin R¹-R⁵, R^{a}, R^{b}, X, Y und n die oben genannte Bedeutung haben, mit einer Verbindung der Formel 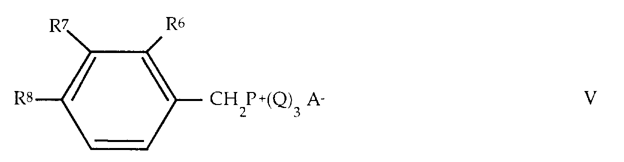 worin R⁶-R⁸ die oben genannte Bedeutung haben; Q Aryl und A⁻ ein Anion ist,
umsetzt, oder
c) eine Verbindung der Formel 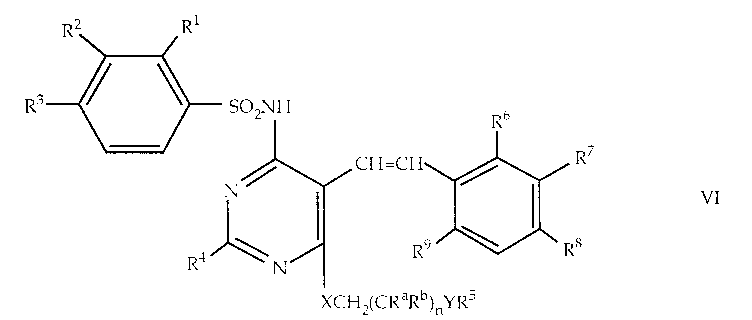 worin R¹-R⁸, R^{a}, R^{b}, X, Y und n die oben genannte Bedeutung haben, hydriert, oder
d) eine Verbindung der Formel 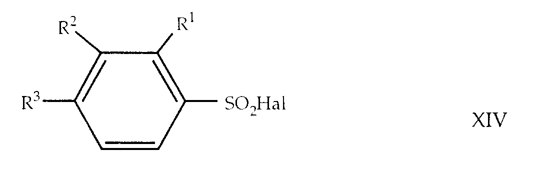 mit einer Verbindung der Formel 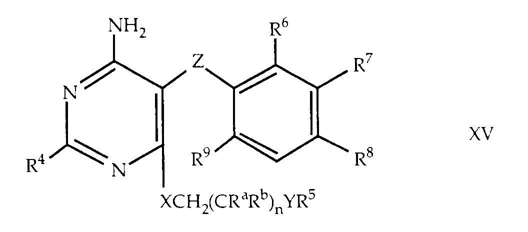 wobei R¹-R⁹, R^{a}, R^{b}, X, Y, Z und n die oben genannte Bedeutung haben, umsetzt, und gegebenenfalls in der erhaltenen Verbindung der Formel I enthaltene Substituenten abwandelt und/oder die erhaltene Verbindung der Formel I in ein Salz überführt.

2. Verfahren nach Anspruch 1 zur Herstellung von Verbindungen der Formel I und Salze davon, worin
R¹ Wasserstoff, C₁₋₇-Alkyl, C₁₋₇-Alkoxy, C₁₋₇-Alkylthio, Halogen oder Trifluormethyl;
R² Wasserstoff, Halogen, C₁₋₇-Alkoxy oder Trifluormethyl;
R³ Wasserstoff, Halogen, C₁₋₇-Alkyl, C₁₋₇-Alkylthio, Trifluormethyl, C₃₋₈-Cycloalkyl, C₁₋₇-Alkoxy oder Trifluormethoxy;
R² und R³ zusammen Butadienyl oder Methylendioxy;
R⁴ Wasserstoff, C₁₋₇-Alkyl, Trifluormethyl, C₁₋₇-Alkoxy, C₁₋₇-Alkylthio, C₁₋₇-Alkylthio-C₁₋₇-alkyl, Hydroxy-C₁₋₇-alkyl, Amino-C₁₋₇-alkyl, C₁₋₇-Alkylamino-C₁₋₇-alkyl, Di-C₁₋₇-alkylamino-C₁₋₇-alkyl, Amino, C₁₋₇-Alkylamino, Di-C₁₋₇-alkylamino, Arylamino, Aryl, Arylthio, Aryloxy, Aryl- C₁₋₇-alkyl, worin Aryl unsubstituiertes Phenyl oder Phenyl substituiert mit C₁₋₇-Alkyl, Trifluormethyl, C₁₋₇-Alkoxy, Carboxyl, Halogen ist, unsubstituiertes Heterocyclyl aus der Gruppe von 2-Furyl, 3-Furyl, Pyrimidinyl, 2-Pyridyl, 3-Pyridyl, 4-Pyridyl, 2-, 3-, oder 4-Pyridyl-N-oxid, 1,2- oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purrinolyl, Chinolyl, Isochinolyl, Chinazolyl oder Tetrahydropyran-2-yl oder Heterocyclyl, wie vorher definiert substituiert mit C₁₋₇-Alkyl, C₁₋₇-Alkoxy, Halogen, Aryl oder Aryl-C₁₋₇-alkyl;
R⁵ Wasserstoff, C₁₋₇-Alkanoyl, Benzoyl oder Tetrahydropyran-2-yl;
R⁶ bis R⁹ Wasserstoff, Halogen, Trifluormethyl, C₁₋₇-Alkyl, C₁₋₇-Alkoxy, C₁₋₇-Alkylthio, Hydroxy, Hydroxymethyl, Cyano, Carboxyl, Formyl, Methylsulfinyl, Methylsulfonyl, Methylsulfonyloxy oder C₁₋₇-Alkyloxy-carbonyloxy;
R⁷ zusammen mit R⁶ oder R⁸ Butadienyl oder -OCH₂O-;
Z -O-, -S-, Vinylen, -CO-, -OCHR¹⁰- oder -SCHR¹⁰;
R¹⁰ Wasserstoff oder C₁₋₇-Alkyl;
X und Y unabhängig voneinander O, S oder NH;
R^{a} und R^{b} Wasserstoff und
n 1, 2, oder 3 bedeuten.

3. Verfahren nach Anspruch 1 oder 2, zur Herstellung von Verbindungen der Formel I, in denen Z -O- ist.

4. Verfahren nach den Ansprüchen 1-3, zur Herstellung von Verbindungen der Formel I, in denen R⁶ C₁₋₇-Alkoxy und R⁷, R⁸ und R⁹ Wasserstoff bedeuten.

5. Verfahren nach den Ansprüchen 1-3, zur Herstellung von Verbindungen der Formel I, in denen R⁶ und R⁸ Wasserstoff, R⁷ C₁₋₇-Alkoxy und R⁹ Halogen bedeuten.

6. Verfahren nach den Ansprüchen 1-3, zur Herstellung von Verbindungender Formel I, in denen R⁴ Wasserstoff, 2-Pyrimidinyl, 2- oder 3-Furyl, 2- oder 3-Thienyl, Morpholino oder p-Methoxyphenyl ist.

7. Verfahren nach den Ansprüchen 1-3, zur Herstellung von Verbindungen der Formel I, in denen YR⁵ Hydroxy, C₁₋₇-Alkoxysulfinyl oder Furoyloxy ist.

8. Verfahren nach Anspruch 3 zur Herstellung der Verbindungen
p-t-Butyl-N-[6-(2-hydroxyäthoxy)-5-(o-methoxyphenoxy)-4-pyrimidinyl]benzolsulfonamid,
N-[6-(Hydroxyäthoxy)-5-(o-methoxyphenoxy)-4-pyrimidinyl]-p-isopropylbenzolsulfonamid,
p-t-Butyl-N-[6-(2-hydroxyäthoxy)-5-(o-tolyloxy)-4-pyrimidinyl]benzolsulfonamid,
p-t-Butyl-N-[6-(2-hydroxyäthoxy)-5-(o-chlorphenyloxy)-4-pyrimidinyl]benzolsulfonamid,
N-[6-(2-Hydroxyäthoxy)-5-(o-chlorphenoxy)-4-pyrimidinyl]-p-isopropylbenzolsulfonamid,
p-t-Butyl-N-[6-(2-hydroxyäthoxy)-5-(m-methoxyphenoxy)-4-pyrimidinyl]benzolsulfonamid,
p-t-Butyl-N-[6-(2-hydroxyäthoxy)-5-phenoxy-4-pyrimidinyl]benzolsulfonamid,
p-t-Butyl-N-[6-(2-hydroxyäthoxy)-5-(p-methoxyphenoxy)-4-pyrimidinyl]benzolsulfonamid,
p-t-Butyl-N-[6-(2-hydroxyäthoxy)-5-(o-äthoxyphenoxy)-4-pyrimidinyl]benzolsulfonamid,
p-(2,2-Dimethylpropyl)-N-[6-(2-hydroxyäthoxy)-5-(o-methoxyphenoxy)-4-pyrimidinyl]benzolsulfonamid,
p-Isopropyl-N-[6-(2-hydroxyäthoxy)-2-methyl-5-(m-methoxyphenoxy)-4-pyrimidinyl]benzolsulfonamid,
N-[6-(2-Hydroxyäthoxy)-5-(o-methoxyphenoxy)-2-phenyl-4-pyrimidinyl]-p-isopropylbenzolsulfonamid,
N-[6-(2-Hydroxyäthoxy)-5-(2,4,6-trichlorphenoxy)-4-pyrimidinyl]-p-isopropylbenzolsulfonamid,
N-[6-(2-Hydroxyäthoxy)-6-(2,4,6-trichlorphenoxy)-4-pyrimidinyl]-o-toluolsulfonamid,
N-[6-(2-Hydroxyäthoxy)-5-(2,4,6-trichlorphenoxy)-4-pyrimidinyl]-2,4-xylolsulfonamid,
p-t-Butyl-N-[6-(2-hydroxyäthoxy)-5-[(2-methoxy-p-tolyl)oxy]-4-pyrimidinyl]benzolsulfonamid,
p-tert-Butyl-N-[6-(2-hydroxyäthoxy)-5-(o-methoxyphenoxy)-2-methyl-4-pyrimidinyl]benzolsulfonamid,
N-[6-(2-Hydroxyäthoxy)-5-(o-methoxyphenoxy)-2-methyl-4-pyrimidinyl)-p-isopropylbenzolsulfonamid,
N-[6-(2-Hydroxyäthoxy)-5-(o-methoxyphenoxy)-(2-trifluormethyl)-4-pyrimidinyl]-p-isopropylbenzolsulfonamid,
p-tert-Butyl-N-[6-(2-hydroxyäthoxy)-5-(o-methoxyphenoxy)-2-(trifluormethyl)-4-pyrimidinyl]benzolsulfonamid,
N-[5-(1,3-Benzodioxol-5-yloxy)-6-(2-hydroxyäthoxy)-4-pyrimidinyl]-p-tert-butylbenzolsulfonamid,
N-[5-(1,3-Benzodioxol-5-yloxy)-6-(2-hydroxyäthoxy)-4-pyrimidinyl]-p-isopropylbenzolsulfonamid,
N-[6-(2-Hydroxyäthoxy)-5-(o-methoxyphenoxy)-4-pyrimidinyl]-o-methoxybenzolsulfonamid,
p-tert-Butyl-N-[6-(4-hydroxybutoxy)-5-(o-methoxyphenoxy)-2-methyl-4-pyrimidinyl]benzolsulfonamid
N-[6-(2-Hydroxyäthoxy)-5-(2-naphthyloxy)-4-pyrimidinyl]-p-isopropylbenzolsulfonamid,
N-[6-(2-Hydroxyäthoxy)-5-(2-naphthyloxy)-4-pyrimidinyl]-p-tert-butylbenzolsulfonamid,
N-[6-(2-Hydroxyäthoxy)-5-(o-methoxyphenoxy)-2-propyl-4-pyrimidinyl]-p-isopropylbenzolsulfonamid,
p-tert-Butyl-N-[6-(2-hydroxyäthoxy)-5-(o-methoxyphenoxy)-2-propyl-4-pyrimidinyl]benzolsulfonamid,
α,α,α-Trifluor-N-[6-(2-hydroxyäthoxy)-5-(o-methoxyphenoxy)-2-methyl-4-pyrimidinyl]-p-toluolsulfonamid,
p-Chlor-N-[6-(2-hydroxyäthoxy)-5-(o-methoxyphenoxy)-2-methyl-4-pyrimidinyl]benzolsulfonamid,
N-[6-(2-Hydroxyäthoxy)-5-(o-methoxyphenoxy)-2-methyl-4-pyrimidinyl]-p-(trifluormethoxy)benzolsulfonamid,
o-Aethyl-N-[6-(2-hydroxyäthoxy)-5-(o-methoxyphenoxy)-2-methyl-4-pyrimidinyl]benzolsulfonamid,
N-[6-(2-Hydroxyäthoxy)-5-(o-methoxyphenoxy)-2-methyl-4-pyrimidinyl]-p-toluolsulfonamid,
N-[6-(2-Hydroxyäthoxy)-5-(o-methoxyphenoxy)-2-methyl-4-pyrimidinyl]-2-naphthylsulfonamid,
p-tert-Butyl-N-[6-(3-hydroxypropoxy)-5-(o-methoxyphenoxy)-2-methyl-4-pyrimidinyl]benzolsulfonamid,
p-t-Butyl-N-[6-(2-hydroxyäthoxy)-5-[(o-methylthio)phenoxy]-4-pyrimidinyl]benzolsulfonamid,
p-t-Butyl-N-[6-(2-hydroxyäthoxy)-5-(o-methoxyphenoxy)-2-phenyl-4-pyrimidinyl]benzolsulfonamid,
N-[2-Amino-6-(2-hydroxyäthoxy)-5-(o-methoxyphenoxy)-4-pyrimidinyl]-p-t-butylbenzolsulfonamid,
p-t-Butyl-N-[6-(2-hydroxyäthoxy)-2-methyl-5-[o-(methylthio)phenoxy]-4-pyrimidinyl]benzolsulfonamid und
p-t-Butyl-N-[6-(2-hydroxyäthoxy)-2-methyl-5-[o-(R,S-methylsulfinyl)-phenoxy]-4-pyrimidinyl]benzolsulfonamid.

9. Verfahren nach Anspruch 3 zur Herstellung der Verbindungen
4-tert-Butyl-N-[5-(2-chlor-5-methoxy-phenoxy)-6-(2-hydroxy-äthoxy)-2-methyl-pyrimidin-4-yl]-benzolsulfonamid,
4-tert-Butyl-N-[5-(2-chlor-5-methoxy-phenoxy)-6-(2-(3-furoyloxy)äthoxy)-2-methyl-pyrimidin-4-yl]-benzolsulfonamid,
4-tert-Butyl-N-[6-(2-hydroxy-äthoxy)-5-(2-methoxy-phenoxy)-2-(thiophen-2-yl)-pyrimidin-4-yl]-benzolsulfonamid,
4-tert-Butyl-N-[6-(2-hydroxy-äthoxy)-5-(2-methoxy-phenoxy)-2-(thiophen-3-yl)-pyrimidin-4-yl]-benzolsulfonamid,
4-tert-Butyl-N-[2-(furan-2-yl)-6-(2-hydroxy-äthoxy)-5-(2-methoxy-phenoxy)-pyrimidin-4-yl]-benzolsulfonamid,
4-tert-Butyl-N-[2-furan-3-yl-6-(2-hydroxy-äthoxy)-5-(2-methoxyphenoxy)-pyrimidin-4-yl]-benzolsulfonamid,
4-tert-Butyl-N-[6-(2-hydroxy-äthoxy)-5-(2-methoxy-phenoxy)-2-(pyridin-2-yl)-pyrimidin-4-yl]-benzolsulfonamid,
4-tert-Butyl-N-[6-(2-hydroxy-äthoxy)-5-(2-methoxy-phenoxy)-2-pyridin-4-yl)-pyrimidin-4-yl]-benzolsulfonamid,
4-tert-Butyl-N-[6-(2-hydroxy-äthoxy)-5-(2-methoxy-phenoxy)-2-pyridin-3-yl)-pyrimidin-4-yl]-benzolsulfonamid,
2-[4-(4-tert-Butyl-phenylsulfonylamino)-6-(2-hydroxy-äthoxy)-5-(2-methoxy-phenoxy)-pyrimidin-2-yl]-pyridin-1-oxid,
4-[4-(4-tert-Butyl-phenylsulfonylamino)-6-(2-hydroxy-äthoxy)-5-(2-methoxy-phenoxy)-pyrimidin-2-yl]-pyridin-1-oxid,
4-tert-Butyl-N-[6-(2-hydroxy-äthoxy)-2-[2-hydroxy-äthoxy)-äthyl]-5-(2-methoxy-phenoxy)-pyrimidin-4-yl]-benzolsulfonamid,
4-tert-Butyl-N-[2-cyclopropyl-6-(2-hydroxy-äthoxy)-5-(2-methoxy-phenoxy)-pyrimidin-4-yl]-benzolsulfonamid,
4-tert-Butyl-N-[2-äthyl-6-(2-hydroxy-äthoxy)-5-(2-methoxy-phenoxy)-pyrimidin-4-yl]-benzolsulfonamid,
4-tert-Butyl-N-[6-(2-hydroxy-äthoxy)-2-isopropyl-5-(2-methoxy-phenoxy)-pyrimidin-4-yl]-benzolsulfonamid,
N-[5-(5-Fluor-2-methoxy-phenoxy)-6-(2-hydroxy-äthoxy)-pyrimidin-4-yl]-4-trifluormethyl-benzolsulfonamid,
4-tert-Butyl-N-[6-(2-hydroxy-äthoxy)-5-(3-methoxy-phenoxy)-2,2'-bipyrimidin-4-yl]-benzolsulfonamid,
4-tert-Butyl-N-[5-(4-fluor-2-methoxy-phenoxy)-6-(2-hydroxy-äthoxy)-2,2'-bipyrimidin-4-yl]-benzolsulfonamid,
4-tert-Butyl-N-[5-(4-fluor-2-methoxy-phenoxy)-6-(2-hydroxy-äthoxy)-2-methyl-pyrimidin-4-yl]-benzolsulfonamid,
4-tert-Butyl-N-[5-(4-fluor-2-methoxy-phenoxy)-6-(2-hydroxy-äthoxy)-pyrimidin-4-yl]-benzolsulfonamid,
N-[5-(5-Fluor-2-methoxy-phenoxy)-6-(2-hydroxy-äthoxy)-pyrimidin-4-yl]-4-isopropyl-benzolsulfonamid,
N-[5-(5-Fluor-2-methoxy-phenoxy)-6-(2-hydroxy-äthoxy)-pyrimidin-4-yl]-4-tert-butyl-benzolsulfonamid,
4-tert-Butyl-N-[5-(2-fluor-6-methoxy)-6-(2-hydroxy-äthoxy)-pyrimidin-4-yl]-benzolsulfonamid,
4-tert-Butyl-N-[6-(2-hydroxy-äthoxy)-5-(3-methoxy-phenoxy)-2-(thiophen-2-yl)-pyrimidin-4-yl]-benzolsulfonamid,
4-tert-Butyl-N-[6-(2-hydroxy-äthoxy)-2-(2-methoxy-äthyl)-5-(3-methoxy-phenoxy)-pyrimidin-4-yl]-benzolsulfonamid,
3-Methylisoxazol-5-carbonsäure-2-[6-(4-t-butylbenzolsulfonamino)-5-(3-methoxyphenoxy)pyrimidin-4-yloxy]äthylester,
Indol-2-carbonsäure-2-[6-(4-t-butylbenzolsulfonamino)-5-(3-methoxy-phenoxy)pyrimidin-4-yloxy]äthylester,
5-[N-[6-(2-Hydroxyäthoxy)-5-(2-methoxyphenoxy)pyrimidin-4-yl]aminosulfonyl]-2-methoxy-phenoxyessigsäureäthylester.

10. Verfahren nach Anspruch 3 zur Herstellung von (RS)-4-tert-Butyl-N-[5-(2-chlor-5-methoxy-phenoxy)-2-äthyl-6-(2-methylsulfinyl-äthoxy)-pyrimidin-4-yl]-benzolsulfonamid.

11. Verfahren nach Anspruch 3 zur Herstellung von (RS)-N-[5-(2-Chlor-5-methoxy-phenoxy)-6-(2-methylsulfinyl-äthoxy)-pyrimidin-4-yl]-1,3-benzodioxol-5-sulfonamid.

12. Verfahren nach Anspruch 3 zur Herstellung von 4-tert-Butyl-N-[6-(2-hydroxy-äthoxy)-5-(2-methoxy-phenoxy)-2-(pyrimidin-2-yl)-pyrimidin-4-yl]-benzolsulfonamid.

13. Verfahren nach den Ansprüchen 1 oder 2 zur Herstellung von Verbindungen der Formel I, in denen Z Vinylen ist.

14. Verfahren nach Anspruch 14 zur Herstellung der Verbindungen
2-[[5-[(E/Z)-Styryl]-6-p-toluolsulfonamid-4-pyrimidinyl]oxy]äthylacetat,
N-[6-(2-Hydroxyäthoxy)-5-[(E/Z)-styryl]-4-pyrimidinyl]-p-toluolsulfonamid.

15. Verfahren zur Herstellung pharmazeutischer Präparate, dadurch gekennzeichnet, dass man eine Verbindung der Formel I von Anspruch 1 oder Salze davon mit üblichen pharmazeutischen Hilfs- und Trägerstoffen vermischt und in eine zur therapeutischen Anwendung geeignete Form bringt.

16. Verwendung von Verbindungen der Formel I von Anspruch 1 und Salzen davon als Wirkstoffe bei der Herstellung zur Behandlung von Erkrankungen, die mit Endothelin-Aktivitäten assoziiert sind, insbesondere Kreislauferkrankungen wie Hypertonie, Ischämie, Vasospasmen und Angina pectoris.

17. Verbindungen der Formel 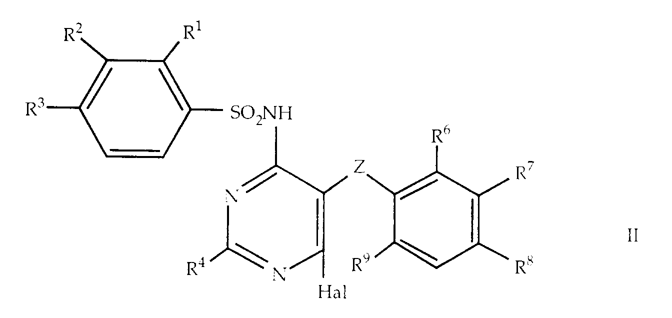 worin R¹-R⁵, R^{a}, R^{b}, Y und n die in Anspruch 1 genannte Bedeutung haben und Hal Halogen ist.

## Patentansprüche (Patentansprüche für folgende(n) Vertragsstaat(en): ES)

1. Verfahren zur Herstellung von Verbindungen der Formel 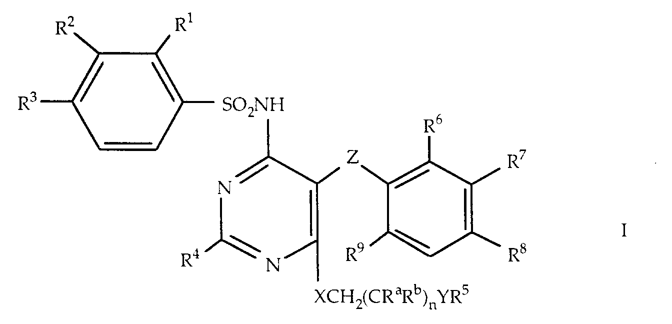 worin
R¹ Wasserstoff, C₁₋₇-Alkyl, C₁₋₇-Alkoxy, C₁₋₇-Alkylthio, Halogen oder Trifluormethyl;
R² Wasserstoff, Halogen, C₁₋₇-Alkoxy, Trifluormethyl oder -OCH₂COOR^{a};
R³ Wasserstoff, Halogen, C₁₋₇-Alkyl, C₁₋₇-Alkylthio, Trifluormethyl, C₃₋₈-Cycloalkyl, C₁₋₇-Alkoxy oder Trifluormethoxy;
R² und R³ zusammen Butadienyl, Methylendioxy, Aethylendioxy oder Isopropylidendioxy;
R⁴ Wasserstoff, C₁₋₇-Alkyl, C₃₋₈-Cycloalkyl, Trifluormethyl, C₁₋₇-Alkoxy, C₁₋₇-Alkylthio, C₁₋₇-Alkylthio C₁₋₇-alkyl, Hydroxy-C₁₋₇-alkyl, Hydroxy-C₁₋₇-alkoxy, C₁₋₇-Alkoxy-C₁₋₇-alkyl, Hydroxy-C₁₋₇-alkoxy-C₁₋₇-alkyl, Hydroxy-C₁₋₇-alkoxy-C₁₋₇-alkoxy, C₁₋₇-Alkylsulfinyl, C₁₋₇-Alkylsulfonyl, 2-Methoxy-3-hydroxypropoxy, 2-Hydroxy-3-phenylpropyl, Amino-C₁₋₇-alkyl, C₁₋₇-Alkylamino-C₁₋₇-alkyl, Di-C₁₋₇-alkylamino-C₁₋₇-alkyl, Amino, C₁₋₇-Alkylamino, Di-C₁₋₇-alkylamino, Arylamino, Aryl, Arylthio, Aryloxy, Aryl-C₁₋₇-alkyl, worin Aryl unsubstituiertes Phenyl oder Phenyl substituiert mit C₁₋₇-Alkyl, Trifluormethyl, C₁₋₇-Alkoxy, Carboxyl, Halogen darstellt, unsubstituiertes Heterocyclyl aus der Gruppe von 2-Furyl, 3-Furyl, Pyrimidinyl, 2-Pyridyl, 3-Pyridyl, 4-Pyridyl oder 2, 3, oder 4-Pyridyl-N-oxid, 1,2-oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl, Chinazolyl oder Heterocyclyl wie vorher definiert substituiert mit C₁₋₇-Alkyl,C₁₋₇-Alkoxy, Halogen, Aryl oder Aryl-C₁₋₇-Alkyl;
R⁵ Wasserstoff, C₁₋₇-Alkyl, C₁₋₇-Alkanoyl, Benzoyl, Heterocyclylcarbonyl, Heterocyclylmethyl, worin Heterocyclyl ausgewählt aus unsubstituiertem 2-Furyl, 3-Furyl, Pyrimidinyl, 2-Pyridyl, 3-Pyridyl, 4-Pyridyl, 2-, 3-, oder 4-Pyridyl-N-oxid, 1,2- oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl, Chinazolyl oder Heterocyclyl wie vorher definiert substituiert mit C₁₋₇-Alkyl, C₁₋₇-Alkoxy, Halogen, Phenyl oder Aryl-C₁₋₇-Alkyl ist; oder Tetrahydropyran-2-yl;
R⁶ bis R⁹ Wasserstoff, Halogen, Trifluormethyl, C₁₋₇-Alkyl, C₁₋₇-Alkoxy, C₁₋₇-Alkylthio, Hydroxy, Hydroxymethyl, Cyano, Carboxyl, Formyl, Methylsulfinyl, Methylsulfonyl, Methylsulfonyloxy oder C₁₋₇-Alkyloxy-carbonyloxy;
R⁷ zusammen mit R⁶ oder R⁸ Butadienyl, Methylendioxy, Aethylendioxy oder Isopropylidendioxy;
Z -O-, -S-, Vinylen, -CO-, -OCHR¹⁰- oder -SCHR¹⁰;
R¹⁰ Wasserstoff oder C₁₋₇-Alkyl;
X und Y unabhängig voneinander O, S oder NH;
YR⁵ auch C₁₋₇-Alkylsulfinyl;
R^{a}, R^{b}, R^{c} und R^{d} unabhängig voneinander Wasserstoff oder C₁₋₇-Alkyl; oder
R^{c} und R^{d} zusammen Methylen, Aethylen oder Isopropyliden; und
n 1, 2 oder 3 bedeuten,
und Salze davon, dadurch gekennzeichnet, dass man
a) eine Verbindung der Formel 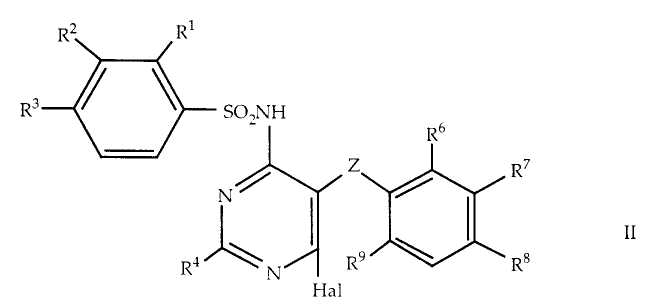 worin R¹-R⁴ und R⁶-R⁹ die oben genannte Bedeutung haben und Hal Halogen ist,
mit einer Verbindung der Formel
MXCH₂(CR^{a}R^{b})ₙYR⁵ III
worin X, Y, n, R^{a}, R^{b} und R⁵ die oben genannte Bedeutung haben, und
M ein Alkalimetall darstellt,
umsetzt, oder
b) eine Verbindung der Formel 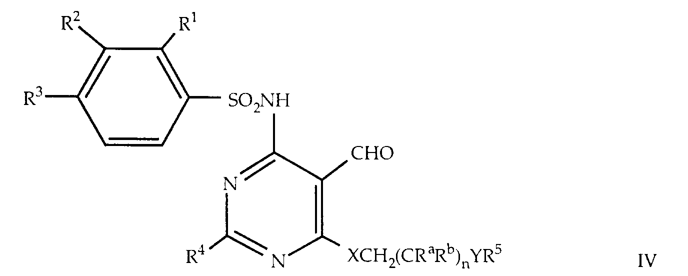 worin R¹-R⁵, R^{a}, R^{b}, X, Y und n die oben genannte Bedeutung haben, mit einer Verbindung der Formel 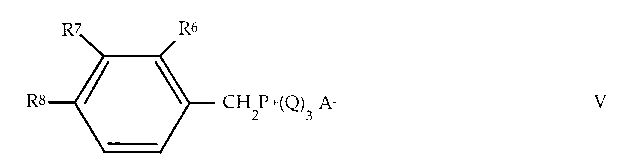 worin R⁶-R⁸ die oben genannte Bedeutung haben; Q Aryl und A⁻ ein Anion ist,
umsetzt, oder
c) eine Verbindung der Formel 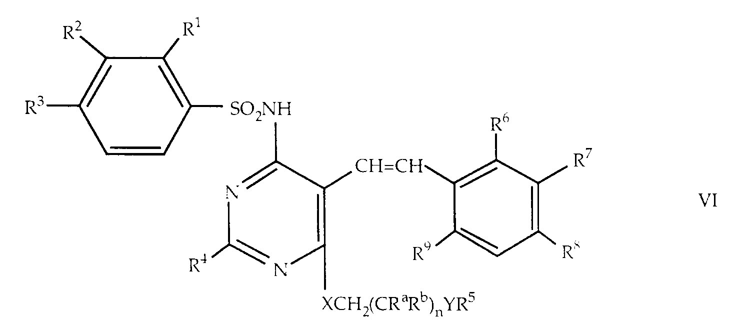 worin R¹-R⁸, R^{a}, R^{b}, X, Y und n die oben genannte Bedeutung haben, hydriert, oder
d) eine Verbindung der Formel 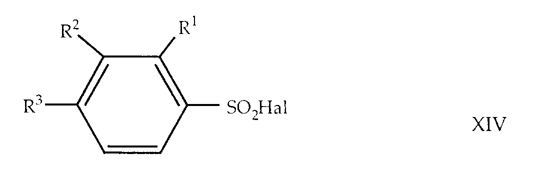 mit einer Verbindung der Formel 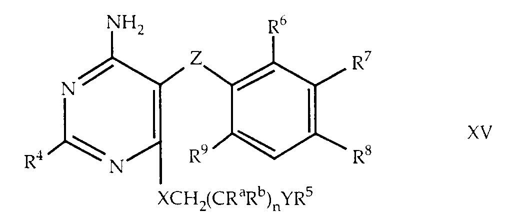 wobei R¹-R⁹, R^{a}, R^{b}, X, Y, Z und n die oben genannte Bedeutung haben, umsetzt, und gegebenenfalls in der erhaltenen Verbindung der Formel I enthaltene Substituenten abwandelt und/oder die erhaltene Verbindung der Formel I in ein Salz überführt.

2. Verfahren nach Anspruch 1 zur Herstellung von Verbindungen der Formel I und Salze davon, worin
R¹ Wasserstoff, C₁₋₇-Alkyl, C₁₋₇-Alkoxy, C₁₋₇-Alkylthio, Halogen oder Trifluormethyl;
R² Wasserstoff, Halogen, C₁₋₇-Alkoxy oder Trifluormethyl;
R³ Wasserstoff, Halogen, C₁₋₇-Alkyl, C₁₋₇-Alkylthio, Trifluormethyl, C₃₋₈-Cycloalkyl, C₁₋₇-Alkoxy oder Trifluormethoxy;
R² und R³ zusammen Butadienyl oder Methylendioxy;
R⁴ Wasserstoff, C₁₋₇-Alkyl, Trifluormethyl, C₁₋₇-Alkoxy, C₁₋₇-Alkylthio, C₁₋₇-Alkylthio-C₁₋₇-alkyl, Hydroxy-C₁₋₇-alkyl, Amino-C₁₋₇-alkyl, C₁₋₇-Alkylamino- C₁₋₇-alkyl, Di-C₁₋₇-alkylamino- C₁₋₇-alkyl, Amino, C₁₋₇-Alkylamino, Di-C₁₋₇-alkylamino, Arylamino, Aryl, Arylthio, Aryloxy, Aryl- C₁₋₇-alkyl, worin Aryl unsubstituiertes Phenyl oder Phenyl substituiert mit C₁₋₇-Alkyl, Trifluormethyl, C₁₋₇-Alkoxy, Carboxyl, Halogen ist, unsubstituiertes Heterocyclyl aus der Gruppe von 2-Furyl, 3-Furyl, Pyrimidinyl, 2-Pyridyl, 3-Pyridyl, 4-Pyridyl, 2-, 3-, oder 4-Pyridyl-N-oxid, 1,2- oder 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purrinolyl, Chinolyl, Isochinolyl, Chinazolyl oder Tetrahydropyran-2-yl oder Heterocyclyl, wie vorher definiert substituiert mit C₁₋₇-Alkyl, C₁₋₇-Alkoxy, Halogen, Aryl oder Aryl- C₁₋₇-alkyl;
R⁵ Wasserstoff, C₁₋₇-Alkanoyl, Benzoyl oder Tetrahydropyran-2-yl;
R⁶ bis R⁹ Wasserstoff, Halogen, Trifluormethyl, C₁₋₇-Alkyl, C₁₋₇-Alkoxy, C₁₋₇-Alkylthio, Hydroxy, Hydroxymethyl, Cyano, Carboxyl, Formyl, Methylsulfinyl, Methylsulfonyl, Methylsulfonyloxy oder C₁₋₇-Alkyloxy-carbonyloxy;
R⁷ zusammen mit R⁶ oder R⁸ Butadienyl oder -OCH₂O-;
Z -O-, -S-, Vinylen, -CO-, -OCHR¹⁰- oder -SCHR¹⁰;
R¹⁰ Wasserstoff oder C₁₋₇-Alkyl;
X und Y unabhängig voneinander O, S oder NH;
R^{a} und R^{b} Wasserstoff und
n 1, 2, oder 3 bedeuten.

3. Verfahren nach Anspruch 1 oder 2, zur Herstellung von Verbindungen der Formel I, in denen Z -O- ist.

4. Verfahren nach den Ansprüchen 1-3, zur Herstellung von Verbindungen der Formel I, in denen R⁶ C₁₋₇-Alkoxy und R⁷, R⁸ und R⁹ Wasserstoff bedeuten.

5. Verfahren nach den Ansprüchen 1-3, zur Herstellung von Verbindungen der Formel I, in denen R⁶ und R⁸ Wasserstoff, R⁷ C₁₋₇-Alkoxy und R⁹ Halogen bedeuten.

6. Verfahren nach den Ansprüchen 1-3, zur Herstellung von Verbindungender Formel I, in denen R⁴ Wasserstoff, 2-Pyrimidinyl, 2- oder 3-Furyl, 2- oder 3-Thienyl, Morpholino oder p-Methoxyphenyl ist.

7. Verfahren nach den Ansprüchen 1-3, zur Herstellung von Verbindungen der Formel I, in denen YR⁵ Hydroxy, C₁₋₇-Alkoxysulfinyl oder Furoyloxy ist.

8. Verfahren nach Anspruch 3 zur Herstellung der Verbindungen
p-t-Butyl-N-[6-(2-hydroxyäthoxy)-5-(o-methoxyphenoxy)-4-pyrimidinyl]-benzolsulfonamid,
N-[6-(Hydroxyäthoxy)-5-(o-methoxyphenoxy)-4-pyrimidinyl]-p-isopropyl-benzolsulfonamid,
p-t-Butyl-N-[6-(2-hydroxyäthoxy)-5-(o-tolyloxy)-4-pyrimidinyl]benzolsulfonamid,
p-t-Butyl-N-[6-(2-hydroxyäthoxy)-5-(o-chlorphenyloxy)-4-pyrimidinyl]benzolsulfonamid,
N-[6-(2-Hydroxyäthoxy)-5-(o-chlorphenoxy)-4-pyrimidinyl]-p-isopropylbenzolsulfonamid,
p-t-Butyl-N-[6-(2-hydroxyäthoxy)-5-(m-methoxyphenoxy)-4-pyrimidinyl]benzolsulfonamid,
p-t-Butyl-N-[6-(2-hydroxyäthoxy)-5-phenoxy-4-pyrimidinyl]benzolsulfonamid,
p-t-Butyl-N-[6-(2-hydroxyäthoxy)-5-(p-methoxyphenoxy)-4-pyrimidinyl]benzolsulfonamid,
p-t-Butyl-N-[6-(2-hydroxyäthoxy)-5-(o-äthoxyphenoxy)-4-pyrimidinyl]benzolsulfonamid,
p-(2,2-Dimethylpropyl)-N-[6-(2-hydroxyäthoxy)-5-(o-methoxyphenoxy)-4-pyrimidinyl]benzolsulfonamid,
p-Isopropyl-N-[6-(2-hydroxyäthoxy)-2-methyl-5-(m-methoxyphenoxy)-4-pyrimidinyl]benzolsulfonamid,
N-[6-(2-Hydroxyäthoxy)-5-(o-methoxyphenoxy)-2-phenyl-4-pyrimidinyl]-p-isopropylbenzolsulfonamid,
N-[6-(2-Hydroxyäthoxy)-5-(2,4,6-trichlorphenoxy)-4-pyrimidinyl]-p-isopropylbenzolsulfonamid,
N-[6-(2-Hydroxyäthoxy)-6-(2,4,6-trichlorphenoxy)-4-pyrimidinyl]-o-toluolsulfonamid,
N-[6-(2-Hydroxyäthoxy)-5-(2,4,6-trichlorphenoxy)-4-pyrimidinyl]-2,4-xylolsulfonamid,
p-t-Butyl-N-[6-(2-hydroxyäthoxy)-5-[(2-methoxy-p-tolyl)oxy]-4-pyrimidinyl]benzolsulfonamid,
p-tert-Butyl-N-[6-(2-hydroxyäthoxy)-5-(o-methoxyphenoxy)-2-methyl-4-pyrimidinyl]benzolsulfonamid,
N-[6-(2-Hydroxyäthoxy)-5-(o-methoxyphenoxy)-2-methyl-4-pyrimidinyl)-p-isopropylbenzolsulfonamid,
N-[6-(2-Hydroxyäthoxy)-5-(o-methoxyphenoxy)-(2-trifluormethyl)-4-pyrimidinyl]-p-isopropylbenzolsulfonamid,
p-tert-Butyl-N-[6-(2-hydroxyäthoxy)-5-(o-methoxyphenoxy)-2-(trifluormethyl)-4-pyrimidinyl]benzolsulfonamid,
N-[5-(1,3-Benzodioxol-5-yloxy)-6-(2-hydroxyäthoxy)-4-pyrimidinyl]-p-tert-butylbenzolsulfonamid,
N-[5-(1,3-Benzodioxol-5-yloxy)-6-(2-hydroxyäthoxy)-4-pyrimidinyl]-p-isopropylbenzolsulfonamid,
N-[6-(2-Hydroxyäthoxy)-5-(o-methoxyphenoxy)-4-pyrimidinyl]-o-methoxybenzolsulfonamid,
p-tert-Butyl-N-[6-(4-hydroxybutoxy)-5-(o-methoxyphenoxy)-2-methyl-4-pyrimidinyl]benzolsulfonamid
N-[6-(2-Hydroxyäthoxy)-5-(2-naphthyloxy)-4-pyrimidinyl]-p-isopropylbenzolsulfonamid,
N-[6-(2-Hydroxyäthoxy)-5-(2-naphthyloxy)-4-pyrimidinyl]-p-tert-butylbenzolsulfonamid,
N-[6-(2-Hydroxyäthoxy)-5-(o-methoxyphenoxy)-2-propyl-4-pyrimidinyl]-p-isopropylbenzolsulfonamid,
p-tert-Butyl-N-[6-(2-hydroxyäthoxy)-5-(o-methoxyphenoxy)-2-propyl-4-pyrimidinyl]benzolsulfonamid,
α,α,α-Trifluor-N-[6-(2-hydroxyäthoxy)-5-(o-methoxyphenoxy)-2-methyl-4-pyrimidinyl]-p-toluolsulfonamid,
p-Chlor-N-[6-(2-hydroxyäthoxy)-5-(o-methoxyphenoxy)-2-methyl-4-pyrimidinyl]benzolsulfonamid,
N-[6-(2-Hydroxyäthoxy)-5-(o-methoxyphenoxy)-2-methyl-4-pyrimidinyl]-p-(trifluormethoxy)benzolsulfonamid,
o-Aethyl-N-[6-(2-hydroxyäthoxy)-5-(o-methoxyphenoxy)-2-methyl-4-pyrimidinyl]benzolsulfonamid,
N-[6-(2-Hydroxyäthoxy)-5-(o-methoxyphenoxy)-2-methyl-4-pyrimidinyl]-p-toluolsulfonamid,
N-[6-(2-Hydroxyäthoxy)-5-(o-methoxyphenoxy)-2-methyl-4-pyrimidinyl]-2-naphthylsulfonamid,
p-tert-Butyl-N-[6-(3-hydroxypropoxy)-5-(o-methoxyphenoxy)-2-methyl-4-pyrimidinyl]benzolsulfonamid,
p-t-Butyl-N-[6-(2-hydroxyäthoxy)-5-[(o-methylthio)phenoxy]-4-pyrimidinyl]benzolsulfonamid,
p-t-Butyl-N-[6-(2-hydroxyäthoxy)-5-(o-methoxyphenoxy)-2-phenyl-4-pyrimidinyl]benzolsulfonamid,
N-[2-Amino-6-(2-hydroxyäthoxy)-5-(o-methoxyphenoxy)-4-pyrimidinyl]-p-t-butylbenzolsulfonamid,
p-t-Butyl-N-[6-(2-hydroxyäthoxy)-2-methyl-5-[o-(methylthio)phenoxy]-4-pyrimidinyl]benzolsulfonamid und
p-t-Butyl-N-[6-(2-hydroxyäthoxy)-2-methyl-5-[o-(R,S-methylsulfinyl)-phenoxy]-4-pyrimidinyl]benzolsulfonamid.

9. Verfahren nach Anspruch 3 zur Herstellung der Verbindungen
4-tert-Butyl-N-[5-(2-chlor-5-methoxy-phenoxy)-6-(2-hydroxy-äthoxy)-2-methyl-pyrimidin-4-yl]-benzolsulfonamid,
4-tert-Butyl-N-[5-(2-chlor-5-methoxy-phenoxy)-6-(2-(3-furoyloxy)äthoxy)-2-methyl-pyrimidin-4-yl]-benzolsulfonamid,
4-tert-Butyl-N-[6-(2-hydroxy-äthoxy)-5-(2-methoxy-phenoxy)-2-(thiophen-2-yl)-pyrimidin-4-yl]-benzolsulfonamid,
4-tert-Butyl-N-[6-(2-hydroxy-äthoxy)-5-(2-methoxy-phenoxy)-2-(thiophen-3-yl)-pyrimidin-4-yl]-benzolsulfonamid,
4-tert-Butyl-N-[2-(furan-2-yl)-6-(2-hydroxy-äthoxy)-5-2-methoxy-phenoxy)-pyrimidin-4-yl]-benzolsulfonamid,
4-tert-Butyl-N-[2-furan-3-yl-6-(2-hydroxy-äthoxy)-5-(2-methoxyphenoxy)-pyrimidin-4-yl]-benzolsulfonamid,
4-tert-Butyl-N-[6-(2-hydroxy-äthoxy)-5-(2-methoxy-phenoxy)-2-(pyridin-2-yl)-pyrimidin-4-yl]-benzolsulfonamid,
4-tert-Butyl-N-[6-(2-hydroxy-äthoxy)-5-(2-methoxy-phenoxy)-2-pyridin-4-yl)-pyrimidin-4-yl]-benzolsulfonamid,
4-tert-Butyl-N-[6-(2-hydroxy-äthoxy)-5-(2-methoxy-phenoxy)-2-pyridin-3-yl)-pyrimidin-4-yl]-benzolsulfonamid,
2-[4-(4-tert-Butyl-phenylsulfonylamino)-6-(2-hydroxy-äthoxy)-5-(2-methoxy-phenoxy)-pyrimidin-2-yl]-pyridin-1-oxid,
4-[4-(4-tert-Butyl-phenylsulfonylamino)-6-(2-hydroxy-äthoxy)-5-(2-methoxy-phenoxy)-pyrimidin-2-yl]-pyridin-1-oxid,
4-tert-Butyl-N-[6-(2-hydroxy-äthoxy)-2-[2-hydroxy-äthoxy)-äthyl]-5-(2-methoxy-phenoxy)-pyrimidin-4-yl]-benzolsulfonamid,
4-tert-Butyl-N-[2-cyclopropyl-6-(2-hydroxy-äthoxy)-5-(2-methoxy-phenoxy)-pyrimidin-4-yl]-benzolsulfonamid,
4-tert-Butyl-N-[2-äthyl-6-(2-hydroxy-äthoxy)-5-(2-methoxy-phenoxy)-pyrimidin-4-yl]-benzolsulfonamid,
4-tert-Butyl-N-[6-(2-hydroxy-äthoxy)-2-isopropyl-5-(2-methoxy-phenoxy)-pyrimidin-4-yl]-benzolsulfonamid,
N-[5-(5-Fluor-2-methoxy-phenoxy)-6-(2-hydroxy-äthoxy)-pyrimidin-4-yl]-4-trifluormethyl-benzolsulfonamid,
4-tert-Butyl-N-[6-(2-hydroxy-äthoxy)-5-(3-methoxy-phenoxy)-2,2'-bipyrimidin-4-yl]-benzolsulfonamid,
4-tert-Butyl-N-[5-(4-fluor-2-methoxy-phenoxy)-6-(2-hydroxy-äthoxy)-2,2'-bipyrimidin-4-yl]-benzolsulfonamid,
4-tert-Butyl-N-[5-(4-fluor-2-methoxy-phenoxy)-6-(2-hydroxy-äthoxy)-2-methyl-pyrimidin-4-yl]-benzolsulfonamid,
4-tert-Butyl-N-[5-(4-fluor-2-methoxy-phenoxy)-6-(2-hydroxy-äthoxy)-pyrimidin-4-yl]-benzolsulfonamid,
N-[5-(5-Fluor-2-methoxy-phenoxy)-6-(2-hydroxy-äthoxy)-pyrimidin-4-yl]-4-isopropyl-benzolsulfonamid,
N-[5-(5-Fluor-2-methoxy-phenoxy)-6-(2-hydroxy-äthoxy)-pyrimidin-4-yl]-4-tert-butyl-benzolsulfonamid,
4-tert-Butyl-N-[5-(2-fluor-6-methoxy)-6-(2-hydroxy-äthoxy)-pyrimidin-4-yl]-benzolsulfonamid,
4-tert-Butyl-N-[6-(2-hydroxy-äthoxy)-5-(3-methoxy-phenoxy)-2-(thiophen-2-yl)-pyrimidin-4-yl]-benzolsulfonamid,
4-tert-Butyl-N-[6-(2-hydroxy-äthoxy)-2-(2-methoxy-äthyl)-5-(3-methoxy-phenoxy)-pyrimidin-4-yl]-benzolsulfonamid,
3-Methylisoxazol-5-carbonsäure-2-[6-(4-t-butylbenzolsulfonamino)-5-(3-methoxyphenoxy)pyrimidin-4-yloxy]äthylester,
Indol-2-carbonsäure-2-[6-(4-t-butylbenzolsulfonamino)-5-(3-methoxyphenoxy)pyrimidin-4-yloxy]äthylester,
5-[N-[6-(2-Hydroxyäthoxy)-5-(2-methoxyphenoxy)pyrimidin-4-yl]aminosulfonyl]-2-methoxy-phenoxyessigsäureäthylester.

10. Verfahren nach Anspruch 3 zur Herstellung von (RS)-4-tert-Butyl-N-[5-(2-chlor-5-methoxy-phenoxy)-2-äthyl-6-(2-methylsulfinyl-äthoxy)-pyrimidin-4-yl]-benzolsulfonamid.

11. Verfahren nach Anspruch 3 zur Herstellung von (RS)-N-[5-(2-Chlor-5-methoxy-phenoxy)-6-(2-methylsulfinyl-äthoxy)-pyrimidin-4-yl]-1,3-benzodioxol-5-sulfonamid.

12. Verfahren nach Anspruch 3 zur Herstellung von 4-tert-Butyl-N-[6-(2-hydroxy-äthoxy)-5-(2-methoxy-phenoxy)-2-(pyrimidin-2-yl)-pyrimidin-4-yl]-benzolsulfonamid.

13. Verfahren nach den Ansprüchen 1 oder 2 zur Herstellung von Verbindungen der Formel I, in denen Z Vinylen ist.

14. Verfahren nach Anspruch 14 zur Herstellung der Verbindungen
2-[[5-[(E/Z)-Styryl]-6-p-toluolsulfonamid-4-pyrimidinyl]oxy]äthylacetat,
N-[6-(2-Hydroxyäthoxy)-5-[(E/Z)-styryl]-4-pyrimidinyl]-p-toluolsulfonamid.

## Claims (Claims for the following Contracting State(s): AT, BE, CH, DE, DK, FR, GB, IT, LI, LU, MC, NL, PT, SE)

1. Compounds of the formula 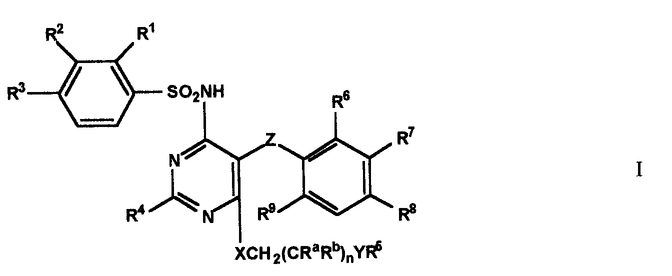 wherein
R¹ signifies hydrogen, C₁₋₇-alkyl, C₁₋₇-alkoxy, C₁₋₇-alkylthio, halogen or trifluoromethyl;
R² signifies hydrogen, halogen, C₁₋₇-alkoxy, trifluoromethyl or -OCH₂COOR^{a};
R³ signifies hydrogen, halogen, C₁₋₇-alkyl, C₁₋₇-alkylthio, trifluoromethyl, C₃₋₈-cycloalkyl, C₁₋₇-alkoxy or trifluoromethoxy;
R² and R³ together signify butadienyl, methylenedioxy, ethylenedioxy or isopropylidenedioxy;
R⁴ signifies hydrogen, C₁₋₇-alkyl, C₃₋₈-cycloalkyl, trifluoromethyl, C₁₋₇-alkoxy, C₁₋₇-alkylthio, C₁₋₇-alkylthio-C₁₋₇-alkyl, hydroxy-C₁₋₇-alkyl, hydroxy-C₁₋₇-alkoxy, C₁₋₇-alkoxy-C₁₋₇-alkyl, hydroxy-C₁₋₇-alkoxy- C₁₋₇-alkyl, hydroxy-C₁₋₇-alkoxy-C₁₋₇-alkoxy, C₁₋₇-alkylsulphinyl, C₁₋₇-alkylsulphonyl, 2-methoxy-3-hydroxypropoxy, 2-hydroxy-3-phenylpropyl, amino-C₁₋₇-alkyl, C₁₋₇-alkylamino-C₁₋₇-alkyl, di-C₁₋₇-alkylamino-C₁₋₇-alkyl, amino, C₁₋₇-alkylamino, di-C₁₋₇-alkylamino, arylamino, aryl, arylthio, aryloxy, aryl-C₁₋₇-alky, wherein aryl represents unsubstituted phenyl or phenyl substituted with C₁₋₇-alkyl, trifluoromethyl, C₁₋₇-alkoxy, carboxyl or halogen, unsubstituted heterocyclyl from the group of 2-furyl, 3-furyl, pyrimidinyl, 2-pyridyl, 3-pyridyl, 4-pyridyl or 2, 3, or 4-pyridyl N-oxide, 1,2- or 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinyl, quinolyl, isoquinolyl, quinazolyl or heterocyclyl as hereinbefore defined substituted with C₁₋₇-alkyl, C₁₋₇-alkoxy, halogen, aryl or aryl-C₁₋₇-alkyl;
R⁵ signifies hydrogen, C₁₋₇-alkyl, C₁₋₇-alkanoyl, benzoyl, heterocyclylcarbonyl, heterocyclylmethyl, wherein heterocyclyl is selected from unsubstituted 2-furyl, 3-furyl, pyrimidinyl, 2-pyridyl, 3-pyridyl, 4-pyridyl, 2-, 3-, or 4-pyridyl N-oxide, 1,2- or 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinyl, quinolyl, isoquinolyl, quinazolyl or heterocyclyl as hereinbefore defined substituted with C₁₋₇-alkyl, C₁₋₇-alkoxy, halogen, phenyl or aryl-C₁₋₇-alkyl; or tetrahydropyran-2-yl;
R⁶ to R⁹ signify hydrogen, halogen, trifluoromethyl, C₁₋₇-alkyl, C₁₋₇-alkoxy, C₁₋₇-alkylthio, hydroxy, hydroxymethyl, cyano, carboxyl, formyl, methylsulphinyl, methylsulphonyl, methylsulphonyloxy or C₁₋₇-alkyloxy-carbonyloxy;
R⁷ together with R⁶ or R⁸ signify butadienyl, methylenedioxy, ethylenedioxy or isopropylidenedioxy;
Z signifies -O-, -S-, vinylene, -CO-, -OCHR¹⁰- or -SCHR¹⁰-;
R¹⁰ signifies hydrogen or C₁₋₇-alkyl;
X and Y each independently signify O, S or NH;
YR⁵ also signifies C₁₋₇-alkylsulphinyl;
R^{a}, R^{b}, R^{c} and R^{d} each independently signify hydrogen or C₁₋₇-alkyl; or
R^{c} and R^{d} together signify methylene, ethylene or isopropylidene; and
n signifies 1, 2 or 3,
and salts thereof.

2. Compounds of formula I of claim 1, wherein
R¹ signifies hydrogen, C₁₋₇-alkyl, C₁₋₇alkoxy, C₁₋₇-alkylthio, halogen or trifluoromethyl;
R² signifies hydrogen, halogen, C₁₋₇-alkoxy or trifluoromethyl;
R³ signifies hydrogen, halogen, C₁₋₇-alkyl, C₁₋₇-alkylthio, trifluoromethyl, C₃₋₈-cycloalkyl, C₁₋₇-alkoxy or trifluoromethoxy;
R² and R³ together signify butadienyl or methylenedioxy;
R⁴ signifies hydrogen, C₁₋₇-alkyl, trifluoromethyl, C₁₋₇-alkoxy, C₁₋₇-alkylthio, C₁₋₇-alkylthio-C₁₋₇-alkyl, hydroxy-C₁₋₇-alkyl, amino-C₁₋₇-alkyl, C₁₋₇-alkylamino-C₁₋₇-alkyl, di-C₁₋₇-alkylamino-C₁₋₇-alkyl, amino, C₁₋₇-alkylamino, di-C₁₋₇-alkylamino, arylamino, aryl, arylthio, aryloxy, aryl-C₁₋₇-alkyl, wherein aryl represents unsubstituted phenyl or phenyl substituted with C₁₋₇-alkyl, trifluoromethyl, C₁₋₇-alkoxy, carboxyl or halogen, unsubstituted heterocyclyl from the group of 2-furyl, 3-furyl, pyrimidinyl, 2-pyridyl, 3-pyridyl, 4-pyridyl, 2-, 3-, or 4-pyridyl N-oxide, 1,2- or 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinolyl, quinolyl, isoquinolyl, quinazolyl or tetrahydropyran-2-yl or heterocyclyl as hereinbefore defined substituted with C₁₋₇-alkyl, C₁₋₇-alkoxy, halogen, aryl or aryl-C₁₋₇-alkyl;
R⁵ signifies hydrogen, C₁₋₇-alkyl, C₁₋₇-alkanoyl, benzoyl or tetrahydropyran-2-yl;
R⁶ to R⁹ signify hydrogen, halogen, trifluoromethyl, C₁₋₇-alkyl, C₁₋₇-alkoxy, C₁₋₇-alkylthio, hydroxy, hydroxymethyl, cyano, carboxyl, formyl, methylsulphinyl, methylsulphonyl, methylsulphonyloxy or C₁₋₇-alkyloxy-carbonyloxy;
R⁷ together with R⁶ or R⁸ signify butadienyl or -OCH₂O-;
Z signifies -O-, -S-, vinylene, -CO-, -OCHR¹⁰- or -SCHR¹⁰-;
R¹⁰ signifies hydrogen or C₁₋₇-alkyl;
X and Y each independently signify O, S or NH;
R^{a} and R^{b} signify hydrogen and
n signifies 1, 2 or 3,
and salts thereof.

3. Compounds according to claim 1 or 2, in which Z is -O-.

4. Compounds according to claims 1-3, in which R⁶ signifies C₁₋₇-alkoxy and R⁷, R⁸ and R⁹ signify hydrogen.

5. Compounds according to claims 1-3, in which R⁶ and R⁸ signify hydrogen, R⁷ signifies C₁₋₇-alkoxy and R⁹ signifies halogen.

6. Compounds according to claims 1-5, in which R⁴ signifies hydrogen, 2-pyrimidinyl, 2- or 3-furyl, 2- or 3-thienyl, morpholino or p-methoxyphenyl.

7. Compounds according to claims 1-6, in which YR⁵ is hydroxy, C₁₋₇-alkoxysulphinyl or furoyloxy.

8. The compounds according to claim 3,
p-t-butyl-N-[6-(2-hydroxyethoxy)-5-(o-methoxyphenoxy)-4-pyrimidinyl]benzenesulphonamide,
N-[6-(hydroxyethoxy)-5-(o-methoxyphenoxy)-4-pyrimidinyl]-p-isopropylbenzenesulphonamide,
p-t-butyl-N-[6-(2-hydroxyethoxy)-5-(o-tolyloxy)-4-pyrimidinyl]benzenesulphonamide,
p-t-butyl-N-[6-(2-hydroxyethoxy)-5-(o-chlorophenyloxy)-4-pyrimidinyl]benzenesulphonamide,
N-[6-(2-hydroxyethoxy)-5-(o-chlorophenoxy)-4-pyrimidinyl]-p-isopropylbenzenesulphonamide,
p-t-butyl-N-[6-(2-hydroxyethoxy)-5-(m-methoxyphenoxy)-4-pyrimidinyl]benzenesulphonamide,
p-t-butyl-N-[6-(2-hydroxyethoxy)-5-phenoxy-4-pyrimidinyl]benzenesulphonamide,
p-t-butyl-N-[6-(2-hydroxyethoxy)-5-(p-methoxyphenoxy)-4-pyrimidinyl]benzenesulphonamide,
p-t-butyl-N-[6-(2-hydroxyethoxy)-5-(o-ethoxyphenoxy)-4-pyrimidinyl]benzenesulphonamide,
p-(2,2-dimethylpropyl)-N-[6-(2-hydroxyethoxy)-5-(o-methoxyphenoxy)-4-pyrimidinyl]benzenesulphonamide,
p-isopropyl-N-[6-(2-hydroxyethoxy)-2-methyl-5-(m-methoxyphenoxy)-4-pyrimidinyl]benzenesulphonamide,
N-[6-(2-hydroxyethoxy)-5-(o-methoxyphenoxy)-2-phenyl-4-pyrimidinyl]-p-isopropylbenzenesulphonamide,
N-[6-(2-hydroxyethoxy)-5-(2,4,6-trichlorophenoxy)-4-pyrimidinyl]-p-isopropylbenzenesulphonamide,
N-[6-(2-hydroxyethoxy)-6-(2,4,6-trichlorophenoxy)-4-pyrimidinyl]-o-toluenesulphonamide,
N-[6-(2-hydroxyethoxy)-5-(2,4,6-trichlorophenoxy)-4-pyrimidinyl]-2,4-xylenesulphonamide,
p-t-butyl-N-[6-(2-hydroxyethoxy)-5-[(2-methoxy-p-tolyl)oxy]-4-pyrimidinyl]benzenesulphonamide,
p-tert-butyl-N-[6-(2-hydroxyethoxy)-5-(o-methoxyphenoxy)-2-methyl-4-pyrimidinyl]benzenesulphonamide,
N-[6-(2-hydroxyethoxy)-5-(o-methoxyphenoxy)-2-methyl-4-pyrimidinyl)-p-isopropylbenzenesulphonamide,
N-[6-(2-hydroxyethoxy)-5-(o-methoxyphenoxy)-(2-trifluoromethyl)-4-pyrimidinyl)-p-isopropylbenzenesulphonamide,
p-tert-butyl-N-[6-(2-hydroxyethoxy)-5-(o-methoxyphenoxy)-2-(trifluoromethyl)-4-pyrimidinyl]benzenesulphonamide,
N-[5-(1,3-benzodioxol-5-yloxy)-6-(2-hydroxyethoxy)-4-pyrimidinyl]-p-tert-butylbenzenesulphonamide,
N-[5-(1,3-benzodioxol-5-yloxy)-6-(2-hydroxyethoxy)-4-pyrimidinyl]-p-isopropylbenzenesulphonamide,
N-[6-(2-hydroxyethoxy)-5-(o-methoxyphenoxy)-4-pyrimidinyl)-o-methoxybenzenesulphonamide,
p-tert-butyl-N-[6-(4-hydroxybutoxy)-5-(o-methoxyphenoxy)-2-methyl-4-pyrimidinyl]benzenesulphonamide,
N-[6-(2-hydroxyethoxy)-5-(2-naphthyloxy)-4-pyrimidinyl]-p-isopropylbenzenesulphonamide,
N-[6-(2-hydroxyethoxy)-5-(2-naphthyloxy)-4-pyrimidinyl]-p-tert-butylbenzenesulphonamide,
N-[6-(2-hydroxyethoxy)-5-(o-methoxyphenoxy)-2-propyl-4-pyrimidinyl]-p-isopropylbenzenesulphonamide,
p-tert-butyl-N-[6-(2-hydroxyethoxy)-5-(o-methoxyphenoxy)-2-propyl-4-pyrimidinyl]benzenesulphonamide,
α,α,α-trifluoro-N-[6-(2-hydroxyethoxy)-5-(o-methoxyphenoxy)-2-methyl-4-pyrimidinyl]-p-toluenesulphonamide,
p-chloro-N-[6-(2-hydroxyethoxy)-5-(o-methoxyphenoxy)-2-methyl-4-pyrimidinyl]benzenesulphonamide,
N-[6-(2-hydroxyethoxy)-5-(o-methoxyphenoxy)-2-methyl-4-pyrimidinyl]-p-(trifluoromethoxy)benzenesulphonamide,
o-ethyl-N-[6-(2-hydroxyethoxy)-5-(o-methoxyphenoxy)-2-methyl-4-pyrimidinyl]benzenesulphonamide,
N-[6-(2-hydroxyethoxy)-5-(o-methoxyphenoxy)-2-methyl-4-pyrimidinyl]-p-toluenesulphonamide,
N-[6-(2-hydroxyethoxy)-5-(o-methoxyphenoxy)-2-methyl-4-pyrimidinyl]-2-naphthylsulphonamide,
p-tert-butyl-N-[6-(3-hydroxypropoxy)-5-(o-methoxyphenoxy)-2-methyl-4-pyrimidinyl]benzenesulphonamide,
p-t-butyl-N-[6-(2-hydroxyethoxy)-5-[(o-methylthio)phenoxy]-4-pyrimidinyl]benzenesulphonamide,
p-t-butyl-N-[6-(2-hydroxyethoxy)-5-(o-methoxyphenoxy)-2-phenyl-4-pyrimidinyl]benzenesulphonamide,
N-[2-amino-6-(2-hydroxyethoxy)-5-(o-methoxyphenoxy)-4-pyrimidinyl]p-t-butylbenzenesulphonamide,
p-t-butyl-N-[6-(2-hydroxyethoxy)-2-methyl-5-[(o-methylthio)phenoxy)-4-pyrimidinyl]benzenesulphonamide and
p-t-butyl-N-[6-(2-hydroxyethoxy)-2-methyl-5-[o-(R,S-methylsulphinyl)phenoxyl-4-pyrimidinyl]benzenesulphonamide.

9. The compounds according to claim 3,
4-tert-butyl-N-[5-(2-chloro-5-methoxy-phenoxy)-6-(2-hydroxy-ethoxy)-2-methylpyrimidin-4-yl]-benzenesulphonamide,
4-tert-butyl-N-[5-(2-chloro-5-methoxy-phenoxy)-6-(2-(3-furoyloxy)ethoxy)-2-methylpyrimidin-4-yl]-benzenesulphonamide,
4-tert-butyl-N-[6-(2-hydroxy-ethoxy)-5-(2-methoxy-phenoxy)-2-(thiophen-2-yl)-pyrimidin-4-yl]-benzenesulphonamide,
4-tert-butyl-N-[6-(2-hydroxy-ethoxy)-5-(2-methoxy-phenoxy)-2-(thiophen-3-yl)-pyrimidin-4-yl]-benzenesulphonamide,
4-tert-butyl-N-[2-(furan-2-yl)-6-(2-hydroxy-ethoxy)-5-(2-methoxy-phenoxy)-pyrimidin-4-yl]-benzenesulphonamide,
4-tert-butyl-N-[2-furan-3-yl-6-(2-hydroxy-ethoxy)-5-(2-methoxy-phenoxy)-pyrimidin-4-yl]-benzenesulphonamide,
4-tert-butyl-N-[6-(2-hydroxy-ethoxy)-5-(2-methoxy-phenoxy)-2-(pyridin-2-yl)-pyrimidin-4-yl]-benzenesulphonamide,
4-tert-butyl-N-[6-(2-hydroxy-ethoxy)-5-(2-methoxy-phenoxy)-2-pyridin-4-yl)-pyrimidin-4-yl]-benzenesulphonamide,
4-tert-butyl-N-[6-(2-hydroxy-ethoxy)-5-(2-methoxy-phenoxy)-2-pyridin-3-yl)-pyrimidin-4-yl]-benzenesulphonamide,
2-[4-(4-tert-butyl-phenylsulphonylamino)-6-(2-hydroxy-ethoxy)-5-(2-methoxy-phenoxy)-pyrimidin-2-yl]-pyridine 1-oxide,
4-[4-(4-tert-butyl-phenylsulphonylamino)-6-(2-hydroxy-ethoxy)-5-(2-methoxy-phenoxy)-pyrimidin-2-yl]-pyridine 1-oxide,
4-tert-butyl-N-[6-(2-hydroxy-ethoxy)-2-[2-hydroxy-ethoxy)-ethyl]-5-(2-methoxy-phenoxy)-pyrimidin-4-yl]-benzenesulphonamide,
4-tert-butyl-N-[2-cyclopropyl-6-(2-hydroxy-ethoxy)-5-(2-methoxy-phenoxy)-pyrimidin-4-yl]-benzenesulphonamide,
4-tert-butyl-N-[2-ethyl-6-(2-hydroxy-ethoxy)-5-(2-methoxy-phenoxy)-pyrimidin-4-yl]-benzenesulphonamide,
4-tert-butyl-N-[6-(2-hydroxy-ethoxy)-2-isopropyl-5-(2-methoxy-phenoxy)-pyrimidin-4-yl]-benzenesulphonamide,
N-[5-(5-fluoro-2-methoxy-phenoxy)-6-(2-hydroxy-ethoxy)-pyrimidin-4-yl]-4-trifluoromethyl-benzenesulphonamide,
4-tert-butyl-N-[6-(2-hydroxy-ethoxy)-5-(3-methoxy-phenoxy)-2,2'-bipyrimidin-4-yl]-benzenesulphonamide,
4-tert-butyl-N-[5-(4-fluoro-2-methoxy-phenoxy)-6-(2-hydroxy-ethoxy)-2,2'-bipyrimidin-4-yl]-benzenesulphonamide,
4-tert-butyl-N-[5-(4-fluoro-2-methoxy-phenoxy)-6-(2-hydroxy-ethoxy)-2-methylpyrimidin-4-yl]-benzenesulphonamide,
4-tert-butyl-N-[5-(4-fluoro-2-methoxy-phenoxy)-6-(2-hydroxy-ethoxy)-pyrimidin-4-yl]-benzenesulphonamide,
N-[5-(5-fluoro-2-methoxy-phenoxy)-6-(2-hydroxy-ethoxy)-pyrimidin-4-yl]-4-isopropyl-benzenesulphonamide,
N-[5-(5-fluoro-2-methoxy-phenoxy)-6-(2-hydroxy-ethoxy)-pyrimidin-4-yl]-4-tert-butyl-benzenesulphonamide,
4-tert-butyl-N-[5-(2-fluoro-6-methoxy)-6-(2-hydroxy-ethoxy)-pyrimidin-4-yl]-benzenesulphonamide,
4-tert-butyl-N-[6-(2-hydroxy-ethoxy)-5-(3-methoxy-phenoxy)-2-(thiophen-2-yl)-pyrimidin-4-yl]-benzenesulphonamide,
4-tert-butyl-N-[6-(2-hydroxy-ethoxy)-2-(2-methoxy-ethyl)-5-(3-methoxy-phenoxy)-pyrimidin-4-yl]-benzenesulphonamide,
2-[6-(4-t-butylbenzenesulphonamino)-5-(3-methoxyphenoxy)pyrimidin-4-yloxy]ethyl 3-methylisoxazole-5-carboxylate,
2-[6-(4-t-butylbenzenesulphonamino)-5-(3-methoxyphenoxy)pyrimidin-4-yloxy]ethyl indole-2-carboxylate,
ethyl 5-[N-[6-(2-hydroxyethoxy)-5-(2-methoxyphenoxy)pyrimidin-4-yl]aminosulphonyl]-2-methoxy-phenoxyacetate.

10. The compound according to claim 1, characterized in that it is (RS)-4-tert-butyl-N-[5-(2-chloro-5-methoxy-phenoxy)-2-ethyl-6-(2-methylsulphinyl-ethoxy)-pyrimidin-4-yl]-benzenesulphonamide.

11. The compound according to claim 1, characterized in that it is (RS)-N-[5-(2-chloro-5-methoxy-phenoxy)-6-(2-methylsulphinyl-ethoxy)-pyrimidin-4-yl]-1,3-benzodioxol-5-sulphonamide.

12. The compound according to claim 1, characterized in that it is 4-tert-butyl-N-[6-(2-hydroxy-ethoxy)-5-(2-methoxy-phenoxy)-2-(pyrimidin-2-yl)-pyrimidin-4-yl]-benzenesulphonamide.

13. Compounds according to claim 1 or 2 in which Z is vinylene.

14. The compounds according to claim 13
2-[[5-[(E/Z)-styryl]-6-p-toluenesulphonamido-4-pyrimidinyl]oxy]ethyl acetate,
N-[6-(2-hydroxyethoxy)-5-[(E/Z)-styryl]-4-pyrimidinyl]-p-toluenesulphonamide.

15. Compounds of the formula 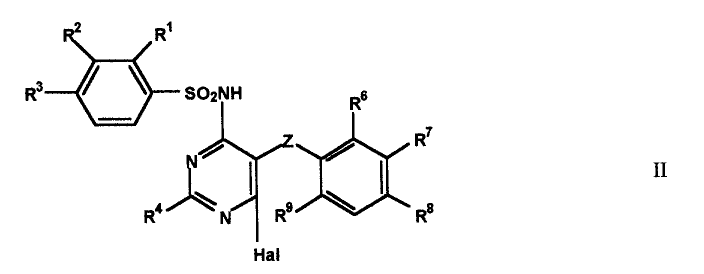 wherein R¹-R⁴, R⁶-R⁹ and Z have the significance given in claim 1 and Hal is halogen.

16. The compounds of claims 1-14 for use as medicaments.

17. Pharmaceutical preparations, containing a compound of claims 1-14 and usual carriers and adjuvants.

18. The use of compounds of claims 1-14 as active substances in the production of medicaments for the treatment of disorders which are associated with endothelin activities, especially circulatory disorders such as hypertension, ischaemia, vasospasms and angina pectoris.

19. A process for the manufacture of compounds of claims 1-14, characterized by
a) reacting a compound of the formula 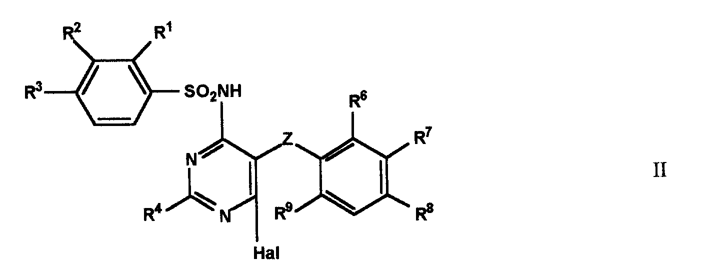 wherein R¹-R⁴, R⁶-R⁹ and Z have the significance given in claim 1 and Hal is halogen, with a compound of the formula
MXCH₂(CR^{a}R^{b})ₙYR⁵ III
wherein X, Y, n, R^{a}, R^{b} and R⁵ have the significance given in claim 1 and M represents an alkali metal,
or
b) reacting a compound of the formula 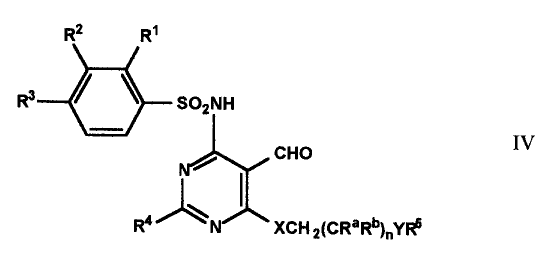 wherein R¹-R⁵, R^{a}, R^{b}, X, Y and n have the significance given above, with a compound of the formula 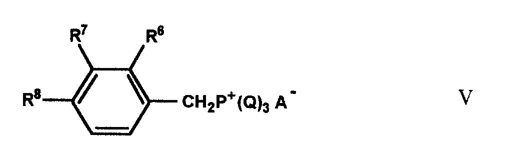 wherein R⁶-R⁸ have the significance given above; Q is aryl and A⁻ is an anion,
or
c) hydrogenating a compound of the formula 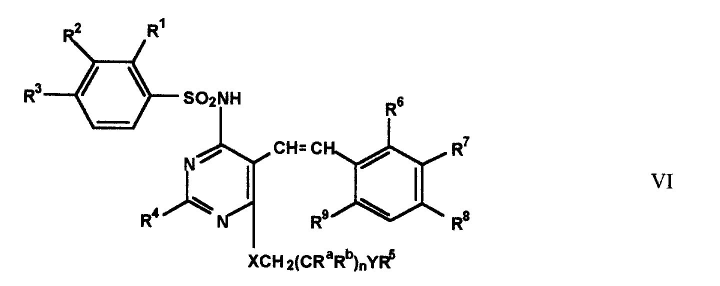 wherein R¹-R⁹, R^{a}, R^{b}, X, Y and n have the significance given above,
or
d) reading a compound of the formula 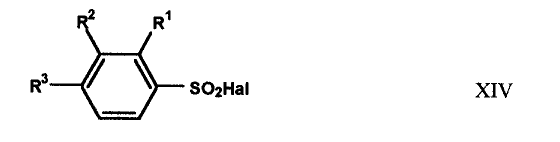 with a compound of the formula 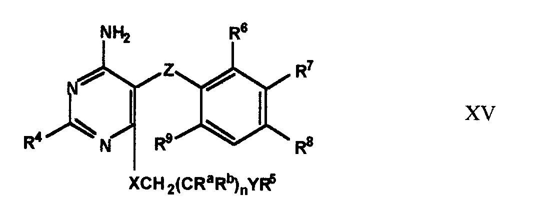 wherein R¹-R⁹, R^{a}, R^{b}, X, Y, Z and n have the significance given above, and, if desired, modifying substituents present in the compound of formula I obtained and/or converting the compound of formula I obtained into a salt.

## Claims (Claims for the following Contracting State(s): GR)

1. A process for the manufacture of compounds of the formula 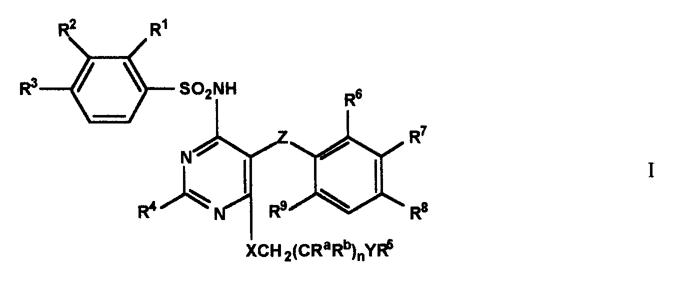 wherein
R¹ signifies hydrogen, C₁₋₇-alkyl, C₁₋₇-alkoxy, C₁₋₇-alkylthio, halogen or trifluoromethyl;
R² signifies hydrogen, halogen, C₁₋₇-alkoxy, trifluoromethyl or -OCH₂COOR^{a};
R³ signifies hydrogen, halogen, C₁₋₇-alkyl, C₁₋₇-alkylthio, trifluoromethyl, C₃₋₈-cycloalkyl, C₁₋₇-alkoxy or trifluoromethoxy;
R² and R³ together signify butadienyl, methylenedioxy, ethylenedioxy or isopropylidenedioxy;
R⁴ signifies hydrogen, C₁₋₇-alkyl, C₃₋₈-cycloalkyl, trifluoromethyl, C₁₋₇-alkoxy, C₁₋₇-alkylthio, C₁₋₇-alkylthio-C₁₋₇-alkyl, hydroxy-C₁₋₇-alkyl, hydroxy-C₁₋₇-alkoxy, C₁₋₇-alkoxy-C₁₋₇-alkyl, hydroxy-C₁₋₇-alkoxy- C₁₋₇-alkyl, hydroxy-C₁₋₇-alkoxy-C₁₋₇-alkoxy, C₁₋₇-alkylsulphinyl, C₁₋₇alkylsulphonyl, 2-methoxy-3-hydroxypropoxy, 2-hydroxy-3-phenylpropyl, amino-C₁₋₇-alkyl, C₁₋₇-alkylamino-C₁₋₇-alkyl, di-C₁₋₇-alkylamino-C₁₋₇-alkyl, amino, C₁₋₇-alkylamino, di-C₁₋₇-alkylamino, arylamino, aryl, arylthio, aryloxy, aryl-C₁₋₇-alkyl, wherein aryl represents unsubstituted phenyl or phenyl substituted with C₁₋₇-alkyl, trifluoromethyl, C₁₋₇-alkoxy, carboxyl or halogen, unsubstituted heterocyclyl from the group of 2-furyl, 3-furyl, pyrimidinyl, 2-pyridyl, 3-pyridyl, 4-pyridyl or 2, 3, or 4-pyridyl N-oxide, 1,2- or 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinyl, quinolyl, isoquinolyl, quinazolyl or heterocyclyl as hereinbefore defined substituted with C₁₋₇-alkyl, C₁₋₇-alkoxy, halogen, aryl or aryl-C₁₋₇-alkyl;
R⁵ signifies hydrogen, C₁₋₇-alkyl, C₁₋₇-alkanoyl, benzoyl, heterocyclylcarbonyl, heterocyclylmethyl, wherein heterocyclyl is selected from unsubstituted 2-furyl, 3-furyl, pyrimidinyl, 2-pyridyl, 3-pyridyl, 4-pyridyl, 2-, 3-, or 4-pyridyl N-oxide, 1,2- or 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinyl, quinolyl, isoquinolyl, quinazolyl or heterocyclyl as hereinbefore defined substituted with C₁₋₇-alkyl, C₁₋₇-alkoxy, halogen, phenyl or aryl-C₁₋₇-alkyl; or tetrahydropyran-2-yl;
R⁶ to R⁹ signify hydrogen, halogen, trifluoromethyl, C₁₋₇-alkyl, C₁₋₇-alkoxy, C₁₋₇-alkylthio, hydroxy, hydroxymethyl, cyano, carboxyl, formyl, methylsulphinyl, methylsulphonyl, methylsulphonyloxy or C₁₋₇-alkyloxy-carbonyloxy;
R⁷ together with R⁶ or R⁸ signify butadienyl, methylenedioxy, ethylenedioxy or isopropylidenedioxy;
Z signifies -O-, -S-, vinylene, -CO-, -OCHR¹⁰- or -SCHR¹⁰-;
R¹⁰ signifies hydrogen or C₁₋₇-alkyl;
X and Y each independently signify O, S or NH;
YR⁵ also signifies C₁₋₇-alkylsulphinyl;
R^{a}, R^{b}, R^{c} and R^{d} each independently signify hydrogen or C₁₋₇-alkyl; or
R^{c} and R^{d} together signify methylene, ethylene or isopropylidene; and
n signifies 1, 2, or 3,
and salts thereof, characterized by
a) reacting a compound of the formula 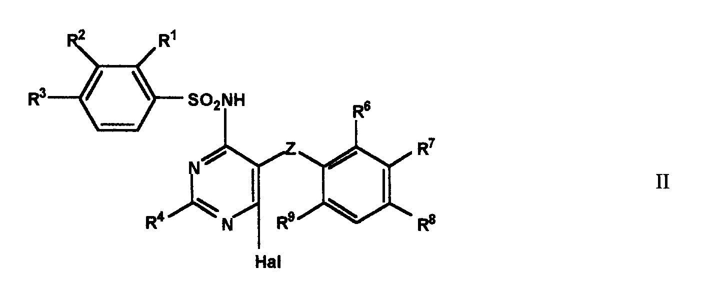 wherein R¹-R⁴ and R⁶-R⁹ have the significance given above and Hal is halogen, with a compound of the formula
MXCH₂(CR^{a}R^{b})ₙYR⁵ III
wherein X, Y, n, R^{a}, R^{b} and R⁵ have the significance given above and M represents
an alkali metal,
or
b) reacting a compound of the formula 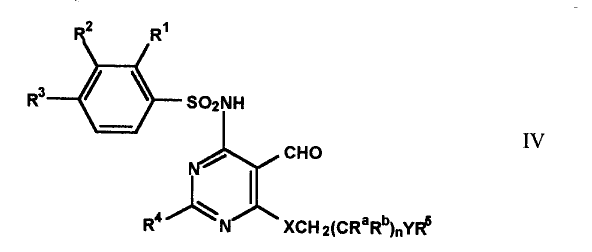 wherein R¹-R⁵, R^{a,} R^{b}, X, Y and n have the significance given above, with a compound of the formula 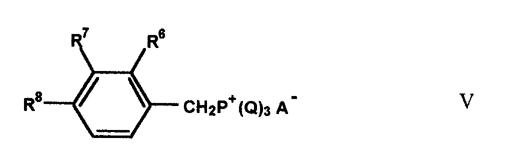 wherein R⁶-R⁸ have the significance given above; Q is aryl and A⁻ is an anion,
or
c) hydrogenating a compound of the formula 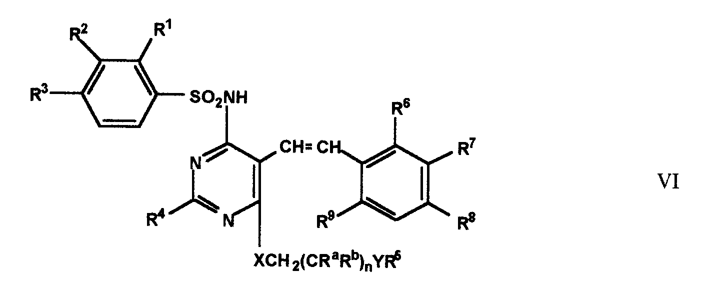 wherein R¹-R⁹, R^{a}, R^{b}, X, Y and n have the significance given above,
or
d) reacting a compound of the formula 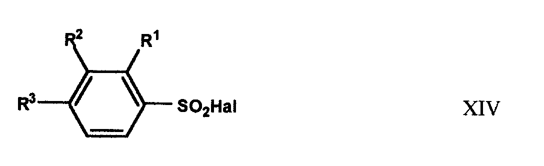 with a compound of the formula 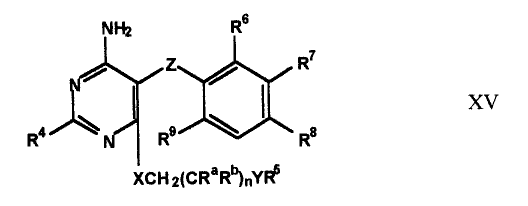 wherein R¹-R⁹, R^{a}, R^{b}, X, Y, Z and n have the significance given above, and, if desired, modifying substituents present in the compound of formula I obtained and/or converting the compound of formula I obtained into a salt.

2. A process according to claim 1 for the manufacture of compounds of formula I and salts thereof, wherein
R¹ signifies hydrogen, C₁₋₇-alkyl, C₁₋₇-alkoxy, C₁₋₇-alkylthio, halogen or trifluoromethyl;
R² signifies hydrogen, halogen, C₁₋₇-alkoxy or trifluoromethyl;
R³ signifies hydrogen, halogen, C₁₋₇-alkyl, C₁₋₇-alkylthio, trifluoromethyl, C₃₋₈cycloalkyl, C₁₋₇-alkoxy or trifluoromethoxy;
R² and R³ together signify butadienyl or methylenedioxy;
R⁴ signifies hydrogen, C₁₋₇-alkyl, trifluoromethyl, C₁₋₇-alkoxy, C₁₋₇-alkylthio, C₁₋₇-alkylthio-C₁₋₇-alkyl, hydroxy-C₁₋₇-alkyl, amino-C₁₋₇-alkyl, C₁₋₇-alkylamino-C₁₋₇-alkyl, di-C₁₋₇-alkylamino-C₁₋₇-alkyl, amino, C₁₋₇-alkylamino, di-C₁₋₇-alkylamino, arylamino, aryl, arylthio, aryloxy, aryl-C₁₋₇-alkyl, wherein aryl represents unsubstituted phenyl or phenyl substituted with C₁₋₇-alkyl, trifluoromethyl, C₁₋₇-alkoxy, carboxyl or halogen, unsubstituted heterocyclyl from the group of 2-furyl, 3-furyl, pyrimidinyl, 2-pyridyl, 3-pyridyl, 4-pyridyl, 2-, 3-, or 4-pyridyl N-oxide, 1,2- or 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinolyl, quinolyl, isoquinolyl, quinazolyl or tetrahydropyran-2-yl or heterocyclyl as hereinbefore defined substituted with C₁₋₇-alkyl, C₁₋₇-alkoxy, halogen, aryl or aryl-C₁₋₇-alkyl;
R⁵ signifies hydrogen, C₁₋₇-alkyl, C₁₋₇-alkanoyl, benzoyl or tetrahydropyran-2-yl;
R⁶ to R⁹ signify hydrogen, halogen, trifluoromethyl, C₁₋₇-alkyl, C₁₋₇-alkoxy, C₁₋₇-alkylthio, hydroxy, hydroxymethyl, cyano, carboxyl, formyl, methylsulphinyl, methylsulphonyl, methylsulphonyloxy or C₁₋₇-alkyloxy-carbonyloxy;
R⁷ together with R⁶ or R⁸ signify butadienyl or -OCH₂O-;
Z signifies -O-, -S-, vinylene, -CO-, -OCHR¹⁰- or -SCHR¹⁰-;
R¹⁰ signifies hydrogen or C₁₋₇-alkyl;
X and Y each independently signify O, S or NH;
R^{a} and R^{b} signify hydrogen and
n signifies 1, 2 or 3,
and salts thereof.

3. A process according to claim 1 or 2 for the manufacture of compounds of formula I in which Z is -O-.

4. A process according to claims 1-3 for the manufacture of compounds of formula I in which R⁶ signifies C₁₋₇-alkoxy and R⁷, R⁸ and R⁹ signify hydrogen.

5. A process according to claims 1-3 for the manufacture of compounds of formula I in which R⁶ and R⁸ signify hydrogen, R⁷ signifies C₁₋₇-alkoxy and R⁹ signifies halogen.

6. A process according to claims 1-3 for the manufacture of compounds of formula I in which R⁴ signifies hydrogen, 2-pyrimidinyl, 2- or 3-furyl, 2- or 3-thienyl, morpholino or p-methoxyphenyl.

7. A process according to claims 1-3 for the manufacture of compounds of formula I in which YR⁵ is hydroxy, C₁₋₇-alkoxysulphinyl or furoyloxy.

8. A process according to claim 3 for the manufacture of the compounds
p-t-butyl-N-[6-(2-hydroxyethoxy)-5-(o-methoxyphenoxy)-4-pyrimidinyl]benzenesulphonamide,
N-[6-(hydroxyethoxy)-5-(o-methoxyphenoxy)-4-pyrimidinyl]-p-isopropylbenzenesulphonamide,
p-t-butyl-N-[6-(2-hydroxyethoxy)-5-(o-tolyloxy)-4-pyrimidinyl]benzenesulphonamide,
p-t-butyl-N-[6-(2-hydroxyethoxy)-5-(o-chlorophenyloxy)-4-pyrimidinyl]benzenesulphonamide,
N-[6-(2-hydroxyethoxy)-5-(o-chlorophenoxy)-4-pyrimidinyl]-p-isopropylbenzenesulphonamide,
p-t-butyl-N-[6-(2-hydroxyethoxy)-5-(m-methoxyphenoxy)-4-pyrimidinyl]benzenesulphonamide,
p-t-butyl-N-[6-(2-hydroxyethoxy)-5-phenoxy-4-pyrimidinyl]benzenesulphonamide,
p-t-butyl-N-[6-(2-hydroxyethoxy)-5-(p-methoxyphenoxy)-4-pyrimidinyl]benzenesulphonamide,
p-t-butyl-N-[6-(2-hydroxyethoxy)-5-(o-ethoxyphenoxy)-4-pyrimidinyl]benzenesulphonamide,
p-(2,2-dimethylpropyl)-N-[6-(2-hydroxyethoxy)-5-(o-methoxyphenoxy)-4-pyrimidinyl]benzenesulphonamide,
p-isopropyl-N-[6-(2-hydroxyethoxy)-2-methyl-5-(m-methoxyphenoxy)-4-pyrimidinyl]benzenesulphonamide,
N-[6-(2-hydroxyethoxy)-5-(o-methoxyphenoxy)-2-phenyl-4-pyrimidinyl]-p-isopropylbenzenesulphonamide,
N-[6-(2-hydroxyethoxy)-5-(2,4,6-trichlorophenoxy)-4-pyrimidinyl]-p-isopropylbenzenesulphonamide,
N-[6-(2-hydroxyethoxy)-6-(2,4,6-trichlorophenoxy)-4-pyrimidinyl]-o-toluenesulphonamide,
N-[6-(2-hydroxyethoxy)-5-(2,4,6-trichlorophenoxy)-4-pyrimidinyl]-2,4-xylene-sulphonamide,
p-t-butyl-N-[6-(2-hydroxyethoxy)-5-[(2-methoxy-p-tolyl)oxy]-4-pyrimidinyl]benzenesulphonamide,
p-tert-butyl-N-[6-(2-hydroxyethoxy)-5-(o-methoxyphenoxy)-2-methyl-4-pyrimidinyl]benzenesulphonamide,
N-[6-(2-hydroxyethoxy)-5-(o-methoxyphenoxy)-2-methyl-4-pyrimidinyl)-p-isopropylbenzenesulphonamide,
N-[6-(2-hydroxyethoxy)-5-(o-methoxyphenoxy)-(2-trifluoromethyl)-4-pyrimidinyl)-p-isopropylbenzenesulphonamide,
p-tert-butyl-N-[6-(2-hydroxyethoxy)-5-(o-methoxyphenoxy)-2-(trifluoromethyl)-4-pyrimidinyl]benzenesulphonamide,
N-[5-(1,3-benzodioxol-5-yloxy)-6-(2-hydroxyethoxy)-4-pyrimidinyl]-p-tert-butylbenzenesulphonamide,
N-[5-(1,3-benzodioxol-5-yloxy)-6-(2-hydroxyethoxy)-4-pyrimidinyl]-p-isopropylbenzenesulphonamide,
N-[6-(2-hydroxyethoxy)-5-(o-methoxyphenoxy)-4-pyrimidinyl)-o-methoxybenzenesulphonamide,
p-tert-butyl-N-[6-(4-hydroxybutoxy)-5-(o-methoxyphenoxy)-2-methyl-4-pyrimidinyl]benzenesulphonamide,
N-[6-(2-hydroxyethoxy)-5-(2-naphthyloxy)-4-pyrimidinyl]-p-isopropylbenzenesulphonamide,
N-[6-(2-hydroxyethoxy)-5-(2-naphthyloxy)-4-pyrimidinyl]-p-tert-butylbenzenesulphonamide,
N-[6-(2-hydroxyethoxy)-5-(o-methoxyphenoxy)-2-propyl-4-pyrimidinyl]-p-isopropylbenzenesulphonamide,
p-tert-butyl-N-[6-(2-hydroxyethoxy)-5-(o-methoxyphenoxy)-2-propyl-4-pyrimidinyl]benzenesulphonamide,
α,α,α-trifluoro-N-[6-(2-hydroxyethoxy)-5-(o-methoxyphenoxy)-2-methyl-4-pyrimidinyl]-p-toluenesulphonamide,
p-chloro-N-[6-(2-hydroxyethoxy)-5-(o-methoxyphenoxy)-2-methyl-4-pyrimidinyl]benzenesulphonamide,
N-[6-(2-hydroxyethoxy)-5-(o-methoxyphenoxy)-2-methyl-4-pyrimidinyl]-p-(trifluoromethoxy)benzenesulphonamide,
o-ethyl-N-[6-(2-hydroxyethoxy)-5-(o-methoxyphenoxy)-2-methyl-4-pyrimidinyl]benzenesulphonamide,
N-[6-(2-hydroxyethoxy)-5-(o-methoxyphenoxy)-2-methyl-4-pyrimidinyl]-p-toluenesulphonamide,
N-[6-(2-hydroxyethoxy)-5-(o-methoxyphenoxy)-2-methyl-4-pyrimidinyl]-2-naphthylsulphonamide,
p-tert-butyl-N-[6-(3-hydroxypropoxy)-5-(o-methoxyphenoxy)-2-methyl-4-pyrimidinyl]benzenesulphonamide,
p-t-butyl-N-[6-(2-hydroxyethoxy)-5-[(o-methylthio)phenoxy]-4-pyrimidinyl]benzenesulphonamide,
p-t-butyl-N-[6-(2-hydroxyethoxy)-5-(o-methoxyphenoxy)-2-phenyl-4-pyrimidinyl]benzenesulphonamide,
N-[2-amino-6-(2-hydroxyethoxy)-5-(o-methoxyphenoxy)-4-pyrimidinyl]p-t-butylbenzenesulphonamide,
p-t-butyl-N-[6-(2-hydroxyethoxy)-2-methyl-5-[(o-methylthio)phenoxy)-4-pyrimidinyl]benzenesulphonamide and
p-t-butyl-N-[6-(2-hydroxyethoxy)-2-methyl-5-[o-(R,S-methylsulphinyl)phenoxy]-4-pyrimidinyl]benzenesulphonamide.

9. A process according to claim 3 for the manufacture of the compounds
4-tert-butyl-N-[5-(2-chloro-5-methoxy-phenoxy)-6-(2-hydroxy-ethoxy)-2-methyl-pyrimidin-4-yl]-benzenesulphonamide,
4-tert-butyl-N-[5-(2-chloro-5-methoxy-phenoxy)-6-(2-(3-furoyloxy)ethoxy)-2-methyl-pyrimidin-4-yl]-benzenesulphonamide,
4-tert-butyl-N-[6-(2-hydroxy-ethoxy)-5-(2-methoxy-phenoxy)-2-(thiophen-2-yl)-pyrimidin4-yl]-benzenesulphonamide,
4-tert-butyl-N-[6-(2-hydroxy-ethoxy)-5-(2-methoxy-phenoxy)-2-(thiophen-3-yl)-pyrimidin-4-yl]-benzenesulphonamide,
4-tert-butyl-N-[2-(furan-2-yl)-6-(2-hydroxy-ethoxy)-5-(2-methoxy-phenoxy)-pyrimidin-4-yl]-benzenesulphonamide,
4-tert-butyl-N-[2-furan-3-yl-6-(2-hydroxy-ethoxy)-5-(2-methoxy-phenoxy)-pyrimidin-4-yl]-benzenesulphonamide,
4-tert-butyl-N-[6-(2-hydroxy-ethoxy)-5-(2-methoxy-phenoxy)-2-(pyridin-2-yl)-pyrimidin-4-yl]-benzenesulphonamide,
4-tert-butyl-N-[6-(2-hydroxy-ethoxy)-5-(2-methoxy-phenoxy)-2-pyridin-4-yl)-pyrimidin-4-yl]-benzenesulphonamide,
4-tert-butyl-N-[6-(2-hydroxy-ethoxy)-5-(2-methoxy-phenoxy)-2-pyridin-3-yl)-pyrimidin-4-yl]-benzenesulphonamide,
2-[4-(4-tert-butyl-phenylsulphonylamino)-6-(2-hydroxy-ethoxy)-5-(2-methoxy-phenoxy)-pyrimidin-2-yl]-pyridine 1-oxide,
4-[4-(4-tert-butyl-phenylsulphonylamino)-6-(2-hydroxy-ethoxy)-5-(2-methoxy-phenoxy)-pyrimidin-2-yl]-pyridine 1-oxide,
4-tert-butyl-N-[6-(2-hydroxy-ethoxy)-2-[2-hydroxy-ethoxy)-ethyl]-5-(2-methoxy-phenoxy)-pyrimidin-4-yl]-benzenesulphonamide,
4-tert-butyl-N-[2-cyclopropyl-6-(2-hydroxy-ethoxy)-5-(2-methoxy-phenoxy)-pyrimidin-4-yl]-benzenesulphonamide,
4-tert-butyl-N-[2-ethyl-6-(2-hydroxy-ethoxy)-5-(2-methoxy-phenoxy)-pyrimidin-4-yl]-benzenesulphonamide,
4-tert-butyl-N-[6-(2-hydroxy-ethoxy)-2-isopropyl-5-(2-methoxy-phenoxy)-pyrimidin-4-yl]-benzenesulphonamide,
N-[5-(5-fluoro-2-methoxy-phenoxy)-6-(2-hydroxy-ethoxy)-pyrimidin-4-yl]-4-trifluoromethyl-benzenesulphonamide,
4-tert-butyl-N-[6-(2-hydroxy-ethoxy)-5-(3-methoxy-phenoxy)-2,2'-bipyrimidin-4-yl]-benzenesulphonamide,
4-tert-butyl-N-[5-(4-fluoro-2-methoxy-phenoxy)-6-(2-hydroxy-ethoxy)-2,2'-bipyrimidin-4-yl]-benzenesulphonamide,
4-tert-butyl-N-[5-(4-fluoro-2-methoxy-phenoxy)-6-(2-hydroxy-ethoxy)-2-methyl-pyrimidin-4-yl]-benzenesulphonamide,
4-tert-butyl-N-[5-(4-fluoro-2-methoxy-phenoxy)-6-(2-hydroxy-ethoxy)-pyrimidin-4-yl]-benzenesulphonamide,
N-[5-(5-fluoro-2-methoxy-phenoxy)-6-(2-hydroxy-ethoxy)-pyrimidin-4-yl]-4-isopropyl-benzenesulphonamide,
N-[5-(5-fluoro-2-methoxy-phenoxy)-6-(2-hydroxy-ethoxy)-pyrimidin-4-yl]-4-tert-butyl-benzenesulphonamide,
4-tert-butyl-N-[5-(2-fluoro-6-methoxy)-6-(2-hydroxy-ethoxy)-pyrimidin-4-yl]-benzenesulphonamide,
4-tert-butyl-N-[6-(2-hydroxy-ethoxy)-5-(3-methoxy-phenoxy)-2-(thiophen-2-yl)-pyrimidin-4-yl]-benzenesulphonamide,
4-tert-butyl-N-[6-(2-hydroxy-ethoxy)-2-(2-methoxy-ethyl)-5-(3-methoxy-phenoxy)-pyrimidin-4-yl]-benzenesulphonamide,
2-[6-(4-t-butylbenzenesulphonamino)-5-(3-methoxyphenoxy)pyrimidin-4-yloxy]ethyl 3-methylisoxazole-5-carboxylate,
2-[6-(4-t-butylbenzenesulphonamino)-5-(3-methoxyphenoxy)pyrimidin-4-yloxy]ethyl indole-2-carboxylate,
ethyl 5-[N-[6-(2-hydroxyethoxy)-5-(2-methoxyphenoxy)pyrimidin-4-yl]aminosulphonyl]-2-methoxy-phenoxyacetate.

10. A process according to claim 3 for the manufacture of (RS)-4-tert-butyl-N-[5-(2-chloro-5-methoxy-phenoxy)-2-ethyl-6-(2-methylsulphinyl-ethoxy)-pyrimidin-4-yl]-benzenesulphonamide.

11. A process according to claim 3 for the manufacture of (RS)-N-[5-(2-chloro-5-methoxy-phenoxy)-6-(2-methylsulphinyl-ethoxy)-pyrimidin-4-yl]-1,3-benzodioxol-5-sulphonamide.

12. A process according to claim 3 for the manufacture of 4-tert-butyl-N-[6-(2-hydroxy-ethoxy)-5-(2-methoxy-phenoxy)-2-(pyrimidin-2-yl)-pyrimidin-4-yl]-benzenesulphonamide.

13. A process according to claim 1 or 2 for the manufacture of compounds of formula I in which Z is vinylene.

14. A process according to claim 14 for the manufacture of the compounds
2-[[5-[(E/Z)-styryl]-6-p-toluenesulphonamido-4-pyrimidinyl]oxy]ethyl acetate,
N-[6-(2-hydroxyethoxy)-5-[(E/Z)-styryl]-4-pyrimidinyl]-p-toluenesulphonamide.

15. A process for the manufacture of pharmaceutical preparations, characterized by mixing a compound of formula I of claim 1 or a salt thereof with usual pharmaceutical adjuvants and carriers and bringing the mixture into a form which is suitable for therapeutic use.

16. The use of compounds of formula I of claim 1 and salts thereof as active substances in the production [of medicaments] for the treatment of disorders which are associated with endothelin activities, especially circulatory disorders such as hypertension, ischaemia, vasospasms and angina pectoris.

17. Compounds of the formula 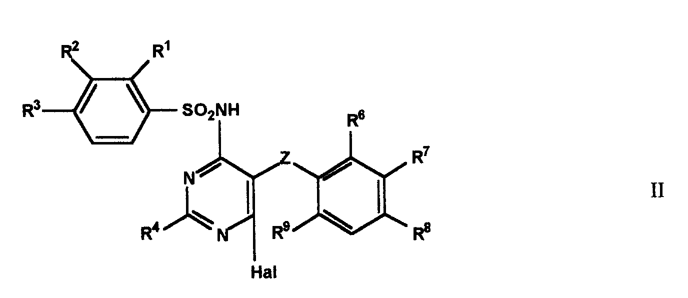 wherein R¹-R⁴, R⁶-R⁹, Y and n have the significance given in claim 1 and Hal is halogen.

## Claims (Claims for the following Contracting State(s): ES)

1. A process for the manufacture of compounds of the formula 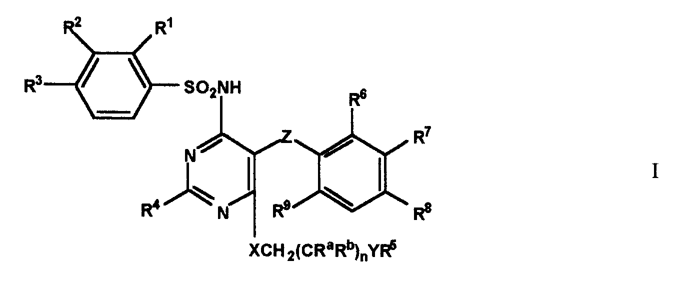 wherein
R¹ signifies hydrogen, C₁₋₇-alkyl, C₁₋₇-alkoxy, C₁₋₇-alkylthio, halogen or trifluoromethyl;
R² signifies hydrogen, halogen, C₁₋₇alkoxy, trifluoromethyl or -OCH₂COOR^{a};
R³ signifies hydrogen, halogen, C₁₋₇-alkyl, C₁₋₇-alkylthio, trifluoromethyl, C₃₋₈-cycloalkyl, C₁₋₇-alkoxy or trifluoromethoxy;
R² and R³ together signify butadienyl, methylenedioxy, ethylenedioxy or isopropylidenedioxy;
R⁴ signifies hydrogen, C₁₋₇-alkyl, C₃₋₈-cycloalkyl, trifluoromethyl, C₁₋₇alkoxy, C₁₋₇-alkylthio, C₁₋₇alkylthio-C₁₋₇-alkyl, hydroxy-C₁₋₇-alkyl, hydroxy-C₁₋₇-alkoxy, C₁₋₇-alkoxy-C₁₋₇-alkyl, hydroxy-C₁₋₇-alkoxy- C₁₋₇-alkyl, hydroxy-C₁₋₇-alkoxy-C₁₋₇alkoxy, C₁₋₇-alkylsulphinyl, C₁₋₇alkylsulphonyl, 2-methoxy-3-hydroxypropoxy, 2-hydroxy-3-phenylpropyl, amino-C₁₋₇-alkyl, C₁₋₇alkylamino-C₁₋₇-alkyl, di-C₁₋₇-alkylamino-C₁₋₇-alkyl, amino, C₁₋₇-alkylamino, di-C₁₋₇-alkylamino, arylamino, aryl, arylthio, aryloxy, aryl-C₁₋₇-alkyl, wherein aryl represents unsubstituted phenyl or phenyl substituted with C₁₋₇-alkyl, trifluoromethyl, C₁₋₇-alkoxy, carboxyl or halogen, unsubstituted heterocyclyl from the group of 2-furyl, 3-furyl, pyrimidinyl, 2-pyridyl, 3-pyridyl, 4-pyridyl or 2, 3, or 4-pyridyl N-oxide, 1,2- or 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinyl, quinolyl, isoquinolyl, quinazolyl or heterocyclyl as hereinbefore defined substituted with C₁₋₇-alkyl, C₁₋₇-alkoxy, halogen, aryl or aryl-C₁₋₇-alkyl;
R⁵ signifies hydrogen, C₁₋₇-alkyl, C₁₋₇-alkanoyl, benzoyl, heterocyclylcarbonyl, heterocyclylmethyl, wherein heterocyclyl is selected from unsubstituted 2-furyl, 3-furyl, pyrimidinyl, 2-pyridyl, 3-pyridyl, 4-pyridyl, 2-, 3-, or 4-pyridyl N-oxide, 1,2- or 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinyl, quinolyl, isoquinolyl, quinazolyl or heterocyclyl as hereinbefore defined substituted with C₁₋₇-alkyl, C₁₋₇-alkoxy, halogen, phenyl or aryl-C₁₋₇-alkyl; or tetrahydropyran-2-yl;
R⁶ to R⁹ signify hydrogen, halogen, trifluoromethyl, C₁₋₇-alkyl, C₁₋₇-alkoxy, C₁₋₇-alkylthio, hydroxy, hydroxymethyl, cyano, carboxyl, formyl, methylsulphinyl, methylsulphonyl, methylsulphonyloxy or C₁₋₇-alkyloxy-carbonyloxy;
R⁷ together with R⁶ or R⁸ signify butadienyl, methylenedioxy, ethylenedioxy or isopropylidenedioxy;
Z signifies -O-, -S-, vinylene, -CO-, -OCHR¹⁰- or -SCHR¹⁰-;
R¹⁰ signifies hydrogen or C₁₋₇-alkyl;
X and Y each independently signify O, S or NH;
YR⁵ also signifies C₁₋₇-alkylsulphinyl;
R^{a}, R^{b}, R^{c} and R^{d} each independently signify hydrogen or C₁₋₇-alkyl; or
R^{c} and R^{d} together signify methylene, ethylene or isopropylidene; and
n signifies 1, 2 or 3,
and salts thereof, characterized by
a) reacting a compound of the formula 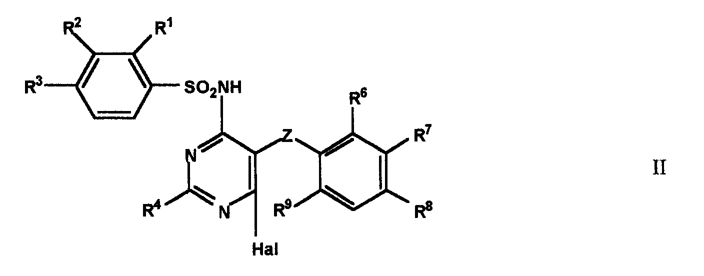 wherein R¹-R⁴ and R⁶-R⁹ have the significance given above and Hal is halogen, with a compound of the formula
MXCH₂(CR^{a}R^{b})ₙYR⁵ III
wherein X, Y, n, R^{a}, R^{b} and R⁵ have the significance given above and M represents an alkali metal,
or
b) reacting a compound of the formula 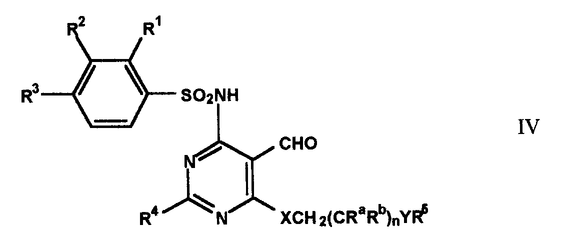 wherein R¹-R⁵, R^{a}, R^{b}, X, Y and n have the significance given above, with a compound of the formula 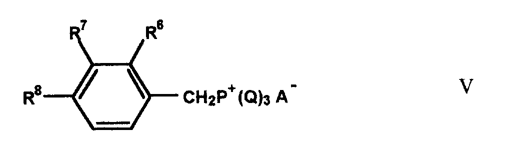 wherein R⁶-R⁸ have the significance given above; Q is aryl and A⁻ is an anion,
or
c) hydrogenating a compound of the formula 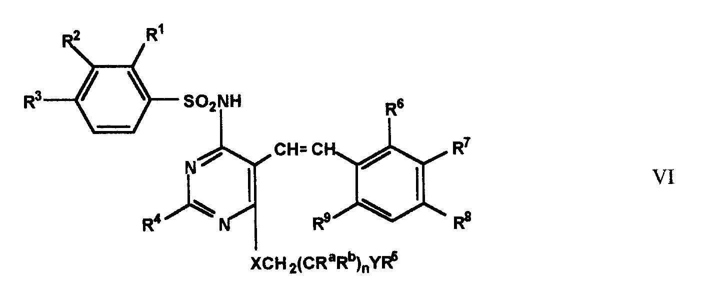 wherein R¹-R⁹, R^{a}, R^{b}, X, Y and n have the significance given above,
or
d) reacting a compound of the formula 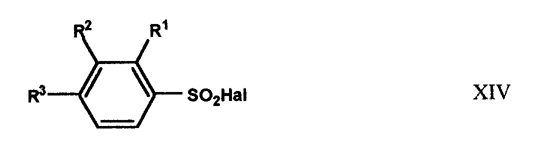 with a compound of the formula 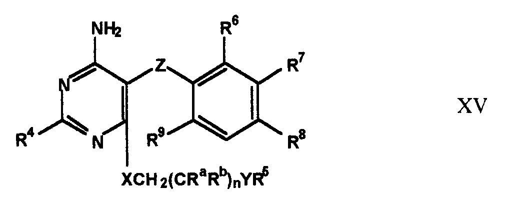 wherein R¹-R⁹, R^{a}, R^{b}, X, Y, Z and n have the significance given above, and, if desired, modifying substituents present in the compound of formula I obtained and/or converting the compound of formula I obtained into a salt.

2. A process according to claim 1 for the manufacture of compounds of formula I and salts thereof, wherein
R¹ signifies hydrogen, C₁₋₇-alkyl, C₁₋₇-alkoxy, C₁₋₇-alkylthio, halogen or trifluoromethyl;
R² signifies hydrogen, halogen, C₁₋₇-alkoxy or trifluoromethyl;
R³ signifies hydrogen, halogen, C₁₋₇-alkyl, C₁₋₇-alkylthio, trifluoromethyl, C₃₋₈-cycloalkyl, C₁₋₇-alkoxy or trifluoromethoxy;
R² and R³ together signify butadienyl or methylenedioxy;
R⁴ signifies hydrogen, C₁₋₇-alkyl, trifluoromethyl, C₁₋₇-alkoxy, C₁₋₇-alkylthio, C₁₋₇-alkylthio-C₁₋₇-alkyl, hydroxy-C₁₋₇-alkyl, amino-C₁₋₇-alkyl, C₁₋₇-alkylamino-C₁₋₇-alkyl, di-C₁₋₇-alkylamino-C₁₋₇-alkyl, amino, C₁₋₇-alkylamino, di-C₁₋₇-alkylamino, arylamino, aryl, arylthio, aryloxy, aryl-C₁₋₇-alkyl, wherein aryl represents unsubstituted phenyl or phenyl substituted with C₁₋₇-alkyl, trifluoromethyl, C₁₋₇-alkoxy, carboxyl or halogen, unsubstituted heterocyclyl from the group of 2-furyl, 3-furyl, pyrimidinyl, 2-pyridyl, 3-pyridyl, 4-pyridyl, 2-, 3-, or 4-pyridyl N-oxide, 1,2- or 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinolyl, quinolyl, isoquinolyl, quinazolyl or tetrahydropyran-2-yl or heterocyclyl as hereinbefore defined substituted with C₁₋₇-alkyl, C₁₋₇-alkoxy, halogen, aryl or aryl-C₁₋₇-alkyl;
R⁵ signifies hydrogen, C₁₋₇-alkyl, C₁₋₇-alkanoyl, benzoyl or tetrahydropyran-2-yl;
R⁶ to R⁹ signify hydrogen, halogen, trifluoromethyl, C₁₋₇-alkyl, C₁₋₇-alkoxy, C₁₋₇-alkylthio, hydroxy, hydroxymethyl, cyano, carboxyl, formyl, methylsulphinyl, methylsulphonyl, methylsulphonyloxy or C₁₋₇-alkyloxy-carbonyloxy;
R⁷ together with R⁶ or R⁸ signify butadienyl or -OCH₂O-;
Z signifies -O-, -S-, vinylene, -CO-, -OCHR¹⁰- or -SCHR¹⁰-;
R¹⁰ signifies hydrogen or C₁₋₇-alkyl;
X and Y each independently signify O, S or NH;
R^{a} and R^{b} signify hydrogen and
n signifies 1, 2 or 3,
and salts thereof.

3. A process according to claim 1 or 2 for the manufacture of compounds of formula I in which Z is -O-.

4. A process according to claims 1-3 for the manufacture of compounds of formula I in which R⁶ signifies C₁₋₇-alkoxy and R⁷, R⁸ and R⁹ signify hydrogen.

5. A process according to claims 1-3 for the manufacture of compounds of formula I in which R⁶ and R⁸ signify hydrogen, R⁷ signifies C₁₋₇-alkoxy and R⁹ signifies halogen.

6. A process according to claims 1-3 for the manufacture of compounds of formula I in which R⁴ signifies hydrogen, 2-pyrimidinyl, 2- or 3-furyl, 2- or 3-thienyl, morpholino or p-methoxyphenyl.

7. A process according to claims 1-3 for the manufacture of compounds of formula I in which YR⁵ is hydroxy, C₁₋₇-alkoxysulphinyl or furoyloxy.

8. A process according to claim 3 for the manufacture of the compounds
p-t-butyl-N-[6-(2-hydroxyethoxy)-5-(o-methoxyphenoxy)-4-pyrimidinyl]benzenesulphonamide,
N-[6-(hydroxyethoxy)-5-(o-methoxyphenoxy)-4-pyrimidinyl]-p-isopropylbenzenesulphonamide,
p-t-butyl-N-[6-(2-hydroxyethoxy)-5-(o-tolyloxy)-4-pyrimidinyl]benzenesulphonamide,
p-t-butyl-N-[6-(2-hydroxyethoxy)-5-(o-chlorophenyloxy)-4-pyrimidinyl]benzenesulphonamide,
N-[6-(2-hydroxyethoxy)-5-(o-chlorophenoxy)-4-pyrimidinyl]-p-isopropylbenzenesulphonamide,
p-t-butyl-N-[6-(2-hydroxyethoxy)-5-(m-methoxyphenoxy)-4-pyrimidinyl]benzenesulphonamide,
p-t-butyl-N-[6-(2-hydroxyethoxy)-5-phenoxy-4-pyrimidinyl]benzenesulphonamide,
p-t-butyl-N-[6-(2-hydroxyethoxy)-5-(p-methoxyphenoxy)-4-pyrimidinyl]benzenesulphonamide,
p-t-butyl-N-[6-(2-hydroxyethoxy)-5-(o-ethoxyphenoxy)-4-pyrimidinyl]benzenesulphonamide,
p-(2,2-dimethylpropyl)-N-[6-(2-hydroxyethoxy)-5-(o-methoxyphenoxy)-4-pyrimidinyl]-benzenesulphonamide,
p-isopropyl-N-[6-(2-hydroxyethoxy)-2-methyl-5-(m-methoxyphenoxy)-4-pyrimidinyl]-benzenesulphonamide,
N-[6-(2-hydroxyethoxy)-5-(o-methoxyphenoxy)-2-phenyl-4-pyrimidinyl]-p-isopropylbenzenesulphonamide,
N-[6-(2-hydroxyethoxy)-5-(2,4,6-trichlorophenoxy)-4-pyrimidinyl]-p-isopropylbenzenesulphonamide,
N-[6-(2-hydroxyethoxy)-6-(2,4,6-trichiorophenoxy)-4-pyrimidinyl]-o-toluenesulphonamide,
N-[6-(2-hydroxyethoxy)-5-(2,4,6-trichlorophenoxy)-4-pyrimidinyl]-2,4-xylene-sulphonamide,
p-t-butyl-N-[6-(2-hydroxyethoxy)-5-[(2-methoxy-p-tolyl)oxy]-4-pyrimidinyl]benzenesulphonamide,
p-tert-butyl-N-[6-(2-hydroxyethoxy)-5-(o-methoxyphenoxy)-2-methyl-4-pyrimidinyl]-benzenesulphonamide,
N-[6-(2-hydroxyethoxy)-5-(o-methoxyphenoxy)-2-methyl-4-pyrimidinyl)-p-isopropylbenzenesulphonamide,
N-[6-(2-hydroxyethoxy)-5-(o-methoxyphenoxy)-(2-trifluoromethyl)-4-pyrimidinyl)-p-isopropylbenzenesulphonamide,
p-tert-butyl-N-[6-(2-hydroxyethoxy)-5-(o-methoxyphenoxy)-2-(trifluoromethyl)-4-pyrimidinyl]benzenesulphonamide,
N-[5-(1,3-benzodioxol-5-yloxy)-6-(2-hydroxyethoxy)-4-pyrimidinyl]-p-tert-butylbenzenesulphonamide,
N-[5-(1,3-benzodioxol-5-yloxy)-6-(2-hydroxyethoxy)-4-pyrimidinyl]-p-isopropylbenzenesulphonamide,
N-[6-(2-hydroxyethoxy)-5-(o-methoxyphenoxy)-4-pyrimidinyl)-o-methoxybenzenesulphonamide,
p-tert-butyl-N-[6-(4-hydroxybutoxy)-5-(o-methoxyphenoxy)-2-methyl-4-pyrimidinyl]-benzenesulphonamide,
N-[6-(2-hydroxyethoxy)-5-(2-naphthyloxy)-4-pyrimidinyl]-p-isopropylbenzenesulphonamide,
N-[6-(2-hydroxyethoxy)-5-(2-naphthyloxy)-4-pyrimidinyl]-p-tert-butylbenzenesulphonamide,
N-[6-(2-hydroxyethoxy)-5-(o-methoxyphenoxy)-2-propyl-4-pyrimidinyl]-p-isopropylbenzenesulphonamide,
p-tert-butyl-N-[6-(2-hydroxyethoxy)-5-(o-methoxyphenoxy)-2-propyl-4-pyrimidinyl]-benzenesulphonamide,
α,α,α-trifluoro-N-[6-(2-hydroxyethoxy)-5-(o-methoxyphenoxy)-2-methyl-4-pyrimidinyl]-p-toluenesulphonamide,
p-chloro-N-[6-(2-hydroxyethoxy)-5-(o-methoxyphenoxy)-2-methyl-4-pyrimidinyl]benzenesulphonamide,
N-[6-(2-hydroxyethoxy)-5-(o-methoxyphenoxy)-2-methyl-4-pyrimidinyl]-p-(trifluoromethoxy)benzenesulphonamide,
o-ethyl-N-[6-(2-hydroxyethoxy)-5-(o-methoxyphenoxy)-2-methyl-4-pyrimidinyl]benzenesulphonamide,
N-[6-(2-hydroxyethoxy)-5-(o-methoxyphenoxy)-2-methyl-4-pyrimidinyl]-p-toluenesulphonamide,
N-[6-(2-hydroxyethoxy)-5-(o-methoxyphenoxy)-2-methyl-4-pyrimidinyl]-2-naphthylsulphonamide,
p-tert-butyl-N-[6-(3-hydroxypropoxy)-5-(o-methoxyphenoxy)-2-methyl-4-pyrimidinyl]-benzenesulphonamide,
p-t-butyl-N-[6-(2-hydroxyethoxy)-5-[(o-methylthio)phenoxy]-4-pyrimidinyl]benzenesulphonamide,
p-t-butyl-N-[6-(2-hydroxyethoxy)-5-(o-methoxyphenoxy)-2-phenyl-4-pyrimidinyl]benzenesulphonamide,
N-[2-amino-6-(2-hydroxyethoxy)-5-(o-methoxyphenoxy)-4-pyrimidinyl]p-t-butylbenzenesulphonamide,
p-t-butyl-N-[6-(2-hydroxyethoxy)-2-methyl-5-[(o-methylthio)phenoxy)-4-pyrimidinyl]-benzenesulphonamide and
p-t-butyl-N-[6-(2-hydroxyethoxy)-2-methyl-5-[o-(R,S-methylsulphinyl)phenoxy]-4-pyrimidinyl]benzenesulphonamide.

9. A process according to claim 3 for the manufacture of the compounds
4-tert-butyl-N-[5-(2-chloro-5-methoxy-phenoxy)-6-(2-hydroxy-ethoxy)-2-methyl-pyrimidin-4-yl]-benzenesulphonamide,
4-tert-butyl-N-[5-(2-chloro-5-methoxy-phenoxy)-6-(2-(3-furoyloxy)ethoxy)-2-methyl-pyrimidin-4-yl]-benzenesulphonamide,
4-tert-butyl-N-[6-(2-hydroxy-ethoxy)-5-(2-methoxy-phenoxy)-2-(thiophen-2-yl)-pyrimidin-4-yl]-benzenesulphonamide,
4-tert-butyl-N-[6-(2-hydroxy-ethoxy)-5-(2-methoxy-phenoxy)-2-(thiophen-3-yl)-pyrimidin-4-yl]-benzenesulphonamide,
4-tert-butyl-N-[2-(furan-2-yl)-6-(2-hydroxy-ethoxy)-5-(2-methoxy-phenoxy)-pyrimidin-4-yl]-benzenesulphonamide,
4-tert-butyl-N-[2-furan-3-yl-6-(2-hydroxy-ethoxy)-5-(2-methoxy-phenoxy)-pyrimidin-4-yl]-benzenesulphonamide,
4-tert-butyl-N-[6-(2-hydroxy-ethoxy)-5-(2-methoxy-phenoxy)-2-(pyridin-2-yl)-pyrimidin-4-yl]-benzenesulphonamide,
4-tert-butyl-N-[6-(2-hydroxy-ethoxy)-5-(2-methoxy-phenoxy)-2-pyridin-4-yl)-pyrimidin-4-yl]-benzenesulphonamide,
4-tert-butyl-N-[6-(2-hydroxy-ethoxy)-5-(2-methoxy-phenoxy)-2-pyridin-3-yl)-pyrimidin-4-yl]-benzenesulphonamide,
2-[4-(4-tert-butyl-phenylsulphonylamino)-6-(2-hydroxy-ethoxy)-5-(2-methoxy-phenoxy)-pyrimidin-2-yl]-pyridine 1-oxide,
4-[4-(4-tert-butyl-phenylsulphonylamino)-6-(2-hydroxy-ethoxy)-5-(2-methoxy-phenoxy)-pyrimidin-2-yl]-pyridine 1-oxide,
4-tert-butyl-N-[6-(2-hydroxy-ethoxy)-2-[2-hydroxy-ethoxy)-ethyl]-5-(2-methoxy-phenoxy)-pyrimidin-4-yl]-benzenesulphonamide,
4-tert-butyl-N-[2-cyclopropyl-6-(2-hydroxy-ethoxy)-5-(2-methoxy-phenoxy)-pyrimidin-4-yl]-benzenesulphonamide,
4-tert-butyl-N-[2-ethyl-6-(2-hydroxy-ethoxy)-5-(2-methoxy-phenoxy)-pyrimidin-4-yl]-benzenesulphonamide,
4-tert-butyl-N-[6-(2-hydroxy-ethoxy)-2-isopropyl-5-(2-methoxy-phenoxy)-pyrimidin-4-yl]-benzenesulphonamide,
N-[5-(5-fluoro-2-methoxy-phenoxy)-6-(2-hydroxy-ethoxy)-pyrimidin-4-yl]-4-trifluoromethyl-benzenesulphonamide,
4-tert-butyl-N-[6-(2-hydroxy-ethoxy)-5-(3-methoxy-phenoxy)-2,2'-bipyrimidin-4-yl]-benzenesulphonamide,
4-tert-butyl-N-[5-(4-fluoro-2-methoxy-phenoxy)-6-(2-hydroxy-ethoxy)-2,2'-bipyrimidin-4-yl]-benzenesulphonamide,
4-tert-butyl-N-[5-(4-fluoro-2-methoxy-phenoxy)-6-(2-hydroxy-ethoxy)-2-methyl-pyrimidin-4-yl]-benzenesulphonamide,
4-tert-butyl-N-[5-(4-fluoro-2-methoxy-phenoxy)-6-(2-hydroxy-ethoxy)-pyrimidin-4-yl]-benzenesulphonamide,
N-[5-(5-fluoro-2-methoxy-phenoxy)-6-(2-hydroxy-ethoxy)-pyrimidin-4-yl]-4-isopropylbenzenesulphonamide,
N-[5-(5-fluoro-2-methoxy-phenoxy)-6-(2-hydroxy-ethoxy)-pyrimidin-4-yl]-4-tert-butyl-benzenesulphonamide,
4-tert-butyl-N-[5-(2-fluoro-6-methoxy)-6-(2-hydroxy-ethoxy)-pyrimidin-4-yl]-benzenesulphonamide,
4-tert-butyl-N-[6-(2-hydroxy-ethoxy)-5-(3-methoxy-phenoxy)-2-(thiophen-2-yl)-pyrimidin-4-yl]-benzenesulphonamide,
4-tert-butyl-N-[6-(2-hydroxy-ethoxy)-2-(2-methoxy-ethyl)-5-(3-methoxy-phenoxy)-pyrimidin-4-yl]-benzenesulphonamide,
2-[6-(4-t-butylbenzenesulphonamino)-5-(3-methoxyphenoxy)pyrimidin-4-yloxy]ethyl 3-methylisoxazole-5-carboxylate,
2-[6-(4-t-butylbenzenesulphonamino)-5-(3-methoxyphenoxy)pyrimidin-4-yloxy]ethyl indole-2-carboxylate,
ethyl 5-[N-[6-(2-hydroxyethoxy)-5-(2-methoxyphenoxy)pyrimidin-4-yl]aminosulphonyl]-2-methoxy-phenoxyacetate.

10. A process according to claim 3 for the manufacture of (RS)-4-tert-butyl-N-[5-(2-chloro-5-methoxy-phenoxy)-2-ethyl-6-(2-methylsulphinyl-ethoxy)-pyrimidin-4-yl]-benzenesulphonamide.

11. A process according to claim 3 for the manufacture of (RS)-N-[5-(2-chloro-5-methoxy-phenoxy)-6-(2-methylsulphinyl-ethoxy)-pyrimidin-4-yl]-1,3-benzodioxol-5-sulphonamide.

12. A process according to claim 3 for the manufacture of 4-tert-butyl-N-[6-(2-hydroxy-ethoxy)-5-(2-methoxy-phenoxy)-2-(pyrimidin-2-yl)-pyrimidin-4-yl]-benzenesulphonamide.

13. A process according to claim 1 or 2 for the manufacture of compounds of formula I in which Z is vinylene.

14. A process according to claim 14 for the manufacture of the compounds
2-[[5-[(E/Z)-styryl]-6-p-toluenesulphonamido-4-pyrimidinyl]oxy]ethyl acetate,
N-[6-(2-hydroxyethoxy)-5-[(E/Z)-styryl]-4-pyrimidinyl]-p-toluenesulphonamide.

## Revendications (Revendications pour l'(les) Etat(s) contractant(s) suivant(s): AT, BE, CH, DE, DK, FR, GB, IT, LI, LU, MC, NL, PT, SE)

1. Composés de formule 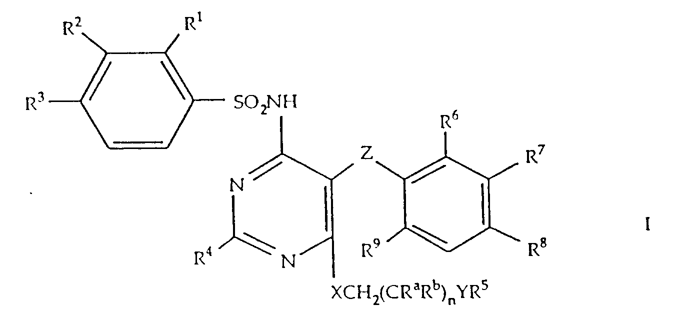 dans laquelle
R¹ représente un atome d'hydrogène ou d'halogène ou un groupe alkyle en C₁₋₇, alcoxy en C₁₋₇, alkyl(C₁₋₇)-thio ou trifluorométhyle;
R² représente un atome d'hydrogène ou d'halogène ou un groupe alcoxy en C₁₋₇, trifluorométhyle ou -OCH₂COOR^{a};
R³ représente un atome d'hydrogène ou d'halogène ou un groupe alkyle en C₁₋₇, alkyl(C₁₋₇)thio, trifluorométhyle, cycloalkyle en C₃₋₈, alcoxy en C₁₋₇ ou trifluorométhoxy;
R² et R³ peuvent former ensemble un groupe butadiényle, méthylènedioxy, éthylènedioxy ou isopropylidènedioxy;
R⁴ représente un atome d'hydrogène ou un groupe alkyle en C₁₋₇, cycloalkyle en C₃₋₈, trifluorométhyle, alcoxy en C₁₋₇, alkyl(C₁₋₇)thio, alkyl(C₁₋₇)thio-alkyle(C₁₋₇), hydroxyalkyle(C₁₋₇), hydroxy-alcoxy(C₁₋₇), alcoxy(C₁₋₇)-alkyle(C₁₋₇), hydroxy-alcoxy(C₁₋₇)-alkyle(C₁₋₇), hydroxy-alcoxy(C₁₋₇)-alcoxy(C₁₋₇), alkyl(C₁₋₇)sulfinyle, alkyl(C₁₋₇)sulfonyle, 2-méthoxy-3-hydroxypropoxy, 2-hydroxy-3-phénylpropyle, aminoalkyle(C₁₋₇), alkyl(C₁₋₇)amino-alkyle(C₁₋₇), dialkyl(C₁₋₇)amino-alkyle(C₁₋₇), amino, alkyl(C₁₋₇)amino, dialkyl(C₁₋₇)amino, arylamino, aryle, arylthio, aryloxy, aryl-alkyle(C₁₋₇), le fragment aryle représentant le groupe phényle non substitué ou un groupe phényle substitué par un groupe alkyle en C₁₋₇, trifluorométhyle, alcoxy en C₁₋₇, carboxyle ou par un atome d'halogène, un groupe hétérocyclique non substitué choisi dans l'ensemble constitué par les groupes 2-furyle, 3-furyle, pyrimidinyle, 2-pyridyle, 3-pyridyle, 4-pyridyle ou N-oxyde de 2-, 3- ou 4-pyridyle, 1,2- ou 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinyle, quinolyle, isoquinolyle, quinazolyle, ou un groupe hétérocyclique tel que défini précédemment, substitué par un atome d'halogène ou par un groupe alkyle en C₁₋₇, alcoxy en C₁₋₇, aryle ou aryl-alkyle(C₁₋₇);
R⁵ représente un atome d'hydrogène ou un groupe alkyle en C₁₋₇, alcanoyle en C₁₋₇, benzoyle, hétérocyclylcarbonyle, hétérocyclylméthyle, le fragment hétérocyclique étant choisi parmi les groupes 2-furyle, 3-furyle, pyrimidinyle, 2-pyridyle, 3-pyridyle, 4-pyridyle, N-oxyde de 2-, 3- ou 4-pyridyle, 1,2- ou 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinyle, quinolyle, isoquinolyle, quinazolyle ou étant un groupe hétérocyclique tel que défini précédemment, substitué par un atome d'halogène ou par un groupe alkyle en C₁₋₇, alcoxy en C₁₋₇, phényle ou arylalkyle(C₁₋₇); ou représente le groupe tétrahydropyrann-2-yle;
R⁶ à R⁹ représentent un atome d'hydrogène ou d'halogène ou un groupe trifluorométhyle, alkyle en C₁₋₇, alcoxy en C₁₋₇, alkyl(C₁₋₇)thio, hydroxyle, hydroxyméthyle, cyano, carboxyle, formyle, méthylsulfinyle, méthylsulfonyle, méthylsulfonyloxy ou alkyloxy(C₁₋₇)carbonyloxy;
R⁷ forme, conjointement avec R⁶ ou R⁸, un groupe butadiényle, méthylènedioxy, éthylènedioxy ou isopropylidènedioxy;
Z représente -O-, -S-, le groupe vinylène, -CO-, -OCHR¹⁰ ou -SCHR¹⁰;
R¹⁰ représente un atome d'hydrogène ou un groupe alkyle en C₁₋₇;
X et Y représentent, indépendamment l'un de l'autre, O, S ou NH;
YR⁵ peut également représenter un groupe alkyl(C₁₋₇)sulfinyle;
R^{a}, R^{b}, R^{c} et R^{d} représentent, indépendamment les uns des autres, un atome d'hydrogène ou un groupe alkyle en C₁₋₇; ou
R^{c} et R^{d} forment ensemble un groupe méthylène, éthylène ou isopropylidène; et
n est égal à 1, 2 ou 3,
et leurs sels.

2. Composés de formule I selon la revendication 1, dans lesquels
R¹ représente un atome d'hydrogène ou d'halogène ou un groupe alkyle en C₁₋₇, alcoxy en C₁₋₇, alkyl(C₁₋₇)thio ou trifluorométhyle;
R² représente un atome d'hydrogène ou d'halogène ou un groupe alcoxy en C₁₋₇ ou trifluorométhyle;
R³ représente un atome d'hydrogène ou d'halogène ou un groupe alkyle en C₁₋₇, alkyl(C₁₋₇)thio, trifluorométhyle, cycloalkyle en C₃₋₈, alcoxy en C₁₋₇ ou trifluorométhoxy;
R² et R³ forment ensemble un groupe butadiényle ou méthylènedioxy;
R⁴ représente un atome d'hydrogène ou un groupe alkyle en C₁₋₇, trifluorométhyle, alcoxy en C₁₋₇, alkyl(C₁₋₇)thio, alkyl(C₁₋₇)thio-alkyle(C₁₋₇), hydroxyalkyle(C₁₋₇), amino-alkyle(C₁₋₇), alkyl(C₁₋₇)amino-alkyle(C₁₋₇), dialkyl(C₁₋₇)amino-alkyle(C₁₋₇), amino, alkyl(C₁₋₇)amino, dialkyl(C₁₋₇)amino, arylamino, aryle, arylthio, aryloxy, aryl-alkyle(C₁₋₇), le fragment aryle étant le groupe phényle non substitué ou un groupe phényle substitué par un atome d'halogène ou par un groupe alkyle en C₁₋₇, trifluorométhyle, alcoxy en C₁₋₇, carboxyle, un groupe hétérocyclique non substitué choisi dans l'ensemble constitué par les groupes 2-furyle, 3-furyle, pyrimidinyle, 2-pyridyle, 3-pyridyle, 4-pyridyle, N-oxyde de 2-, 3- ou 4-pyridyle, 1,2- ou 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinolyle, quinolyle, isoquinolyle, quinazolyle et tétrahydropyrann-2-yle, ou un groupe hétérocyclique tel que défini précédemment, substitué par un atome d'halogène ou par un groupe alkyle en C₁₋₇, alcoxy en C₁₋₇, aryle ou aryl-alkyle(C₁₋₇);
R⁵ représente un atome d'hydrogène ou un groupe alcanoyle en C₁₋₇, benzoyle ou tétrahydropyrann-2-yle;
R⁶ à R⁹ représentent un atome d'hydrogène ou d'halogène ou un groupe trifluorométhyle, alkyle en C₁₋₇, alcoxy en C₁₋₇, alkyl(C₁₋₇)thio, hydroxyle, hydroxyméthyle, cyano, carboxyle, formyle, méthylsulfinyle, méthylsulfonyle, méthylsulfonyloxy ou alkyloxy(C₁₋₇)carbonyloxy;
R⁷ peut former, conjointement avec R⁶ ou R⁸, le groupe butadiényle ou -OCH₂O-;
Z représente -O-, -S-, un groupe vinylène, -CO-, -OCHR¹⁰ ou -SCHR¹⁰;
R¹⁰ représente un atome d'hydrogène ou un groupe alkyle en C₁₋₇;
X et Y représentent, indépendamment l'un de l'autre, O, S ou NH;
R^{a} et R^{b} représentent des atomes d'hydrogène; et
n est égal à 1, 2 ou 3,
et leurs sels.

3. Composés selon la revendication 1 ou 2, dans lesquels Z est -O-.

4. Composés selon les revendications 1 à 3, dans lesquels R⁶ représente un groupe alcoxy en C₁₋₇ et R⁷, R⁸ et R⁹ représentent des atomes d'hydrogène.

5. Composés selon les revendications 1 à 3, dans lesquels R⁶ et R⁸ représentent des atomes d'hydrogène, R⁷ représente un groupe alcoxy en C₁₋₇ et R⁹ représente un atome d'halogène.

6. Composés selon les revendications 1 à 5, dans lesquels R⁴ représente un atome d'hydrogène ou le groupe 2-pyrimidinyle, 2- ou 3-furyle, 2- ou 3-thiényle, morpholino ou p-méthoxyphényle.

7. Composés selon les revendications 1 à 6, dans lesquels YR⁵ représente un groupe hydroxyle, alcoxy(C₁₋₇)-sulfinyle ou furoyloxy.

8. Composés selon la revendication 3, qui sont les suivants:
p-tert-butyl-N-[6-(2-hydroxyéthoxy)-5-(o-méthoxyphénoxy)-4-pyrimidinyl]benzènesulfonamide,
N-[6-(hydroxyéthoxy)-5-(o-méthoxyphénoxy)-4-pyrimidinyl]-p-isopropylbenzènesulfonamide,
p-tert-butyl-N-[6-(2-hydroxyéthoxy)-5-(o-tolyloxy)-4-pyrimidinyl]benzènesulfonamide,
p-tert-butyl-N-[6-(2-hydroxyéthoxy)-5-(o-chlorophényloxy)-4-pyrimidinyl]benzènesulfonamide,
N-[6-(2-hydroxyéthoxy)-5-(o-chlorophénoxy)-4-pyrimidinyl]-p-isopropylbenzènesulfonamide,
p-tert-butyl-N-[6-(2-hydroxyéthoxy)-5-(m-méthoxyphénoxy)-4-pyrimidinyl]benzènesulfonamide,
p-tert-butyl-N-[6-(2-hydroxyéthoxy)-5-phénoxy-4-pyrimidinyl]benzènesulfonamide,
p-tert-butyl-N-[6-(2-hydroxyéthoxy)-5-(p-méthoxyphénoxy)-4-pyrimidinyl]benzènesulfonamide,
p-tert-butyl-N-[6-(2-hydroxyéthoxy)-5-(o-éthoxyphénoxy)-4-pyrimidinyl]benzènesulfonamide,
p-(2,2-diméthylpropyl)-N-[6-(2-hydroxyéthoxy)-5-(o-méthoxyphénoxy)-4-pyrimidinyl]benzènesulfonamide,
p-isopropyl-N-[6-(2-hydroxyéthoxy)-2-méthyl-5-(m-méthoxyphénoxy)-4-pyrimidinyl]benzènesulfonamide,
N-[6-(2-hydroxyéthoxy)-5-(o-méthoxyphénoxy)-2-phényl-4-pyrimidinyl]-p-isopropylbenzènesulfonamide,
N-[6-(2-hydroxyéthoxy)-5-(2,4,6-trichlorophénoxy)-4-pyrimidinyl]-p-isopropylbenzènesulfonamide,
N-[6-(2-hydroxyéthoxy)-6-(2,4,6-trichlorophénoxy)-4-pyrimidinyl]-o-toluènesulfonamide,
N-[6-(2-hydroxyéthoxy)-5-(2,4,6-trichlorophénoxy)-4-pyrimidinyl]-2,4-xylènesulfonamide,
p-tert-butyl-N-[6-(2-hydroxyéthoxy)-5-[(2-méthoxy-p-tolyl)oxy-4-pyrimidinyl]benzènesulfonamide,
p-tert-butyl-N-[6-(2-hydroxyéthoxy)-5-(o-méthoxyphénoxy)-2-méthyl-4-pyrimidinyl]benzènesulfonamide,
N-[6-(2-hydroxyéthoxy)-5-(o-méthoxyphénoxy)-2-méthyl-4-pyrimidinyl]-p-isopropylbenzènesulfonamide,
N-[6-(2-hydroxyéthoxy)-5-(o-méthoxyphénoxy)-(2-trifluorométhyl)-4-pyrimidinyl]-p-isopropylbenzènesulfonamide,
p-tert-butyl-N-[6-(2-hydroxyéthoxy)-5-(o-méthoxyphénoxy)-2-(trifluorométhyl)-4-pyrimidinyl]benzènesulfonamide,
N-[5-(1,3-benzodioxol-5-yloxy)-6-(2-hydroxyéthoxy)-4-pyrimidinyl]-p-tert-butylbenzènesulfonamide,
N-[5-(1,3-benzodioxol-5-yloxy)-6-(2-hydroxyéthoxy)-4-pyrimidinyl]-p-isopropylbenzènesulfonamide,
N-[6-(2-hydroxyéthoxy)-5-(o-méthoxyphénoxy)-4-pyrimidinyl]-o-méthoxybenzènesulfonamide,
p-tert-butyl-N-[6-(4-hydroxybutoxy)-5-(o-méthoxyphénoxy)-2-méthyl-4-pyrimidinyl]benzènesulfonamide,
N-[6-(2-hydroxyéthoxy)-5-(2-naphtyloxy)-4-pyrimidinyl]-p-isopropylbenzènesulfonamide,
N-[6-(2-hydroxyéthoxy)-5-(2-naphtyloxy)-4-pyrimidinyl]-p-tert-butylbenzènesulfonamide,
N-[6-(2-hydroxyéthoxy)-5-(o-méthoxyphénoxy)-2-propyl-4-pyrimidinyl]-p-isopropylbenzènesulfonamide,
p-tert-butyl-N-[6-(2-hydroxyéthoxy)-5-(o-méthoxyphénoxy)-2-propyl-4-pyrimidinyl]benzènesulfonamide,
α,α,α-trifluoro-N-[6-(2-hydroxyéthoxy)-5-(o-méthoxyphénoxy)-2-méthyl-4-pyrimidinyl]-p-toluènesulfonamide,
p-chloro-N-[6-(2-hydroxyéthoxy)-5-(o-méthoxyphénoxy)-2-méthyl-4-pyrimidinyl]benzènesulfonamide,
N-[6-(2-hydroxyéthoxy)-5-(o-méthoxyphénoxy)-2-méthyl-4-pyrimidinyl]-p-(trifluorométhoxy)benzènesulfonamide,
o-éthyl-N-[6-(2-hydroxyéthoxy)-5-(o-méthoxyphénoxy)-2-méthyl-4-pyrimidinyl]benzènesulfonamide,
N-[6-(2-hydroxyéthoxy)-5-(o-méthoxyphénoxy)-2-méthyl-4-pyrimidinyl]-p-toluènesulfonamide,
N-[6-(2-hydroxyéthoxy)-5-(o-méthoxyphénoxy)-2-méthyl-4-pyrimidinyl]-2-naphtylsulfonamide,
p-tert-butyl-N-[6-(3-hydroxypropoxy)-5-(o-méthoxyphénoxy)-2-méthyl-4-pyrimidinyl]benzènesulfonamide,
p-tert-butyl-N-[6-(2-hydroxyéthoxy)-5-[(o-méthylthio)-phénoxy]-4-pyrimidinyl]benzènesulfonamide,
p-tert-butyl-N-[6-(2-hydroxyéthoxy)-5-(o-méthoxyphénoxy)-2-phényl-4-pyrimidinyl]benzènesulfonamide,
N-[2-amino-6-(2-hydroxyéthoxy)-5-(o-méthoxyphénoxy)-4-pyrimidinyl]-p-tert-butylbenzènesulfonamide
p-tert-butyl-N-[6-(2-hydroxyéthoxy)-2-méthyl-5-[o-(méthylthio)phénoxy]-4-pyrimidinyl]benzènesulfonamide et
p-tert-butyl-N-[6-(2-hydroxyéthoxy)-2-méthyl-5-[o-(R,S-méthylsulfinyl)phénoxy]-4-pyrimidinyl]benzènesulfonamide.

9. Composés selon la revendication 3, qui sont les suivants:
4-tert-butyl-N-[5-(2-chloro-5-méthoxyphénoxy)-6-(2-hydroxyéthoxy-2-méthylpyrimidin-4-yl]benzènesulfonamide,
4-tert-butyl-N-[5-(2-chloro-5-méthoxyphénoxy)-6-(2-(3-furoyloxy)éthoxy)-2-méthylpyrimidin-4-yl]benzènesulfonamide,
4-tert-butyl-N-[6-(2-hydroxyéthoxy)-5-(2-méthoxyphénoxy)-2-(thiophén-2-yl)pyrimidin-4-yl]benzènesulfonamide,
4-tert-butyl-N-[6-(2-hydroxyéthoxy)-5-(2-méthoxyphénoxy)-2-(thiophén-3-yl)pyrimidin-4-yl]benzènesulfonamide,
4-tert-butyl-N-[2-(furann-2-yl)-6-(2-hydroxyéthoxy)-5-(2-méthoxyphénoxy)pyrimidin-4-yl]benzènesulfonamide,
4-tert-butyl-N-[2-(furann-3-yl)-6-(2-hydroxyéthoxy)-5-(2-méthoxyphénoxy)pyrimidin-4-yl]benzènesulfonamide,
4-tert-butyl-N-[6-(2-hydroxyéthoxy)-5-(2-méthoxyphénoxy)-2-(pyridin-2-yl)pyrimidin-4-yl]benzènesulfonamide,
4-tert-butyl-N-[6-(2-hydroxyéthoxy)-5-(2-méthoxyphénoxy)-2-pyridin-4-yl)pyrimidin-4-yl]benzènesulfonamide,
4-tert-butyl-N-[6-(2-hydroxyéthoxy)-5-(2-méthoxyphénoxy)-2-pyridin-3-yl)pyrimidin-4-yl]benzènesulfonamide,
oxyde-1 de 2-[4-(4-tert-butylphénylsulfonylamino)-6-(2-hydroxyéthoxy)-5-(2-méthoxyphénoxy)pyrimidin-2-yl]-pyridine,
oxyde-1 de 4-[4-(4-tert-butylphénylsulfonylamino)-6-(2-hydroxyéthoxy)-5-(2-méthoxyphénoxy)pyrimidin-2-yl]-pyridine,
4-tert-butyl-N-[6-(2-hydroxyéthoxy)-2-[2-hydroxyéthoxy)-éthyl]-5-(2-méthoxyphénoxy)pyrimidin-4-yl]benzènesulfonamide,
4-tert-butyl-N-[2-cyclopropyl-6-(2-hydroxyéthoxy)-5-(2-méthoxyphénoxy)pyrimidin-4-yl]benzènesulfonamide,
4-tert-butyl-N-[2-éthyl-6-(hydroxyéthoxy)-5-(2-méthoxyphénoxy)pyrimidin-4-yl]benzènesulfonamide,
4-tert-butyl-N-[6-(2-hydroxyéthoxy)-2-isopropyl-5-(2-méthoxyphénoxy)pyrimidin-4-yl]benzènesulfonamide,
N-[5-(5-fluoro-2-méthoxyphénoxy)-6-(2-hydroxyéthoxy)-pyrimidin-4-yl]-4-trifluorométhylbenzènesulfonamide,
4-tert-butyl-N-[6-(2-hydroxyéthoxy)-5-(3-méthoxyphénoxy)-2,2'-bipyrimidin-4-yl]benzènesulfonamide,
4-tert-butyl-N-[5-(4-fluoro-2-méthoxyphénoxy)-6-(2-hydroxyéthoxy)-2,2'-bipyrimidin-4-yl]benzènesulfonamide,
4-tert-butyl-N-[5-(4-fluoro-2-méthoxyphénoxy)-6-(2-hydroxyéthoxy)-2-méthylpyrimidin-4-yl]benzènesulfonamide,
4-tert-butyl-N-[5-(4-fluoro-2-méthoxyphénoxy)-6-(2-hydroxyéthoxy)pyrimidin-4-yl]benzènesulfonamide,
N-[5-(5-fluoro-2-méthoxyphénoxy)-6-(2-hydroxyéthoxy)-pyrimidin-4-yl]-4-isopropylbenzènesulfonamide,
N-[5-(5-fluoro-2-méthoxyphénoxy)-6-(2-hydroxyéthoxy)-pyrimidin-4-yl]-4-tert-butylbenzènesulfonamide,
4-tert-butyl-N-[5-(2-fluoro-6-méthoxy)-6-(2-hydroxyéthoxy)pyrimidin-4-yl]benzènesulfonamide,
4-tert-butyl-N-[6-(2-hydroxyéthoxy)-5-(3-méthoxyphénoxy)-2-(thiophén-2-yl)pyrimidin-4-yl]benzènesulfonamide,
4-tert-butyl-N-[6-(2-hydroxyéthoxy)-2-(2-méthoxyéthyl)-5-(3-méthoxyphénoxy)pyrimidin-4-yl]benzènesulfonamide,
3-méthylisoxazole-5-carboxylate de 2-[6-(4-tert-butylbenzènesulfonamino)-5-(3-méthoxyphénoxy)pyrimidin-4-yl-oxy)éthyle,
indole-2-carboxylate de 2-[6-(4-tert-butylbenzènesulfonamino)-5-(3-méthoxyphénoxy)pyrimidin-4-yloxy)éthyle,
5-[N-[6-(2-hydroxyéthoxy)-5-(2-méthoxyphénoxy)pyrimidin-4-yl]aminosulfonyl]-2-méthoxyphénoxyacétate d'éthyle.

10. Composé selon la revendication 1, caractérisé en ce qu'il est le (RS)-4-tert-butyl-N-[5-(2-chloro-5-méthoxyphénoxy)-2-éthyl-6-(2-méthylsulfinyléthoxy)- pyrimidin-4-yl]benzènesulfonamide.

11. Composé selon la revendication 1, caractérisé en ce qu'il est le (RS)-N-[5-(2-chloro-5-méthoxyphénoxy)-6-(2-méthylsulfinyléthoxy)pyrimidin-4-yl]-1,3-benzodioxol-5-sulfonamide.

12. Composé selon la revendication 1, caractérisé en ce qu'il est le 4-tert-butyl-N-[6-(2-hydroxyéthoxy)-5-(2-méthoxyphénoxy)-2-(pyrimidin-2-yl]pyrimidin-4-yl]-benzènesulfonamide.

13. Composés selon la revendication 1 ou 2, dans lesquels Z est le groupe vinylène.

14. Composés selon la revendication 13, qui sont les suivants:
acétate de 2-[[5-[(E/Z)-styryl]-6-p-toluènesulfonamide-4-pyrimidinyl]oxy]éthyle,
N-[6-(2-hydroxyéthoxy)-5-[(E/Z)-styryl]-4-pyrimidinyl]-p-toluènesulfonamide.

15. Composés de formule 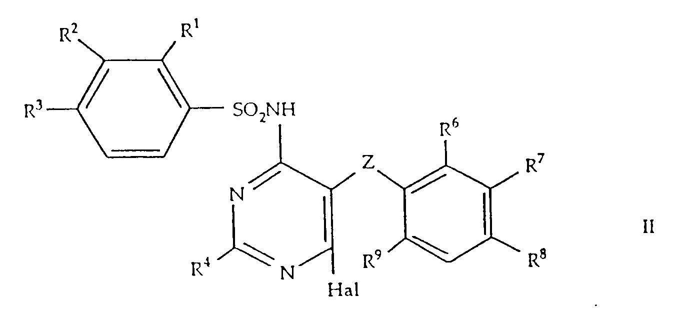 dans laquelle R¹ à R⁴, R⁶ à R⁹ et Z ont les significations données dans la revendication 1, et Hal est un atome d'halogène.

16. Composés des revendication 1 à 14, pour utilisation en tant que médicaments.

17. Composition pharmaceutique, contenant un composé des revendication 1 à 14 et des véhicules et adjuvants supplémentaires.

18. Utilisation des composés des revendications 1 à 14, en tant que substances actives dans la fabrication de médicaments destinés au traitement de maladies qui sont associées à des activités de l'endothéline, en particulier de maladies circulatoires telles que l'hypertension, l'ischémie, les angiospasmes et l'angine de poitrine.

19. Procédé pour la préparation des composés des revendications 1 à 14, caractérisé en ce que
a) on fait réagir un composé de formule 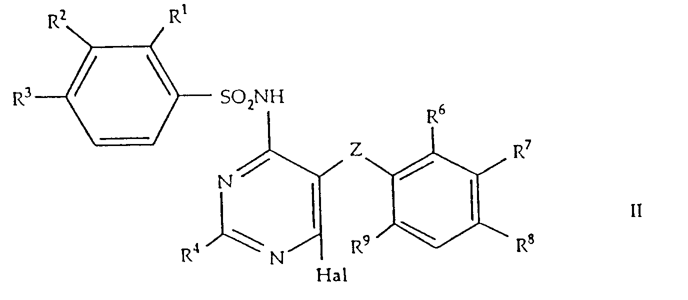 dans laquelle R¹ à R⁴ et R⁶ à R⁹ et Z ont les significations données dans la revendication 1, et Hal est un atome d'halogène, avec un composé de formule
MXCH₂(CR^{a}R^{b})ₙYR⁵ III
dans laquelle X, Y, n, R^{a}, R^{b} et R⁵ ont les significations données dans la revendication 1 et M représente un métal alcalin,
ou
b) on fait réagir un composé de formule 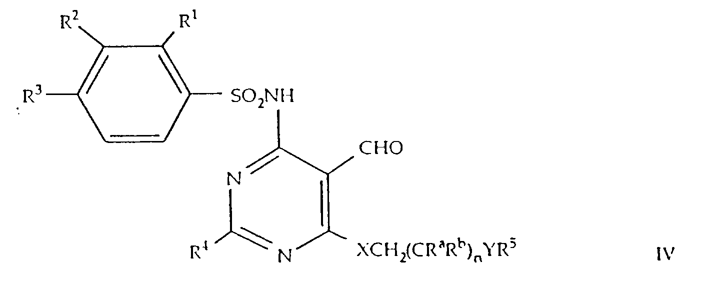 dans laquelle R¹ à R⁵, R^{a}, R^{b}, X, Y et n ont les significations données plus haut, avec un composé de formule 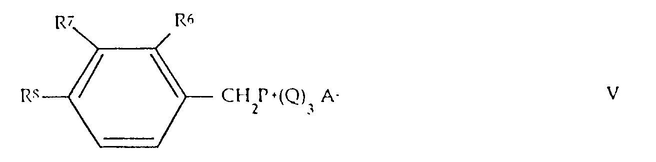 dans laquelle R⁶ à R⁸ ont les significations données plus haut, Q représente un groupe aryle et A⁻ est un anion,
ou
c) on soumet à une hydrogénation un composé de formule 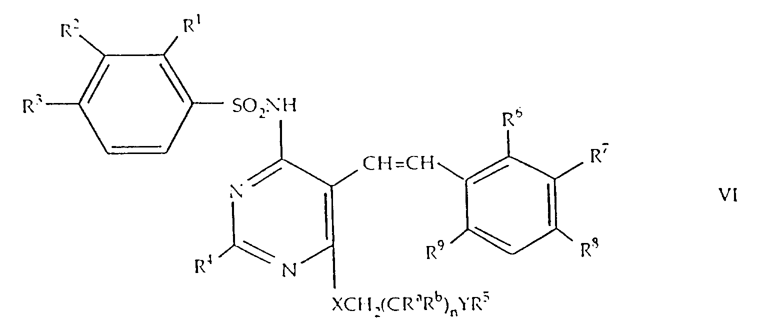 dans laquelle R¹ à R⁹, R^{a}, R^{b}, X, Y et n ont les significations données plus haut,
ou
d) on fait réagir un composé de formule 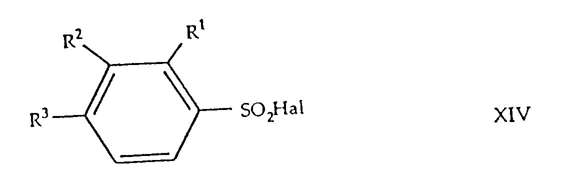 avec un composé de formule 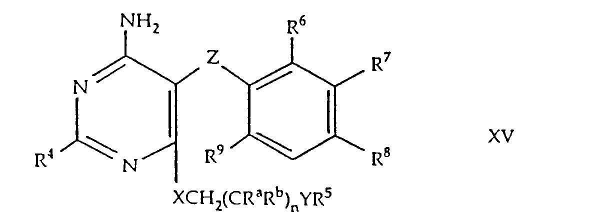 R¹ à R⁹, R^{a}, R^{b}, X, Y, Z et n ayant les significations données plus haut,
et éventuellement on modifie des substituants contenus dans le composé de formule I obtenu et/ou on convertit le composé de formule I obtenu en un sel.

## Revendications (Revendications pour l'(les) Etat(s) contractant(s) suivant(s): GR)

1. Procédé pour la préparation de composés de formule 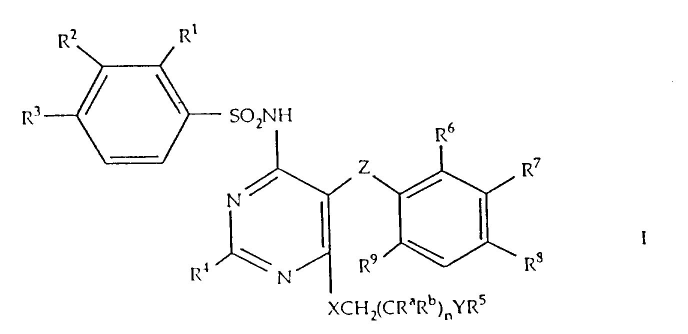 dans laquelle
R¹ représente un atome d'hydrogène ou d'halogène ou un groupe alkyle en C₁₋₇, alcoxy en C₁₋₇, alkyl(C₁₋₇)thio ou trifluorométhyle;
R² représente un atome d'hydrogène ou d'halogène ou un groupe alcoxy en C₁₋₇, trifluorométhyle ou -OCH₂COOR^{a};
R³ représente un atome d'hydrogène ou d'halogène ou un groupe alkyle en C₁₋₇, alkyl(C₁₋₇)thio, trifluorométhyle, cycloalkyle en C₃₋₈, alcoxy en C₁₋₇ ou trifluorométhoxy;
R² et R³ peuvent former ensemble un groupe butadiényle, méthylènedioxy, éthylènedioxy ou isopropylidènedioxy;
R⁴ représente un atome d'hydrogène ou un groupe alkyle en C₁₋₇, cycloalkyle en C₃₋₈, trifluorométhyle, alcoxy en C₁₋₇, alkyl(C₁₋₇)thio, alkyl(C₁₋₇)thio-alkyle(C₁₋₇), hydroxyalkyle(C₁₋₇), hydroxy-alcoxy(C₁₋₇), alcoxy(C₁₋₇)-alkyle(C₁₋₇), hydroxy-alcoxy(C₁₋₇)-alkyle(C₁₋₇), hydroxy-alcoxy(C₁₋₇)-alcoxy(C₁₋₇), alkyl(C₁₋₇)sulfinyle, alkyl(C₁₋₇)sulfonyle, 2-méthoxy-3-hydroxypropoxy, 2 -hydroxy-3-phénylpropyle, amino-alkyle(C₁₋₇), alkyl(C₁₋₇)amino-alkyle(C₁₋₇), dialkyl(C₁₋₇)amino-alkyle(C₁₋₇), amino, alkyl(C₁₋₇)amino, dialkyl(C₁₋₇)amino, arylamino, aryle, arylthio, aryloxy, aryl-alkyle(C₁₋₇), le fragment aryle représentant le groupe phényle non substitué ou un groupe phényle substitué par un groupe alkyle en C₁₋₇, trifluorométhyle, alcoxy en C₁₋₇, carboxyle ou par un atome d'halogène, un groupe hétérocyclique non substitué choisi dans l'ensemble constitué par les groupes 2-furyle, 3-furyle, pyrimidinyle, 2-pyridyle, 3-pyridyle, 4-pyridyle ou N-oxyde de 2-, 3- ou 4-pyridyle, 1,2- ou 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinyle, quinolyle, isoquinolyle, quinazolyle, ou un groupe hétérocyclique tel que défini précédemment, substitué par un atome d'halogène ou par un groupe alkyle en C₁₋₇, alcoxy en C₁₋₇, aryle ou aryl-alkyle(C₁₋₇);
R⁵ représente un atome d'hydrogène ou un groupe alkyle en C₁₋₇, alcanoyle en C₁₋₇, benzoyle, hétérocyclylcarbonyle, hétérocyclylméthyle, le fragment hétérocyclique étant choisi parmi les groupes 2-furyle, 3-furyle, pyrimidinyle, 2-pyridyle, 3-pyridyle, 4-pyridyle, N-oxyde de 2-, 3- ou 4-pyridyle, 1,2- ou 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinyle, quinolyle, isoquinolyle, quinazolyle ou étant un groupe hétérocyclique tel que défini précédemment, substitué par un atome d'halogène ou par un groupe alkyle en C₁₋₇, alcoxy en C₁₋₇, phényle ou arylalkyle(C₁₋₇); ou représente le groupe tétrahydropyrann-2-yle;
R⁶ à R⁹ représentent un atome d'hydrogène ou d'halogène ou un groupe trifluorométhyle, alkyle en C₁₋₇, alcoxy en C₁₋₇, alkyl(C₁₋₇)thio, hydroxyle, hydroxyméthyle, cyano, carboxyle, formyle, méthylsulfinyle, méthylsulfonyle, méthylsulfonyloxy ou alkyloxy(C₁₋₇)-carbonyloxy;
R⁷ forme, conjointement avec R⁶ ou R⁸, un groupe butadiényle, méthylènedioxy, éthylènedioxy ou isopropylidènedioxy;
Z représente -O-, -S-, le groupe vinylène, -CO-, -OCHR¹⁰ ou -SCHR¹⁰;
R¹⁰ représente un atome d'hydrogène ou un groupe alkyle en C₁₋₇;
X et Y représentent, indépendamment l'un de l'autre, O, S ou NH;
YR⁵ peut également représenter un groupe alkyl(C₁₋₇)-sulfinyle;
R^{a}, R^{b}, R^{c} et R^{d} représentent, indépendamment les uns des autres, un atome d'hydrogène ou un groupe alkyle en C₁₋₇; ou
R^{c} et R^{d} forment ensemble un groupe méthylène, éthylène ou isopropylidène; et
n est égal à 1, 2 ou 3,
et de leurs sels, caractérisé en ce que
a) on fait réagir un composé de formule 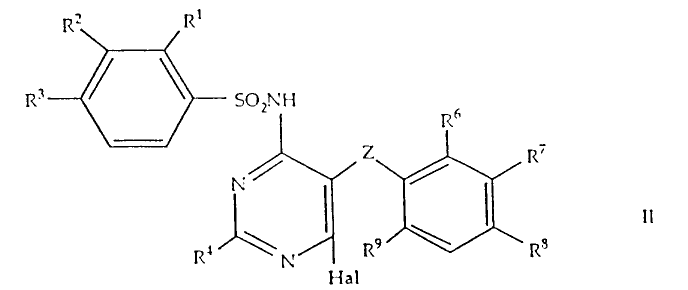 dans laquelle R¹ à R⁴ et R⁶ à R⁹ et Z ont les significations données dans la revendication 1, et Hal est un atome d'halogène,
avec un composé de formule
MXCH₂(CR^{a}R^{b})ₙYR⁵ III
dans laquelle X, Y, n, R^{a}, R^{b} et R⁵ ont les significations données plus haut et M représente un métal alcalin,
ou
b) on fait réagir un composé de formule 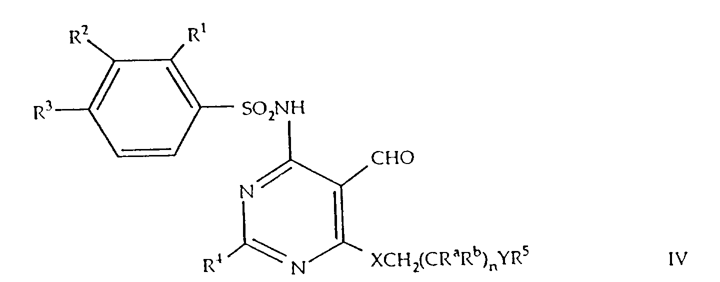 dans laquelle R¹ à R⁵, R^{a}, R^{b}, X, Y et n ont les significations données plus haut,
avec un composé de formule 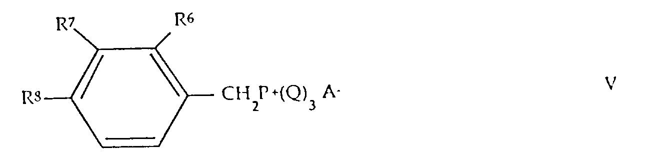 dans laquelle R⁶ à R⁸ ont les significations données plus haut, Q représente un groupe aryle et A⁻ est un anion,
ou
c) on soumet à une hydrogénation un composé de formule 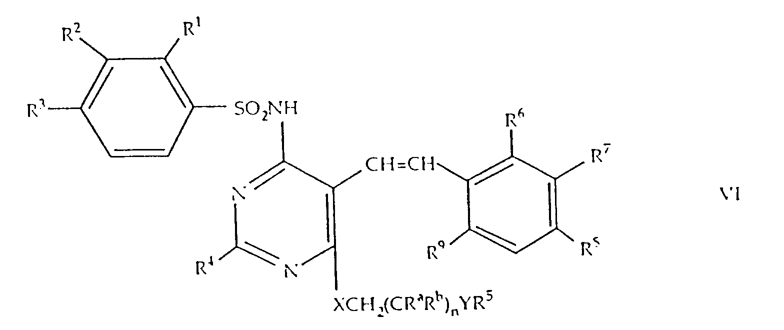 dans laquelle R¹ à R⁹, R^{a}, R^{b}, X, Y et n ont les significations données plus haut,
ou
d) on fait réagir un composé de formule 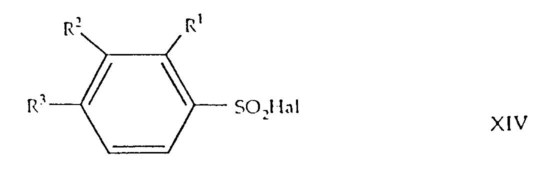 avec un composé de formule 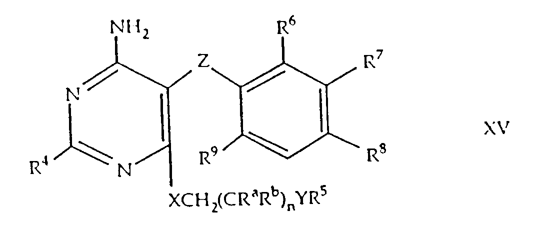 R¹ à R⁹, R^{a}, R^{b}, X, Y, Z et n ayant les significations données plus haut,
et éventuellement on modifie des substituants contenus dans le composé de formule I obtenu et/ou on convertit le composé de formule I obtenu en un sel.

2. Procédé selon la revendication 1, pour la préparation des composés de formule 1 et de leurs sels, dans lesquels
R¹ représente un atome d'hydrogène ou d'halogène ou un groupe alkyle en C₁₋₇, alcoxy en C₁₋₇, alkyl(C₁₋₇)thio ou trifluorométhyle;
R² représente un atome d'hydrogène ou d'halogène ou un groupe alcoxy en C₁₋₇ ou trifluorométhyle;
R³ représente un atome d'hydrogène ou d'halogène ou un groupe alkyle en C₁₋₇, alkyl(C₁₋₇)thio, trifluorométhyle, cycloalkyle en C₃₋₈, alcoxy en C₁₋₇ ou trifluorométhoxy;
R² et R³ forment ensemble un groupe butadiényle ou méthylènedioxy;
R⁴ représente un atome d'hydrogène ou un groupe alkyle en C₁₋₇, trifluorométhyle, alcoxy en C₁₋₇, alkyl(C₁₋₇)thio, alkyl(C₁₋₇)thio-alkyle(C₁₋₇), hydroxyalkyle(C₁₋₇), amino-alkyle(C₁₋₇), alkyl(C₁₋₇)amino-alkyle(C₁₋₇), dialkyl(C₁₋₇)amino-alkyle(C₁₋₇), amino, alkyl(C₁₋₇)amino, dialkyl(C₁₋₇)amino, arylamino, aryle, arylthio, aryloxy, aryl-alkyle(C₁₋₇), le fragment aryle étant le groupe phényle non substitué ou un groupe phényle substitué par un atome d'halogène ou par un groupe alkyle en C₁₋₇, trifluorométhyle, alcoxy en C₁₋₇, carboxyle, un groupe hétérocyclique non substitué choisi dans l'ensemble constitué par les groupes 2-furyle, 3-furyle, pyrimidinyle, 2-pyridyle, 3-pyridyle, 4-pyridyle, N-oxyde de 2-, 3- ou 4-pyridyle, 1,2- ou 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinolyle, quinolyle, isoquinolyle, quinazolyle et tétrahydropyrann-2₋yle, ou un groupe hétérocyclique tel que défini précédemment, substitué par un atome d'halogène ou par un groupe alkyle en C₁₋₇, alcoxy en C₁₋₇, aryle ou aryl-alkyle(C₁₋₇);
R⁵ représente un atome d'hydrogène ou un groupe alcanoyle en C₁₋₇, benzoyle ou tétrahydropyrann-2-yle;
R⁶ à R⁹ représentent un atome d'hydrogène ou d'halogène ou un groupe trifluorométhyle, alkyle en C₁₋₇, alcoxy en C₁₋₇, alkyl(C₁₋₇)thio, hydroxyle, hydroxyméthyle, cyano, carboxyle, formyle, méthylsulfinyle, méthylsulfonyle, méthylsulfonyloxy ou alkyloxy(C₁₋₇)carbonyloxy;
R⁷ peut former, conjointement avec R⁶ ou R⁸, le groupe butadiényle ou -OCH₂O-;
Z représente -O-, -S-, un groupe vinylène, -CO-, -OCHR¹⁰ ou -SCHR¹⁰;
R¹⁰ représente un atome d'hydrogène ou un groupe alkyle en C₁₋₇;
X et Y représentent, indépendamment l'un de l'autre, O, S ou NH;
R^{a} et R^{b} représentent des atomes d'hydrogène; et
n est égal à 1, 2 ou 3.

3. Procédé selon la revendication 1 ou 2, pour la préparation des composés de formule I dans lesquels Z est -O-.

4. Procédé selon les revendications 1 à 3, pour la préparation des composés de formule I dans lesquels R⁶ représente un groupe alcoxy en C₁₋₇ et R⁷, R⁸ et R⁹ représentent des atomes d'hydrogène.

5. Procédé selon les revendications 1 à 3, pour la préparation des composés de formule I dans lesquels R⁶ et R⁸ représentent des atomes d'hydrogène, R⁷ représente un groupe alcoxy en C₁₋₇ et R⁹ représente un atome d'halogène.

6. Procédé selon les revendications 1 à 3, pour la préparation des composés de formule I dans lesquels R⁴ représente un atome d'hydrogène ou le groupe 2-pyrimidinyle, 2- ou 3-furyle, 2- ou 3-thiényle, morpholino ou p-méthoxyphényle.

7. Procédé selon les revendications 1 à 3, pour la préparation des composés de formule I dans lesquels YR⁵ représente un groupe hydroxyle, alcoxy(C₁₋₇)sulfinyle ou furoyloxy.

8. Procédé selon la revendication 3, pour la préparation des composés suivants:
p-tert-butyl-N-[6-(2-hydroxyéthoxy)-5-(o-méthoxyphénoxy)-4-pyrimidinyl]benzènesulfonamide,
N-[6-(hydroxyéthoxy)-5-(o-méthoxyphénoxy)-4-pyrimidinyl]-p-isopropylbenzènesulfonamide,
p-tert-butyl-N-[6-(2-hydroxyéthoxy)-5-(o-tolyloxy)-4-pyrimidinyl]benzènesulfonamide,
p-tert-butyl-N-[6-(2-hydroxyéthoxy)-5-(o-chlorophényloxy)-4-pyrimidinyl]benzènesulfonamide,
N-[6-(2-hydroxyéthoxy)-5-(o-chlorophénoxy)-4-pyrimidinyl]-p-isopropylbenzènesulfonamide,
p-tert-butyl-N-[6-(2-hydroxyéthoxy)-5-(m-méthoxyphénoxy)-4-pyrimidinyl]benzènesulfonamide,
p-tert-butyl-N-[6-(2-hydroxyéthoxy)-5-phénoxy-4-pyrimidinyl]benzènesulfonamide,
p-tert-butyl-N-[6-(2-hydroxyéthoxy)-5-(p-méthoxyphénoxy)-4-pyrimidinyl]benzènesulfonamide,
p-tert-butyl-N-[6-(2-hydroxyéthoxy)-5-(o-éthoxyphénoxy)-4-pyrimidinyl]benzènesulfonamide,
p-(2,2-diméthylpropyl)-N-[6-(2-hydroxyéthoxy)-5-(o-méthoxyphénoxy)-4-pyrimidinyl]benzènesulfonamide,
p-isopropyl-N-[6-(2-hydroxyéthoxy)-2-méthyl-5-(m-méthoxyphénoxy)-4-pyrimidinyl]benzènesulfonamide,
N-[6-(2-hydroxyéthoxy)-5-(o-méthoxyphénoxy)-2-phényl-4-pyrimidinyl]-p-isopropylbenzènesulfonamide,
N-[6-(2-hydroxyéthoxy)-5-(2,4,6-trichlorophénoxy)-4-pyrimidinyl]-p-isopropylbenzènesulfonamide,
N-[6-(2-hydroxyéthoxy)-6-(2,4,6-trichlorophénoxy)-4-pyrimidinyl]-o-toluènesulfonamide,
N-[6-(2-hydroxyéthoxy)-5-(2,4,6-trichlorophénoxy)-4-pyrimidinyl]-2,4-xylènesulfonamide,
p-tert-butyl-N-[6-(2-hydroxyéthoxy)-5-[(2-méthoxy-p-tolyl)oxy-4-pyrimidinyl]benzènesulfonamide,
p-tert-butyl-N-[6-(2-hydroxyéthoxy)-5-(o-méthoxyphénoxy)-2-méthyl-4-pyrimidinyl]benzènesulfonamide,
N-[6-(2-hydroxyéthoxy)-5-(o-méthoxyphénoxy)-2-méthyl-4-pyrimidinyl]-p-isopropylbenzènesulfonamide,
N-[6-(2-hydroxyéthoxy)-5-(o-méthoxyphénoxy)-(2-trifluorométhyl)-4-pyrimidinyl]-p-isopropylbenzènesulfonamide,
p-tert-butyl-N-[6-(2-hydroxyéthoxy)-5-(o-méthoxyphénoxy)-2-(trifluorométhyl)-4-pyrimidinyl]benzènesulfonamide,
N-[5-(1,3-benzodioxol-5-yloxy)-6-(2-hydroxyéthoxy)-4-pyrimidinyl]-p-tert-butylbenzènesulfonamide,
N-[5-(1,3-benzodioxol-5-yloxy)-6-(2-hydroxyéthoxy)-4-pyrimidinyl]-p-isopropylbenzènesulfonamide,
N-[6-(2-hydroxyéthoxy)-5-(o-méthoxyphénoxy)-4-pyrimidinyl]-o-méthoxybenzènesulfonamide,
p-tert-butyl-N-[6-(4-hydroxybutoxy)-5-(o-méthoxyphénoxy)-2-méthyl-4-pyrimidinyl]benzènesulfonamide,
N-[6-(2-hydroxyéthoxy)-5-(2-naphtyloxy)-4-pyrimidinyl]-p-isopropylbenzènesulfonamide,
N-[6-(2-hydroxyéthoxy)-5-(2-naphtyloxy)-4-pyrimidinyl]-p-tert-butylbenzènesulfonamide,
N-[6-(2-hydroxyéthoxy)-5-(o-méthoxyphénoxy)-2-propyl-4-pyrimidinyl]-p-isopropylbenzènesulfonamide,
p-tert-butyl-N-[6-(2-hydroxyéthoxy)-5-(o-méthoxyphénoxy)-2-propyl-4-pyrimidinyl]benzènesulfonamide,
α,α,α-trifluoro-N-[6-(2-hydroxyéthoxy)-5-(o-méthoxyphénoxy)-2-méthyl-4-pyrimidinyl]-p-toluènesulfonamide,
p-chloro-N-[6-(2-hydroxyéthoxy)-5-(o-méthoxyphénoxy)-2-méthyl-4-pyrimidinyl]benzènesulfonamide,
N-[6-(2-hydroxyéthoxy)-5-(o-méthoxyphénoxy)-2-méthyl-4-pyrimidinyl]-p-(trifluorométhoxy)benzènesulfonamide,
o-éthyl-N-[6-(2-hydroxyéthoxy)-5-(o-méthoxyphénoxy)-2-méthyl-4-pyrimidinyl]benzènesulfonamide,
N-[6-(2-hydroxyéthoxy)-5-(o-méthoxyphénoxy)-2-méthyl-4-pyrimidinyl]-p-toluènesulfonamide,
N-[6-(2-hydroxyéthoxy)-5-(o-méthoxyphénoxy)-2-méthyl-4-pyrimidinyl]-2-naphtylsulfonamide,
p-tert-butyl-N-[6-(3-hydroxypropoxy)-5-(o-méthoxyphénoxy)-2-méthyl-4-pyrimidinyl]benzènesulfonamide,
p-tert-butyl-N-[6-(2-hydroxyéthoxy)-5-[(o-méthylthio)-phénoxy]-4-pyrimidinyl]benzènesulfonamide,
p-tert-butyl-N-[6-(2-hydroxyéthoxy)-5-(o-méthoxyphénoxy)-2-phényl-4-pyrimidinyl]benzènesulfonamide,
N-[2-amino-6-(2-hydroxyéthoxy)-5-(o-méthoxyphénoxy)-4-pyrimidinyl]-p-tert-butylbenzènesulfonamide,
p-tert-butyl-N-[6-(2-hydroxyéthoxy)-2-méthyl-5-[o-(méthylthio)phénoxy]-4-pyrimidinyl]benzènesulfonamide et
p-tert-butyl-N-[6-(2-hydroxyéthoxy)-2-méthyl-5-[o-(R,S-méthylsulfinyl)phénoxy]-4-pyrimidinyl]benzènesulfonamide.

9. Procédé selon la revendication 3, pour la préparation des composés suivants:
4-tert-butyl-N-[5-(2-chloro-5-méthoxyphénoxy)-6-(2-hydroxyéthoxy-2-méthylpyrimidin-4-yl]benzènesulfonamide,
4-tert-butyl-N-[5-(2-chloro-5-méthoxyphénoxy)-6-(2-(3-furoyloxy)éthoxy)-2-méthylpyrimidin-4-yl]benzènesulfonamide,
4-tert-butyl-N-[6-(2-hydroxyéthoxy)-5-(2-méthoxyphénoxy)-2-(thiophén-2-yl)pyrimidin-4-yl]benzènesulfonamide,
4-tert-butyl-N-[6-(2-hydroxyéthoxy)-5-(2-méthoxyphénoxy)-2-(thiophén-3-yl)pyrimidin-4-yl]benzènesulfonamide,
4-tert-butyl-N-[2-(furann-2-yl)-6-(2-hydroxyéthoxy)-5-(2-méthoxyphénoxy)pyrimidin-4-yl]benzènesulfonamide,
4-tert-butyl-N-[2-(furann-3-yl)-6-(2-hydroxyéthoxy)-5-(2-méthoxyphénoxy)pyrimidin-4-yl]benzènesulfonamide,
4-tert-butyl-N-[6-(2-hydroxyéthoxy)-5-(2-méthoxyphénoxy)-2-(pyridin-2-yl)pyrimidin-4-yl]benzènesulfonamide,
4-tert-butyl-N-[6-(2-hydroxyéthoxy)-5-(2-méthoxyphénoxy)-2-pyridin-4-yl)pyrimidin-4-yl]benzènesulfonamide,
4-tert-butyl-N-[6-(2-hydroxyéthoxy)-5-(2-méthoxyphénoxy)-2-pyridin-3-yl)pyrimidin-4-yl]benzènesulfonamide,
oxyde-1 de 2-[4-(4-tert-butylphénylsulfonylamino)-6-(2-hydroxyéthoxy)-5-(2-méthoxyphénoxy)pyrimidin-2-yl]-pyridine,
oxyde-1 de 4-[4-(4-tert-butylphénylsulfonylamino)-6-(2-hydroxyéthoxy)-5-(2-méthoxyphénoxy)pyrimidin-2-yl]-pyridine,
4-tert-butyl-N-[6-(2-hydroxyéthoxy)-2-[2-hydroxyéthoxy)-éthyl]-5-(2-méthoxyphénoxy)pyrimidin-4-yl]benzènesulfonamide,
4-tert-butyl-N-[2-cyclopropyl-6-(2-hydroxyéthoxy)-5-(2-méthoxyphénoxy)pyrimidin-4-yl]benzènesulfonamide,
4-tert-butyl-N-[2-éthyl-6-(hydroxyéthoxy)-5-(2-méthoxyphénoxy)pyrimidin-4-yl]benzènesulfonamide,
4-tert-butyl-N-[6-(2-hydroxyéthoxy)-2-isopropyl-5-(2-méthoxyphénoxy)pyrimidin-4-yl]benzènesulfonamide,
N-[5-(5-fluoro-2-méthoxyphénoxy)-6-(2-hydroxyéthoxy)-pyrimidin-4-yl]-4-trifluorométhylbenzènesulfonamide,
4-tert-butyl-N-[6-(2-hydroxyéthoxy)-5-(3-méthoxyphénoxy)-2,2'-bipyrimidin-4-yl]benzènesulfonamide,
4-tert-butyl-N-[5-(4-fluoro-2-méthoxyphénoxy)-6-(2-hydroxyéthoxy)-2,2'-bipyrimidin-4-yl]benzènesulfonamide,
4-tert-butyl-N-[5-(4-fluoro-2-méthoxyphénoxy)-6-(2-hydroxyéthoxy)-2-méthylpyrimidin-4-yl]benzènesulfonamide,
4-tert-butyl-N-[5-(4-fluoro-2-méthoxyphénoxy)-6-(2-hydroxyéthoxy)pyrimidin-4-yl]benzènesulfonamide,
N-[5-(5-fluoro-2-méthoxyphénoxy)-6-(2-hydroxyéthoxy)-pyrimidin-4-yl]-4-isopropylbenzènesulfonamide,
N-[5-(5-fluoro-2-méthoxyphénoxy)-6-(2-hydroxyéthoxy)-pyrimidin-4-yl]-4-tert-butylbenzènesulfonamide,
4-tert-butyl-N-[5-(2-fluoro-6-méthoxy)-6-(2-hydroxyéthoxy)pyrimidin-4-yl]benzènesulfonamide,
4-tert-butyl-N-[6-(2-hydroxyéthoxy)-5-(3-méthoxyphénoxy)-2-(thiophén-2-yl)pyrimidin-4-yl]benzènesulfonamide,
4-tert-butyl-N-[6-(2-hydroxyéthoxy)-2-(2-méthoxyéthyl)-5-(3-méthoxyphénoxy)pyrimidin-4-yl]benzènesulfonamide,
3-méthylisoxazoie-5-carboxylate de 2-[6-(4-tert-butyl-benzènesulfonamino)-5-(3-méthoxyphénoxy)pyrimidin-4-yl-oxy)éthyle,
indole-2-carboxylate de 2-[6-(4-tert-butylbenzènesulfonamino)-5-(3-méthoxyphénoxy)pyrimidin-4-yloxy)éthyle,
5-[N-[6-(2-hydroxyéthoxy)-5-(2-méthoxyphénoxy)pyrimidin-4-yl]aminosulfonyl]-2-méthoxyphénoxyacétate d'éthyle.

10. Procédé selon la revendication 3, pour la préparation du (RS)-4-tert-butyl-N-[5-(2-chloro-5-méthoxyphénoxy)-2-éthyl-6-(2-méthylsulfinyléthoxy)pyrimidin-4-yl]benzènesulfonamide.

11. Procédé selon la revendication 3, pour la préparation du (RS)-N-[5-(2-chloro-5-méthoxyphénoxy)-6-(2-méthylsulfinyléthoxy)pyrimidin-4-yl]-1,3-benzodioxol-5-sulfonamide.

12. Procédé selon la revendication 3, pour la préparation du 4-tert-butyl-N-[6-(2-hydroxyéthoxy)-5-(2-méthoxyphénoxy)-2-(pyrimidin-2-yl]pyrimidin-4-yl]benzènesulfonamide.

13. Procédé selon la revendication 1 ou 2, pour la préparation des composés de formule I dans lesquels Z est le groupe vinylène.

14. Procédé selon la revendication 14, pour la préparation des composés suivants:
acétate de 2-[[5-[(E/Z)-styryl]-6-p-toluènesulfonamide-4-pyrimidinyl]oxy]éthyle,
N-[6-(2-hydroxyéthoxy)-5-[(E/Z)-styryl]-4-pyrimidinyl]-p-toluènesulfonamide.

15. Procédé pour la préparation de compositions pharmaceutiques, caractérisé en ce que l'on mélange un composé de formule I de la revendication 1, ou un de ses sels, avec des adjuvants et véhicules pharmaceutiques usuels, et on met ce mélange sous une forme appropriée à l'application thérapeutique.

16. Utilisation des composés de formule I de la revendication 1 et de leurs sels, en tant que substances actives dans la fabrication de médicaments destinés au traitement de maladies qui sont associées à des activités de l'endothéline, en particulier de maladies circulatoires telles que l'hypertension, l'ischémie, les angiospasmes et l'angine de poitrine.

17. Composés de formule 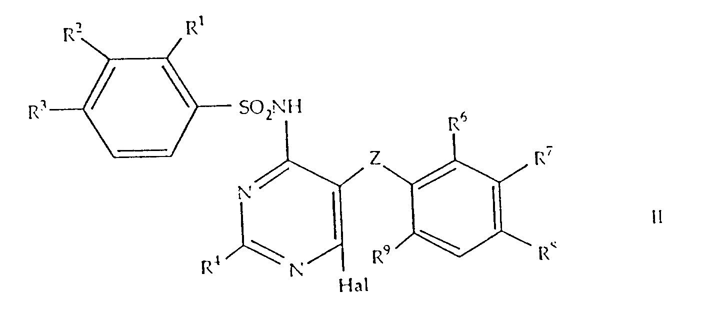 dans laquelle R¹ à R⁵, R^{a}, R^{b}, Y et n ont les significations données dans la revendication 1, et Hal est un atome d'halogène.

## Revendications (Revendications pour l'(les) Etat(s) contractant(s) suivant(s): ES)

1. Procédé pour la préparation de composés de formule 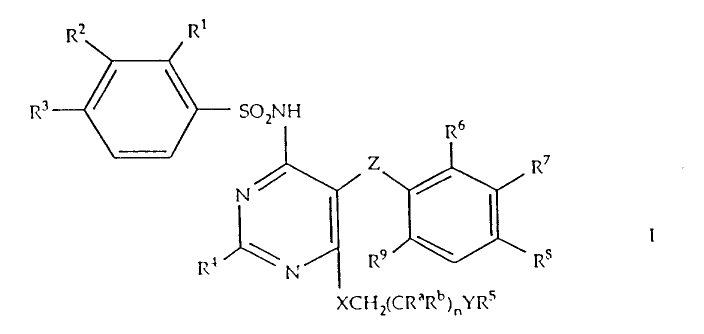 dans laquelle
R¹ représente un atome d'hydrogène ou d'halogène ou un groupe alkyle en C₁₋₇, alcoxy en C₁₋₇, alkyl(C₁₋₇)thio ou trifluorométhyle;
R² représente un atome d'hydrogène ou d'halogène ou un groupe alcoxy en C₁₋₇, trifluorométhyle ou -OCH₂COOR^{a};
R³ représente un atome d'hydrogène ou d'halogène ou un groupe alkyle en C₁₋₇, alkyl(C₁₋₇)thio, trifluorométhyle, cycloalkyle en C₃₋₈, alcoxy en C₁₋₇ ou trifluorométhoxy;
R² et R³ peuvent former ensemble un groupe butadiényle, méthylènedioxy, éthylènedioxy ou isopropylidènedioxy;
R⁴ représente un atome d'hydrogène ou un groupe alkyle en C₁₋₇, cycloalkyle en C₃₋₈, trifluorométhyle, alcoxy en C₁₋₇, alkyl(C₁₋₇)thio, alkyl(C₁₋₇)thio-alkyle(C₁₋₇), hydroxyalkyle(C₁₋₇), hydroxy-alcoxy(C₁₋₇), alcoxy(C₁₋₇)-alkyle(C₁₋₇), hydroxy-alcoxy(C₁₋₇)-alkyle(C₁₋₇), hydroxy-alcoxy(C₁₋₇)-alcoxy(C₁₋₇), alkyl(C₁₋₇)sulfinyle, alkyl(C₁₋₇)sulfonyle, 2-méthoxy-3-hydroxypropoxy, 2-hydroxy-3-phénylpropyle, amino-alkyle(C₁₋₇), alkyl(C₁₋₇)amino-alkyle(C₁₋₇), dialkyl(C₁₋₇)amino-alkyle(C₁₋₇), amino, alkyl(C₁₋₇)amino, dialkyl(C₁₋₇)amino, arylamino, aryle, arylthio, aryloxy, aryl-alkyle(C₁₋₇), le fragment aryle représentant le groupe phényle non substitué ou un groupe phényle substitué par un groupe alkyle en C₁₋₇, trifluorométhyle, alcoxy en C₁₋₇, carboxyle ou par un atome d'halogène, un groupe hétérocyclique non substitué choisi dans l'ensemble constitué par les groupes 2-furyle, 3-furyle, pyrimidinyle, 2-pyridyle, 3-pyridyle, 4-pyridyle ou N-oxyde de 2-, 3- ou 4-pyridyle, 1,2- ou 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinyle, quinolyle, isoquinolyle, quinazolyle, ou un groupe hétérocyclique tel que défini précédemment, substitué par un atome d'halogène ou par un groupe alkyle en C₁₋₇, alcoxy en C₁₋₇, aryle ou aryl-alkyle(C₁₋₇);
R⁵ représente un atome d'hydrogène ou un groupe alkyle en C₁₋₇, alcanoyle en C₁₋₇, benzoyle, hétérocyclylcarbonyle, hétérocyclylméthyle, le fragment hétérocyclique étant choisi parmi les groupes 2-furyle, 3-furyle, pyrimidinyle, 2-pyridyle, 3-pyridyle, 4-pyridyle, N-oxyde de 2-, 3- ou 4-pyridyle, 1,2- ou 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinyle, quinolyle, isoquinolyle, quinazolyle ou étant un groupe hétérocyclique tel que défini précédemment, substitué par un atome d'halogène ou par un groupe alkyle en C₁₋₇, alcoxy en C₁₋₇, phényle ou aryl-alkyle(C₁₋₇); ou représente le groupe tétrahydropyrann-2-yle;
R⁶ à R⁹ représentent un atome d'hydrogène ou d'halogène ou un groupe trifluorométhyle, alkyle en C₁₋₇, alcoxy en C₁₋₇, alkyl(C₁₋₇)thio, hydroxyle, hydroxyméthyle, cyano, carboxyle, formyle, méthylsulfinyle, méthylsulfonyle, méthylsulfonyloxy ou alkyloxy(C₁₋₇)-carbonyloxy;
R⁷ forme, conjointement avec R⁶ ou R⁸, un groupe butadiényle, méthylènedioxy, éthylènedioxy ou isopropylidènedioxy;
Z représente -O-, -S-, le groupe vinylène, -CO-, -OCHR¹⁰ ou -SCHR¹⁰;
R¹⁰ représente un atome d'hydrogène ou un groupe alkyle en C₁₋₇;
X et Y représentent, indépendamment l'un de l'autre, O, S ou NH;
YR⁵ peut également représenter un groupe alkyl(C₁₋₇)-sulfinyle;
R^{a}, R^{b}, R^{c} et R^{d} représentent, indépendamment les uns des autres, un atome d'hydrogène ou un groupe alkyle en C₁₋₇; ou
R^{c} et R^{d} forment ensemble un groupe méthylène, éthylène ou isopropylidène; et
n est égal à 1, 2 ou 3,
et de leurs sels, caractérisé en ce que
a) on fait réagir un composé de formule 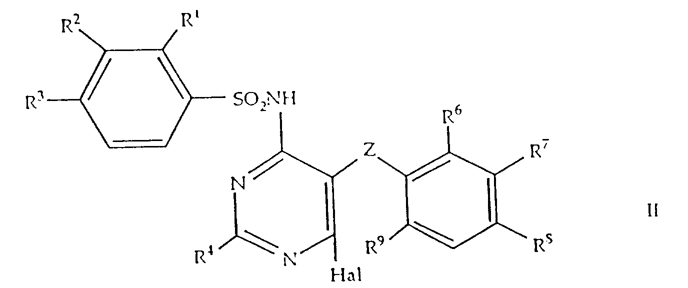 dans laquelle R¹ à R⁴ et R⁶ à R⁹ et Z ont les significations données dans la revendication 1, et Hal est un atome d'halogène,
avec un composé de formule
MXCH₂(CR^{a}R^{b})ₙYR⁵ III
dans laquelle X, Y, n, R^{a}, R^{b} et R⁵ ont les significations données plus haut et M représente un métal alcalin,
ou
b) on fait réagir un composé de formule 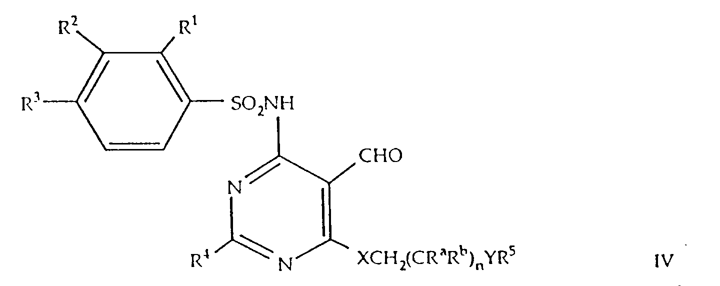 dans laquelle R¹ à R⁵, R^{a}, R^{b}, X, Y et n ont les significations données plus haut,
avec un composé de formule 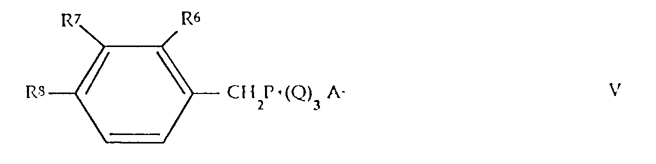 dans laquelle R⁶ à R⁸ ont les significations données plus haut, Q représente un groupe aryle et A⁻ est un anion,
ou
c) on soumet à une hydrogénation un composé de formule 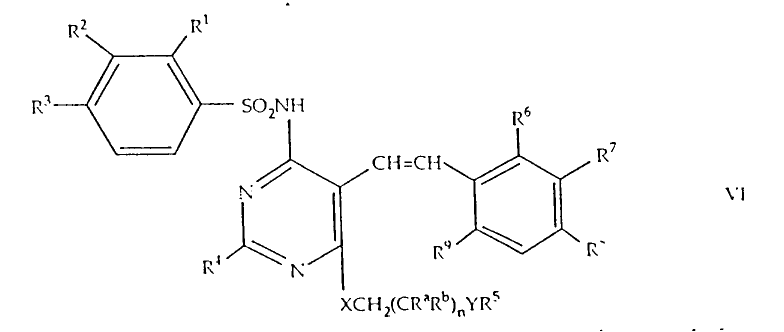 dans laquelle R¹ à R⁹, R^{a}, R^{b}, X, Y et n ont les significations données plus haut,
ou
d) on fait réagir un composé de formule 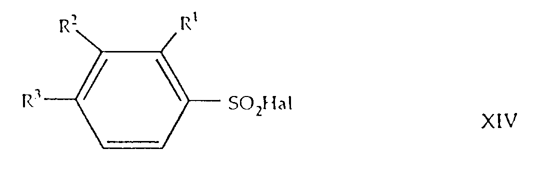 avec un composé de formule 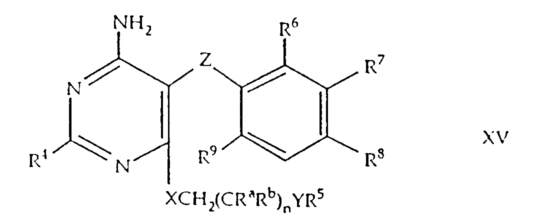 R¹ à R⁹, R^{a}, R^{b}, X, Y, Z et n ayant les significations données plus haut,
et éventuellement on modifie des substituants contenus dans le composé de formule I obtenu et/ou on convertit le composé de formule I obtenu en un sel.

2. Procédé selon la revendication 1, pour la préparation des composés de formule 1 et de leurs sels, dans lesquels
R¹ représente un atome d'hydrogène ou d'halogène ou un groupe alkyle en C₁₋₇, alcoxy en C₁₋₇, alkyl(C₁₋₇)thio ou trifluorométhyle;
R² représente un atome d'hydrogène ou d'halogène ou un groupe alcoxy en C₁₋₇ ou trifluorométhyle;
R³ représente un atome d'hydrogène ou d'halogène ou un groupe alkyle en C₁₋₇, alkyl(C₁₋₇)thio, trifluorométhyle, cycloalkyle en C₃₋₈, alcoxy en C₁₋₇ ou trifluorométhoxy;
R² et R³ forment ensemble un groupe butadiényle ou méthylènedioxy;
R⁴ représente un atome d'hydrogène ou un groupe alkyle en C₁₋₇, trifluorométhyle, alcoxy en C₁₋₇, alkyl(C₁₋₇)thio, alkyl(C₁₋₇)thio-alkyle(C₁₋₇), hydroxyalkyle(C₁₋₇), amino-alkyle(C₁₋₇), alkyl(C₁₋₇)amino-alkyle(C₁₋₇), dialkyl(C₁₋₇)amino-alkyle(C₁₋₇), amino, alkyl(C₁₋₇)amino, dialkyl(C₁₋₇)amino, arylamino, aryle, arylthio, aryloxy, aryl-alkyle(C₁₋₇), le fragment aryle étant le groupe phényle non substitué ou un groupe phényle substitué par un atome d'halogène ou par un groupe alkyle en C₁₋₇, trifluorométhyle, alcoxy en C₁₋₇, carboxyle, un groupe hétérocyclique non substitué choisi dans l'ensemble constitué par les groupes 2-furyle, 3-furyle, pyrimidinyle, 2-pyridyle, 3-pyridyle, 4-pyridyle, N-oxyde de 2-, 3- ou 4-pyridyle, 1,2- ou 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinolyle, quinolyle, isoquinolyle, quinazolyle et tétrahydropyrann-2-yle, ou un groupe hétérocyclique tel que défini précédemment, substitué par un atome d'halogène ou par un groupe alkyle en C₁₋₇, alcoxy en C₁₋₇, aryle ou aryl-alkyle(C₁₋₇);
R⁵ représente un atome d'hydrogène ou un groupe alcanoyle en C₁₋₇, benzoyle ou tétrahydropyrann-2-yle;
R⁶ à R⁹ représentent un atome d'hydrogène ou d'halogène ou un groupe trifluorométhyle, alkyle en C₁₋₇, alcoxy en C₁₋₇, alkyl(C₁₋₇)thio, hydroxyle, hydroxyméthyle, cyano, carboxyle, formyle, méthylsulfinyle, méthylsulfonyle, méthylsulfonyloxy ou alkyloxy(C₁₋₇)-carbonyloxy;
R⁷ peut former, conjointement avec R⁶ ou R⁸, le groupe butadiényle ou -OCH₂O-;
Z représente -O-, -S-, un groupe vinylène, -CO-, -OCHR¹⁰ ou -SCHR¹⁰;
R¹⁰ représente un atome d'hydrogène ou un groupe alkyle en C₁₋₇;
X et Y représentent, indépendamment l'un de l'autre, O, S ou NH;
R^{a} et R^{b} représentent des atomes d'hydrogène; et
n est égal à 1, 2 ou 3.

3. Procédé selon la revendication 1 ou 2, pour la préparation des composés de formule I dans lesquels Z est -O-.

4. Procédé selon les revendications 1 à 3, pour la préparation des composés de formule I dans lesquels R⁶ représente un groupe alcoxy en C₁₋₇ et R⁷, R⁸ et R⁹ représentent des atomes d'hydrogène.

5. Procédé selon les revendications 1 à 3, pour la préparation des composés de formule I dans lesquels R⁶ et R⁸ représentent des atomes d'hydrogène, R⁷ représente un groupe alcoxy en C₁₋₇ et R⁹ représente un atome d'halogène.

6. Procédé selon les revendications 1 à 3, pour la préparation des composés de formule I dans lesquels R⁴ représente un atome d'hydrogène ou le groupe 2-pyrimidinyle, 2- ou 3-furyle, 2- ou 3-thiényle, morpholino ou p-méthoxyphényle.

7. Procédé selon les revendications 1 à 3, pour la préparation des composés de formule I dans lesquels YR⁵ représente un groupe hydroxyle, alcoxy(C₁₋₇)sulfinyle ou furoyloxy.

8. Procédé selon la revendication 3, pour la préparation des composés suivants:
p-tert-butyl-N-[6-(2-hydroxyéthoxy)-5-(o-méthoxyphénoxy)-4-pyrimidinyl]benzènesulfonamide,
N-[6-(hydroxyéthoxy)-5-(o-méthoxyphénoxy)-4-pyrimidinyl]-p-isopropylbenzènesulfonamide,
p-tert-butyl-N-[6-(2-hydroxyéthoxy)-5-(o-tolyloxy)-4-pyrimidinyl]benzènesulfonamide,
p-tert-butyl-N-[6-(2-hydroxyéthoxy)-5-(o-chlorophényloxy)-4-pyrimidinyl]benzènesulfonamide,
N-[6-(2-hydroxyéthoxy)-5-(o-chlorophénoxy)-4-pyrimidinyl]-p-isopropylbenzènesulfonamide,
p-tert-butyl-N-[6-(2-hydroxyéthoxy)-5-(m-méthoxyphénoxy)-4-pyrimidinyl]benzènesulfonamide,
p-tert-butyl-N-[6-(2-hydroxyéthoxy)-5-phénoxy-4-pyrimidinyl]benzènesulfonamide,
p-tert-butyl-N-[6-(2-hydroxyéthoxy)-5-(p-méthoxyphénoxy)-4-pyrimidinyl]benzènesulfonamide,
p-tert-butyl-N-[6-(2-hydroxyéthoxy)-5-(o-éthoxyphénoxy)-4-pyrimidinyl]benzènesulfonamide,
p-(2,2-diméthylpropyl)-N-[6-(2-hydroxyéthoxy)-5-(o-méthoxyphénoxy)-4-pyrimidinyl]benzènesulfonamide,
p-isopropyl-N-[6-(2-hydroxyéthoxy)-2-méthyl-5-(m-méthoxyphénoxy)-4-pyrimidinyl]benzènesulfonamide,
N-[6-(2-hydroxyéthoxy)-5-(o-méthoxyphénoxy)-2-phényl-4-pyrimidinyl]-p-isopropylbenzènesulfonamide,
N-[6-(2-hydroxyéthoxy)-5-(2,4,6-trichlorophénoxy)-4-pyrimidinyl]-p-isopropylbenzènesulfonamide,
N-[6-(2-hydroxyéthoxy)-6-(2,4,6-trichlorophénoxy)-4-pyrimidinyl]-o-toluènesulfonamide,
N-[6-(2-hydroxyéthoxy)-5-(2,4,6-trichlorophénoxy)-4-pyrimidinyl]-2,4-xylènesulfonamide,
p-tert-butyl-N-[6-(2-hydroxyéthoxy)-5-[(2-méthoxy-p-tolyl)oxy-4-pyrimidinyl]benzènesulfonamide,
p-tert-butyl-N-[6-(2-hydroxyéthoxy)-5-(o-méthoxyphénoxy)-2-méthyl-4-pyrimidinyl]benzènesulfonamide,
N-[6-(2-hydroxyéthoxy)-5-(o-méthoxyphénoxy)-2-méthyl-4-pyrimidinyl]-p-isopropylbenzènesulfonamide,
N-[6-(2-hydroxyéthoxy)-5-(o-méthoxyphénoxy)-(2-trifluorométhyl)-4-pyrimidinyl]-p-isopropylbenzènesulfonamide,
p-tert-butyl-N-[6-(2-hydroxyéthoxy)-5-(o-méthoxyphénoxy)-2-(trifluorométhyl)-4-pyrimidinyl]benzènesulfonamide,
N-[5-(1,3-benzodioxol-5-yloxy)-6-(2-hydroxyéthoxy)-4-pyrimidinyl]-p-tert-butylbenzènesulfonamide,
N-[5-(1,3-benzodioxol-5-yloxy)-6-(2-hydroxyéthoxy)-4-pyrimidinyl]-p-isopropylbenzènesulfonamide,
N-[6-(2-hydroxyéthoxy)-5-(o-méthoxyphénoxy)-4-pyrimidinyl]-o-méthoxybenzènesulfonamide,
p-tert-butyl-N-[6-(4-hydroxybutoxy)-5-(o-méthoxyphénoxy)-2-méthyl-4-pyrimidinyl]benzènesulfonamide,
N-[6-(2-hydroxybutoxy)-5-(2-naphtyloxy)-4-pyrimidinyl]-p-isopropylbenzènesulfonamide,
N-[6-(2-hydroxyéthoxy)-5-(2-naphtyloxy)-4-pyrimidinyl]-p-tert-butylbenzènesulfonamide,
N-[6-(2-hydroxyéthoxy)-5-(o-méthoxyphénoxy)-2-propyl-4-pyrimidinyl]-p-isopropylbenzènesulfonamide,
p-tert-butyl-N-[6-(2-hydroxyéthoxy)-5-(o-méthoxyphénoxy)-2-propyl-4-pyrimidinyl]benzènesulfonamide,
α,α,α-trifluoro-N-[6-(2-hydroxyéthoxy)-5-(o-méthoxyphénoxy)-2-méthyl-4-pyrimidinyl]-p-toluènesulfonamide,
p-chloro-N-[6-(2-hydroxyéthoxy)-5-(o-méthoxyphénoxy)-2-méthyl-4-pyrimidinyl]benzènesulfonamide,
N-[6-(2-hydroxyéthoxy)-5-(o-méthoxyphénoxy)-2-méthyl-4-pyrimidinyl]-p-(trifluorométhoxy)benzènesulfonamide,
o-éthyl-N-[6-(2-hydroxyéthoxy)-5-(o-méthoxyphénoxy)-2-méthyl-4-pyrimidinyl]benzènesulfonamide,
N-[6-(2-hydroxyéthoxy)-5-(o-méthoxyphénoxy)-2-méthyl-4-pyrimidinyl]-p-toluènesulfonamide,
N-[6-(2-hydroxyéthoxy)-5-(o-méthoxyphénoxy)-2-méthyl-4-pyrimidinyl]-2-naphtylsulfonamide,
p-tert-butyl-N-[6-(3-hydroxypropoxy)-5-(o-méthoxyphénoxy)-2-méthyl-4-pyrimidinyl]benzènesulfonamide,
p-tert-butyl-N-[6-(2-hydroxyéthoxy)-5-[(o-méthylthio)-phénoxy]-4-pyrimidinyl]benzènesulfonamide,
p-tert-butyl-N-[6-(2-hydroxyéthoxy)-5-(o-méthoxyphénoxy)-2-phényl-4-pyrimidinyl]benzènesulfonamide,
N-[2-amino-6-(2-hydroxyéthoxy)-5-(o-méthoxyphénoxy)-4-pyrimidinyl]-p-tert-butylbenzènesulfonamide,
p-tert-butyl-N-[6-(2-hydroxyéthoxy)-2-méthyl-5-[o-(méthylthio)phénoxy]-4-pyrimidinyl]benzènesulfonamide et
p-tert-butyl-N-[6-(2-hydroxyéthoxy)-2-méthyl-5-[o-(R,S-méthylsulfinyl)phénoxy]-4-pyrimidinyl]benzènesulfonamide.

9. Procédé selon la revendication 3, pour la préparation des composés suivants:
4-tert-butyl-N-[5-(2-chloro-5-méthoxyphénoxy)-6-(2-hydroxyéthoxy-2-méthylpyrimidin-4-yl]benzènesulfonamide,
4-tert-butyl-N-[5-(2-chloro-5-méthoxyphénoxy)-6-(2-(3-furoyloxy)éthoxy)-2-méthylpyrimidin-4-yl]benzènesulfonamide,
4-tert-butyl-N-[6-(2-hydroxyéthoxy)-5-(2-méthoxyphénoxy)-2-(thiophén-2-yl)pyrimidin-4-yl]benzènesulfonamide,
4-tert-butyl-N-[6-(2-hydroxyéthoxy)-5-(2-méthoxyphénoxy)-2-(thiophén-3-yl)pyrimidin-4-yl]benzènesulfonamide,
4-tert-butyl-N-[2-(furann-2-yl)-6-(2-hydroxyéthoxy)-5-(2-méthoxyphénoxy)pyrimidin-4-yl]benzènesulfonamide,
4-tert-butyl-N-[2-(furann-3-yl)-6-(2-hydroxyéthoxy)-5-(2-méthoxyphénoxy)pyrimidin-4-yl]benzènesulfonamide,
4-tert-butyl-N-[6-(2-hydroxyéthoxy)-5-(2-méthoxyphénoxy)-2-(pyridin-2-yl)pyrimidin-4-yl]benzènesulfonamide,
4-tert-butyl-N-[6-(2-hydroxyéthoxy)-5-(2-méthoxyphénoxy)-2-pyridin-4-yl)pyrimidin-4-yl]benzènesulfonamide,
4-tert-butyl-N-[6-(2-hydroxyéthoxy)-5-(2-méthoxyphénoxy)-2-pyridin-3-yl)pyrimidin-4-yl]benzènesulfonamide,
oxyde-1 de 2-[4-(4-tert-butylphénylsulfonylamino)-6-(2-hydroxyéthoxy)-5-(2-méthoxyphénoxy)pyrimidin-2-yl]-pyridine,
oxyde-1 de 4-[4-(4-tert-butylphénylsulfonylamino)-6-(2-hydroxyéthoxy)-5-(2-méthoxyphénoxy)pyrimidin-2-yl]-pyridine,
4-tert-butyl-N-[6-(2-hydroxyéthoxy)-2-[2-hydroxyéthoxy)-éthyl]-5-(2-méthoxyphénoxy)pyrimidin-4-yl]benzène-sulfonamide,
4-tert-butyl-N-[2-cyclopropyl-6-(2-hydroxyéthoxy)-5-(2-méthoxyphénoxy)pyrimidin-4-yl]benzènesulfonamide,
4-tert-butyl-N-[2-éthyl-6-(hydroxyéthoxy)-5-(2-méthoxyphénoxy)pyrimidin-4-yl]benzènesulfonamide,
4-tert-butyl-N-[6-(2-hydroxyéthoxy)-2-isopropyl-5-(2-méthoxyphénoxy)pyrimidin-4-yl)benzènesulfonamide,
N-[5-(5-fluoro-2-méthoxyphénoxy)-6-(2-hydroxyéthoxy)-pyrimidin-4-yl]-4-trifluorométhylbenzènesulfonamide,
4-tert-butyl-N-[6-(2-hydroxyéthoxy)-5-(3-méthoxyphénoxy)-2,2'-bipyrimidin-4-yl]benzènesulfonamide,
4-tert-butyl-N-[5-(4-fluoro-2-méthoxyphénoxy)-6-(2-hydroxyéthoxy)-2,2'-bipyrimidin-4-yl]benzènesulfonamide,
4-tert-butyl-N-[5-(4-fluoro-2-méthoxyphénoxy)-6-(2-hydroxyéthoxy)-2-méthylpyrimidin-4-yl]benzènesulfonamide,
4-tert-butyl-N-[5-(4-fluoro-2-méthoxyphénoxy)-6-(2-hydroxyéthoxy)pyrimidin-4-yl]benzènsulfonamide
N-[5-(5-fluoro-2-méthoxyphénoxy)-6-(2-hydroxyéthoxy)-pyrimidin-4-yl]-4-isopropylbenzènesulfonamide,
N-[5-(5-fluoro-2-méthoxyphénoxy)-6-(2-hydroxyéthoxy)-pyrimidin-4-yl]-4-tert-butylbenzènesulfonamide,
4-tert-butyl-N-[5-(2-fluoro-6-méthoxy)-6-(2-hydroxyéthoxy)pyrimidin-4-yl]benzènesulfonamide,
4-tert-butyl-N-[6-(2-hydroxyéthoxy)-5-(3-méthoxyphénoxy)-2-(thiophén-2-yl)pyrimidin-4-yl]benzènesulfonamide,
4-tert-butyl-N-[6-(2-hydroxyéthoxy)-2-(2-méthoxyéthyl)-5-(3-méthoxyphénoxy)pyrimidin-4-yl]benzènesulfonamide,
3-méthylisoxazole-5-carboxylate de 2-[6-(4-tert-butyl-benzènesulfonamino)-5-(3-méthoxyphénoxy)pyrimidin-4-yl-oxy)éthyle,
indole-2-carboxylate de 2-[6-(4-tert-butylbenzènesulfonamino)-5-(3-méthoxyphénoxy)pyrimidin-4-yloxy)éthyle,
5-[N-[6-(2-hydroxyéthoxy)-5-(2-méthoxyphénoxy)pyrimidin-4-yl]aminosulfonyl]-2-méthoxyphénoxyacétate d'éthyle.

10. Procédé selon la revendication 3, pour la préparation du (RS)-4-tert-butyl-N-[5-(2-chloro-5-méthoxyphénoxy)-2-éthyl-6-(2-méthylsulfinyléthoxy)pyrimidin-4-yl]benzènesulfonamide.

11. Procédé selon la revendication 3, pour la préparation du (RS)-N-[5-(2-chloro-5-méthoxyphénoxy)-6-(2-méthylsulfinyléthoxy)pyrimidin-4-yl]-1,3-benzodioxol-5-sulfonamide.

12. Procédé selon la revendication 3, pour la préparation du 4-tert-butyl-N-[6-(2-hydroxyéthoxy)-5-(2-méthoxyphénoxy)-2-(pyrimidin-2-yl]pyrimidin-4-yl]benzènesulfonamide.

13. Procédé selon la revendication 1 ou 2, pour la préparation des composés de formule I dans lesquels Z est le groupe vinylène.

14. Procédé selon la revendication 14, pour la préparation des composés suivants:
acétate de 2-[[5-[(E/Z)-styryl]-6-p-toluènesulfonamide-4-pyrimidinyl]oxy]éthyle,
N-[6-(2-hydroxyéthoxy)-5-[(E/Z)-styryl]-4-pyrimidinyl]-p-toluènesulfonamide.
